# EUROPEAN PATENT APPLICATION

(11) **EP 3 889 266 A1**
(43) Date of publication of application: **06.10.2021**
(21) Application number: 20884222.9
(22) Date of filing: 13.10.2020
(51) Int. Cl.: C12N 15/63, C12N 15/11

(54) **METHOD FOR GENERATING NEW MUTATIONS IN ORGANISMS, AND APPLICATION THEREOF**

(30) Priority: 07.11.2019 CN 201911081617; 15.08.2020 CN 202010821877; 16.09.2020 CN 202010974151
(71) Applicant: Qingdao KingAgroot Chemical Compound Co., Ltd., Qingdao, Shandong 266000 (CN)
(72) Inventor: JIANG, Linjian, Qingdao, Shandong 266000 (CN); MO, Sudong, Qingdao, Shandong 266000 (CN); WANG, Jiyao, Qingdao, Shandong 266000 (CN); LI, Yucai, Qingdao, Shandong 266000 (CN); QI, Wei, Qingdao, Shandong 266000 (CN); LI, Huarong, Qingdao, Shandong 266000 (CN); CHEN, Bo, Qingdao, Shandong 266000 (CN)
(74) Representative: Finnegan Europe LLP
(86) International application number: PCT/CN2020/120633
(87) International publication number: WO 2021/088601

(57) **Abstract**

The present invention pertains to the technical field of genetic engineering, and specifically relates to a method for generating a site-specific mutation in an organism in the absence of an artificial DNA template and a use thereof. The method comprises the following steps: sequentially generating two or more DNA breaks at a specific site in a genome of an organism and spontaneously repairing them respectively, wherein a later DNA break is generated based on a new sequence generated from a previous DNA break repair. In the present invention, a new target is designed based on a sequence formed by a new repair event generated by sequential editing, and thus, mutations may be sequentially formed for multiple times at a specific site in the genome, which greatly enrich the types of repair events after DNA breaks, and realize new base substitution, deletion and insertion mutations that cannot be obtained in a single gene editing.

Sequence Listing

<110> Qingdao Kingagroot Chemical Compound Co., Ltd.

<120> A method for generating new mutations in organisms and use thereof

<130> IEC200334

<150> 201911081617X
<151> 2019-11-07

<150> 2020108218772
<151> 2020-08-15

<150> 2020109741512
<151> 2020-09-16

<160> 23

<170> PatentIn version 3.5

<210> 1
<211> 670
<212> PRT
<213> Arabidopsis thaliana ALS protein amino acid sequence (amino acid sequence)

<400> 1
Met Ala Ala Ala Thr Thr Thr Thr Thr Thr Ser Ser Ser Ile Ser Phe
1               5                   10                  15
Ser Thr Lys Pro Ser Pro Ser Ser Ser Lys Ser Pro Leu Pro Ile Ser
            20                  25                  30
Arg Phe Ser Leu Pro Phe Ser Leu Asn Pro Asn Lys Ser Ser Ser Ser
        35                  40                  45
Ser Arg Arg Arg Gly Ile Lys Ser Ser Ser Pro Ser Ser Ile Ser Ala
    50                  55                  60
Val Leu Asn Thr Thr Thr Asn Val Thr Thr Thr Pro Ser Pro Thr Lys
65                  70                  75                  80
Pro Thr Lys Pro Glu Thr Phe Ile Ser Arg Phe Ala Pro Asp Gln Pro
                85                  90                  95
Arg Lys Gly Ala Asp Ile Leu Val Glu Ala Leu Glu Arg Gln Gly Val
            100                 105                 110
Glu Thr Val Phe Ala Tyr Pro Gly Gly Ala Ser Met Glu Ile His Gln
        115                 120                 125
Ala Leu Thr Arg Ser Ser Ser Ile Arg Asn Val Leu Pro Arg His Glu
    130                 135                 140
Gln Gly Gly Val Phe Ala Ala Glu Gly Tyr Ala Arg Ser Ser Gly Lys
145                 150                 155                 160
Pro Gly Ile Cys Ile Ala Thr Ser Gly Pro Gly Ala Thr Asn Leu Val
                165                 170                 175
Ser Gly Leu Ala Asp Ala Leu Leu Asp Ser Val Pro Leu Val Ala Ile
            180                 185                 190
Thr Gly Gln Val Pro Arg Arg Met Ile Gly Thr Asp Ala Phe Gln Glu
        195                 200                 205
Thr Pro Ile Val Glu Val Thr Arg Ser Ile Thr Lys His Asn Tyr Leu
    210                 215                 220
Val Met Asp Val Glu Asp Ile Pro Arg Ile Ile Glu Glu Ala Phe Phe
225                 230                 235                 240
Leu Ala Thr Ser Gly Arg Pro Gly Pro Val Leu Val Asp Val Pro Lys
                245                 250                 255
Asp Ile Gln Gln Gln Leu Ala Ile Pro Asn Trp Glu Gln Ala Met Arg
            260                 265                 270
Leu Pro Gly Tyr Met Ser Arg Met Pro Lys Pro Pro Glu Asp Ser His

        275                 280                 285
Leu Glu Gln Ile Val Arg Leu Ile Ser Glu Ser Lys Lys Pro Val Leu
    290                 295                 300
Tyr Val Gly Gly Gly Cys Leu Asn Ser Ser Asp Glu Leu Gly Arg Phe
305                 310                 315                 320
Val Glu Leu Thr Gly Ile Pro Val Ala Ser Thr Leu Met Gly Leu Gly
                325                 330                 335
Ser Tyr Pro Cys Asp Asp Glu Leu Ser Leu His Met Leu Gly Met His
            340                 345                 350
Gly Thr Val Tyr Ala Asn Tyr Ala Val Glu His Ser Asp Leu Leu Leu
        355                 360                 365
Ala Phe Gly Val Arg Phe Asp Asp Arg Val Thr Gly Lys Leu Glu Ala
    370                 375                 380
Phe Ala Ser Arg Ala Lys Ile Val His Ile Asp Ile Asp Ser Ala Glu
385                 390                 395                 400
Ile Gly Lys Asn Lys Thr Pro His Val Ser Val Cys Gly Asp Val Lys
                405                 410                 415
Leu Ala Leu Gln Gly Met Asn Lys Val Leu Glu Asn Arg Ala Glu Glu
            420                 425                 430
Leu Lys Leu Asp Phe Gly Val Trp Arg Asn Glu Leu Asn Val Gln Lys
        435                 440                 445
Gln Lys Phe Pro Leu Ser Phe Lys Thr Phe Gly Glu Ala Ile Pro Pro
    450                 455                 460
Gln Tyr Ala Ile Lys Val Leu Asp Glu Leu Thr Asp Gly Lys Ala Ile
465                 470                 475                 480
Ile Ser Thr Gly Val Gly Gln His Gln Met Trp Ala Ala Gln Phe Tyr
                485                 490                 495
Asn Tyr Lys Lys Pro Arg Gln Trp Leu Ser Ser Gly Gly Leu Gly Ala
            500                 505                 510
Met Gly Phe Gly Leu Pro Ala Ala Ile Gly Ala Ser Val Ala Asn Pro
        515                 520                 525
Asp Ala Ile Val Val Asp Ile Asp Gly Asp Gly Ser Phe Ile Met Asn
    530                 535                 540
Val Gln Glu Leu Ala Thr Ile Arg Val Glu Asn Leu Pro Val Lys Val
545                 550                 555                 560
Leu Leu Leu Asn Asn Gln His Leu Gly Met Val Met Gln Trp Glu Asp
                565                 570                 575
Arg Phe Tyr Lys Ala Asn Arg Ala His Thr Phe Leu Gly Asp Pro Ala
            580                 585                 590
Gln Glu Asp Glu Ile Phe Pro Asn Met Leu Leu Phe Ala Ala Ala Cys
        595                 600                 605
Gly Ile Pro Ala Ala Arg Val Thr Lys Lys Ala Asp Leu Arg Glu Ala
    610                 615                 620
Ile Gln Thr Met Leu Asp Thr Pro Gly Pro Tyr Leu Leu Asp Val Ile
625                 630                 635                 640
Cys Pro His Gln Glu His Val Leu Pro Met Ile Pro Ser Gly Gly Thr
                645                 650                 655
Phe Asn Asp Val Ile Thr Glu Gly Asp Gly Arg Ile Lys Tyr
            660                 665                 670

<210> 2
<211> 2013
<212> DNA
<213> Arabidopsis thaliana ALS gene DNA sequence (DNA sequence)

<400> 2
atggcggcgg caacaacaac aacaacaaca tcttcttcga tctccttctc caccaaacca 60
tctccttcct cctccaaatc accattacca atctccagat tctccctccc attctcccta 120
aaccccaaca aatcatcctc ctcctcccgc cgccgcggta tcaaatccag ctctccctcc 180
tccatctccg ccgtgctcaa cacaaccacc aatgtcacaa ccactccctc tccaaccaaa 240
cctaccaaac ccgaaacatt catctcccga ttcgctccag atcaaccccg caaaggcgct 300
gatatcctcg tcgaagcttt agaacgtcaa ggcgtagaaa ccgtattcgc ttaccctgga 360
ggtgcatcaa tggagattca ccaagcctta acccgctctt cctcaatccg taacgtcctt 420

cctcgtcacg aacaaggagg tgtattcgca gcagaaggat acgctcgatc ctcaggtaaa 480
ccaggtatct gtatagccac ttcaggtccc ggagctacaa atctcgttag cggattagcc 540
gatgcgttgt tagatagtgt tcctcttgta gcaatcacag gacaagtccc tcgtcgtatg 600
attggtacag atgcgtttca agagactccg attgttgagg taacgcgttc gattacgaag 660
cataactatc ttgtgatgga tgttgaagat atccctagga ttattgagga agctttcttt 720
ttagctactt ctggtagacc tggacctgtt ttggttgatg ttcctaaaga tattcaacaa 780
cagcttgcga ttcctaattg ggaacaggct atgagattac ctggttatat gtctaggatg 840
cctaaacctc cggaagattc tcatttggag cagattgtta ggttgatttc tgagtctaag 900
aagcctgtgt tgtatgttgg tggtggttgt ttgaattcta gcgatgaatt gggtaggttt 960
gttgagctta cggggatccc tgttgcgagt acgttgatgg ggctgggatc ttatccttgt 1020
gatgatgagt tgtcgttaca tatgcttgga atgcatggga ctgtgtatgc aaattacgct 1080
gtggagcata gtgatttgtt gttggcgttt ggggtaaggt ttgatgatcg tgtcacgggt 1140
aagcttgagg cttttgctag tagggctaag attgttcata ttgatattga ctcggctgag 1200
attgggaaga ataagactcc tcatgtgtct gtgtgtggtg atgttaagct ggctttgcaa 1260
gggatgaata aggttcttga gaaccgagcg gaggagctta agcttgattt tggagtttgg 1320
aggaatgagt tgaacgtaca gaaacagaag tttccgttga gctttaagac gtttggggaa 1380
gctattcctc cacagtatgc gattaaggtc cttgatgagt tgactgatgg aaaagccata 1440
ataagtactg gtgtcgggca acatcaaatg tgggcggcgc agttctacaa ttacaagaaa 1500
ccaaggcagt ggctatcatc aggaggcctt ggagctatgg gatttggact tcctgctgcg 1560
attggagcgt ctgttgctaa ccctgatgcg atagttgtgg atattgacgg agatggaagc 1620
tttataatga atgtgcaaga gctagccact attcgtgtag agaatcttcc agtgaaggta 1680
cttttattaa acaaccagca tcttggcatg gttatgcaat gggaagatcg gttctacaaa 1740
gctaaccgag ctcacacatt tctcggggat ccggctcagg aggacgagat attcccgaac 1800
atgttgctgt ttgcagcagc ttgcgggatt ccagcggcga gggtgacaaa gaaagcagat 1860
ctccgagaag ctattcagac aatgctggat acaccaggac cttacctgtt ggatgtgatt 1920
tgtccgcacc aagaacatgt gttgccgatg atcccgagtg gtggcacttt caacgatgtc 1980
ataacggaag gagatggccg gattaaatac tga 2013

<210> 3
<211> 2320
<212> PRT
<213> Alopecurus myosuroides ACCase protein amino acid sequence (amino acid
sequence)

<400> 3
Met Gly Ser Thr His Leu Pro Ile Val Gly Phe Asn Ala Ser Thr Thr
1               5                   10                  15
Pro Ser Leu Ser Thr Leu Arg Gln Ile Asn Ser Ala Ala Ala Ala Phe
            20                  25                  30
Gln Ser Ser Ser Pro Ser Arg Ser Ser Lys Lys Lys Ser Arg Arg Val
        35                  40                  45
Lys Ser Ile Arg Asp Asp Gly Asp Gly Ser Val Pro Asp Pro Ala Gly
    50                  55                  60
His Gly Gln Ser Ile Arg Gln Gly Leu Ala Gly Ile Ile Asp Leu Pro
65                  70                  75                  80
Lys Glu Gly Ala Ser Ala Pro Asp Val Asp Ile Ser His Gly Ser Glu
                85                  90                  95
Asp His Lys Ala Ser Tyr Gln Met Asn Gly Ile Leu Asn Glu Ser His
            100                 105                 110
Asn Gly Arg His Ala Ser Leu Ser Lys Val Tyr Glu Phe Cys Thr Glu
        115                 120                 125
Leu Gly Gly Lys Thr Pro Ile His Ser Val Leu Val Ala Asn Asn Gly
    130                 135                 140
Met Ala Ala Ala Lys Phe Met Arg Ser Val Arg Thr Trp Ala Asn Asp
145                 150                 155                 160
Thr Phe Gly Ser Glu Lys Ala Ile Gln Leu Ile Ala Met Ala Thr Pro
                165                 170                 175
Glu Asp Met Arg Ile Asn Ala Glu His Ile Arg Ile Ala Asp Gln Phe
            180                 185                 190
Val Glu Val Pro Gly Gly Thr Asn Asn Asn Asn Tyr Ala Asn Val Gln
        195                 200                 205
Leu Ile Val Glu Ile Ala Glu Arg Thr Gly Val Ser Ala Val Trp Pro
    210                 215                 220

Gly Trp Gly His Ala Ser Glu Asn Pro Glu Leu Pro Asp Ala Leu Thr
225                 230                 235                 240
Ala Lys Gly Ile Val Phe Leu Gly Pro Pro Ala Ser Ser Met Asn Ala
                245                 250                 255
Leu Gly Asp Lys Val Gly Ser Ala Leu Ile Ala Gln Ala Ala Gly Val
            260                 265                 270
Pro Thr Leu Ala Trp Ser Gly Ser His Val Glu Ile Pro Leu Glu Leu
        275                 280                 285
Cys Leu Asp Ser Ile Pro Glu Glu Met Tyr Arg Lys Ala Cys Val Thr
    290                 295                 300
Thr Ala Asp Glu Ala Val Ala Ser Cys Gln Met Ile Gly Tyr Pro Ala
305                 310                 315                 320
Met Ile Lys Ala Ser Trp Gly Gly Gly Gly Lys Gly Ile Arg Lys Val
                325                 330                 335
Asn Asn Asp Asp Glu Val Lys Ala Leu Phe Lys Gln Val Gln Gly Glu
            340                 345                 350
Val Pro Gly Ser Pro Ile Phe Ile Met Arg Leu Ala Ser Gln Ser Arg
        355                 360                 365
His Leu Glu Val Gln Leu Leu Cys Asp Glu Tyr Gly Asn Val Ala Ala
    370                 375                 380
Leu His Ser Arg Asp Cys Ser Val Gln Arg Arg His Gln Lys Ile Ile
385                 390                 395                 400
Glu Glu Gly Pro Val Thr Val Ala Pro Arg Glu Thr Val Lys Glu Leu
                405                 410                 415
Glu Gln Ala Ala Arg Arg Leu Ala Lys Ala Val Gly Tyr Val Gly Ala
            420                 425                 430
Ala Thr Val Glu Tyr Leu Tyr Ser Met Glu Thr Gly Glu Tyr Tyr Phe
        435                 440                 445
Leu Glu Leu Asn Pro Arg Leu Gln Val Glu His Pro Val Thr Glu Ser
    450                 455                 460
Ile Ala Glu Val Asn Leu Pro Ala Ala Gln Val Ala Val Gly Met Gly
465                 470                 475                 480
Ile Pro Leu Trp Gln Ile Pro Glu Ile Arg Arg Phe Tyr Gly Met Asp
                485                 490                 495
Asn Gly Gly Gly Tyr Asp Ile Trp Arg Lys Thr Ala Ala Leu Ala Thr
            500                 505                 510
Pro Phe Asn Phe Asp Glu Val Asp Ser Gln Trp Pro Lys Gly His Cys
        515                 520                 525
Val Ala Val Arg Ile Thr Ser Glu Asn Pro Asp Asp Gly Phe Lys Pro
    530                 535                 540
Thr Gly Gly Lys Val Lys Glu Ile Ser Phe Lys Ser Lys Pro Asn Val
545                 550                 555                 560
Trp Gly Tyr Phe Ser Val Lys Ser Gly Gly Gly Ile His Glu Phe Ala
                565                 570                 575
Asp Ser Gln Phe Gly His Val Phe Ala Tyr Gly Glu Thr Arg Ser Ala
            580                 585                 590
Ala Ile Thr Ser Met Ser Leu Ala Leu Lys Glu Ile Gln Ile Arg Gly
        595                 600                 605
Glu Ile His Thr Asn Val Asp Tyr Thr Val Asp Leu Leu Asn Ala Pro
    610                 615                 620
Asp Phe Arg Glu Asn Thr Ile His Thr Gly Trp Leu Asp Thr Arg Ile
625                 630                 635                 640
Ala Met Arg Val Gln Ala Glu Arg Pro Pro Trp Tyr Ile Ser Val Val
                645                 650                 655
Gly Gly Ala Leu Tyr Lys Thr Ile Thr Thr Asn Ala Glu Thr Val Ser
            660                 665                 670
Glu Tyr Val Ser Tyr Leu Ile Lys Gly Gln Ile Pro Pro Lys His Ile
        675                 680                 685
Ser Leu Val His Ser Thr Ile Ser Leu Asn Ile Glu Glu Ser Lys Tyr
    690                 695                 700
Thr Ile Glu Ile Val Arg Ser Gly Gln Gly Ser Tyr Arg Leu Arg Leu
705                 710                 715                 720
Asn Gly Ser Leu Ile Glu Ala Asn Val Gln Thr Leu Cys Asp Gly Gly

                725                 730                 735
Leu Leu Met Gln Leu Asp Gly Asn Ser His Val Ile Tyr Ala Glu Glu
            740                 745                 750
Glu Ala Gly Gly Thr Arg Leu Leu Ile Asp Gly Lys Thr Cys Leu Leu
        755                 760                 765
Gln Asn Asp His Asp Pro Ser Arg Leu Leu Ala Glu Thr Pro Cys Lys
    770                 775                 780
Leu Leu Arg Phe Leu Ile Ala Asp Gly Ala His Val Asp Ala Asp Val
785                 790                 795                 800
Pro Tyr Ala Glu Val Glu Val Met Lys Met Cys Met Pro Leu Leu Ser
                805                 810                 815
Pro Ala Ala Gly Val Ile Asn Val Leu Leu Ser Glu Gly Gln Ala Met
            820                 825                 830
Gln Ala Gly Asp Leu Ile Ala Arg Leu Asp Leu Asp Asp Pro Ser Ala
        835                 840                 845
Val Lys Arg Ala Glu Pro Phe Glu Gly Ser Phe Pro Glu Met Ser Leu
    850                 855                 860
Pro Ile Ala Ala Ser Gly Gln Val His Lys Arg Cys Ala Ala Ser Leu
865                 870                 875                 880
Asn Ala Ala Arg Met Val Leu Ala Gly Tyr Asp His Ala Ala Asn Lys
                885                 890                 895
Val Val Gln Asp Leu Val Trp Cys Leu Asp Thr Pro Ala Leu Pro Phe
            900                 905                 910
Leu Gln Trp Glu Glu Leu Met Ser Val Leu Ala Thr Arg Leu Pro Arg
        915                 920                 925
Arg Leu Lys Ser Glu Leu Glu Gly Lys Tyr Asn Glu Tyr Lys Leu Asn
    930                 935                 940
Val Asp His Val Lys Ile Lys Asp Phe Pro Thr Glu Met Leu Arg Glu
945                 950                 955                 960
Thr Ile Glu Glu Asn Leu Ala Cys Val Ser Glu Lys Glu Met Val Thr
                965                 970                 975
Ile Glu Arg Leu Val Asp Pro Leu Met Ser Leu Leu Lys Ser Tyr Glu
            980                 985                 990
Gly Gly Arg Glu Ser His Ala His Phe Ile Val Lys Ser Leu Phe Glu
        995                 1000                1005
Glu Tyr Leu Ser Val Glu Glu Leu Phe Ser Asp Gly Ile Gln Ser Asp
    1010                1015                1020
Val Ile Glu Arg Leu Arg Leu Gln Tyr Ser Lys Asp Leu Gln Lys Val
1025                1030                1035                1040
Val Asp Ile Val Leu Ser His Gln Gly Val Arg Asn Lys Thr Lys Leu
                1045                1050                1055
Ile Leu Ala Leu Met Glu Lys Leu Val Tyr Pro Asn Pro Ala Ala Tyr
            1060                1065                1070
Arg Asp Gln Leu Ile Arg Phe Ser Ser Leu Asn His Lys Arg Tyr Tyr
        1075                1080                1085
Lys Leu Ala Leu Lys Ala Ser Glu Leu Leu Glu Gln Thr Lys Leu Ser
    1090                1095                1100
Glu Leu Arg Thr Ser Ile Ala Arg Asn Leu Ser Ala Leu Asp Met Phe
1105                1110                1115                1120
Thr Glu Glu Lys Ala Asp Phe Ser Leu Gln Asp Arg Lys Leu Ala Ile
                1125                1130                1135
Asn Glu Ser Met Gly Asp Leu Val Thr Ala Pro Leu Pro Val Glu Asp
            1140                1145                1150
Ala Leu Val Ser Leu Phe Asp Cys Thr Asp Gln Thr Leu Gln Gln Arg
        1155                1160                1165
Val Ile Gln Thr Tyr Ile Ser Arg Leu Tyr Gln Pro Gln Leu Val Lys
    1170                1175                1180
Asp Ser Ile Gln Leu Lys Tyr Gln Asp Ser Gly Val Ile Ala Leu Trp
1185                1190                1195                1200
Glu Phe Thr Glu Gly Asn His Glu Lys Arg Leu Gly Ala Met Val Ile
                1205                1210                1215
Leu Lys Ser Leu Glu Ser Val Ser Thr Ala Ile Gly Ala Ala Leu Lys
            1220                1225                1230

Asp Ala Ser His Tyr Ala Ser Ser Ala Gly Asn Thr Val His Ile Ala
        1235                1240                1245
Leu Leu Asp Ala Asp Thr Gln Leu Asn Thr Thr Glu Asp Ser Gly Asp
    1250                1255                1260
Asn Asp Gln Ala Gln Asp Lys Met Asp Lys Leu Ser Phe Val Leu Lys
1265                1270                1275                1280
Gln Asp Val Val Met Ala Asp Leu Arg Ala Ala Asp Val Lys Val Val
                1285                1290                1295
Ser Cys Ile Val Gln Arg Asp Gly Ala Ile Met Pro Met Arg Arg Thr
            1300                1305                1310
Phe Leu Leu Ser Glu Glu Lys Leu Cys Tyr Glu Glu Glu Pro Ile Leu
        1315                1320                1325
Arg His Val Glu Pro Pro Leu Ser Ala Leu Leu Glu Leu Asp Lys Leu
    1330                1335                1340
Lys Val Lys Gly Tyr Asn Glu Met Lys Tyr Thr Pro Ser Arg Asp Arg
1345                1350                1355                1360
Gln Trp His Ile Tyr Thr Leu Arg Asn Thr Glu Asn Pro Lys Met Leu
                1365                1370                1375
His Arg Val Phe Phe Arg Thr Leu Val Arg Gln Pro Ser Ala Gly Asn
            1380                1385                1390
Arg Phe Thr Ser Asp His Ile Thr Asp Val Glu Val Gly His Ala Glu
        1395                1400                1405
Glu Pro Leu Ser Phe Thr Ser Ser Ser Ile Leu Lys Ser Leu Lys Ile
    1410                1415                1420
Ala Lys Glu Glu Leu Glu Leu His Ala Ile Arg Thr Gly His Ser His
1425                1430                1435                1440
Met Tyr Leu Cys Ile Leu Lys Glu Gln Lys Leu Leu Asp Leu Val Pro
                1445                1450                1455
Val Ser Gly Asn Thr Val Val Asp Val Gly Gln Asp Glu Ala Thr Ala
            1460                1465                1470
Cys Ser Leu Leu Lys Glu Met Ala Leu Lys Ile His Glu Leu Val Gly
        1475                1480                1485
Ala Arg Met His His Leu Ser Val Cys Gln Trp Glu Val Lys Leu Lys
    1490                1495                1500
Leu Val Ser Asp Gly Pro Ala Ser Gly Ser Trp Arg Val Val Thr Thr
1505                1510                1515                1520
Asn Val Thr Gly His Thr Cys Thr Val Asp Ile Tyr Arg Glu Val Glu
                1525                1530                1535
Asp Thr Glu Ser Gln Lys Leu Val Tyr His Ser Thr Ala Leu Ser Ser
            1540                1545                1550
Gly Pro Leu His Gly Val Ala Leu Asn Thr Ser Tyr Gln Pro Leu Ser
        1555                1560                1565
Val Ile Asp Leu Lys Arg Cys Ser Ala Arg Asn Asn Lys Thr Thr Tyr
    1570                1575                1580
Cys Tyr Asp Phe Pro Leu Thr Phe Glu Ala Ala Val Gln Lys Ser Trp
1585                1590                1595                1600
Ser Asn Ile Ser Ser Glu Asn Asn Gln Cys Tyr Val Lys Ala Thr Glu
                1605                1610                1615
Leu Val Phe Ala Glu Lys Asn Gly Ser Trp Gly Thr Pro Ile Ile Pro
            1620                1625                1630
Met Gln Arg Ala Ala Gly Leu Asn Asp Ile Gly Met Val Ala Trp Ile
        1635                1640                1645
Leu Asp Met Ser Thr Pro Glu Phe Pro Ser Gly Arg Gln Ile Ile Val
    1650                1655                1660
Ile Ala Asn Asp Ile Thr Phe Arg Ala Gly Ser Phe Gly Pro Arg Glu
1665                1670                1675                1680
Asp Ala Phe Phe Glu Ala Val Thr Asn Leu Ala Cys Glu Lys Lys Leu
                1685                1690                1695
Pro Leu Ile Tyr Leu Ala Ala Asn Ser Gly Ala Arg Ile Gly Ile Ala
            1700                1705                1710
Asp Glu Val Lys Ser Cys Phe Arg Val Gly Trp Thr Asp Asp Ser Ser
        1715                1720                1725
Pro Glu Arg Gly Phe Arg Tyr Ile Tyr Met Thr Asp Glu Asp His Asp

1730                1735                1740
Arg Ile Gly Ser Ser Val Ile Ala His Lys Met Gln Leu Asp Ser Gly
1745                1750                1755                1760
Glu Ile Arg Trp Val Ile Asp Ser Val Val Gly Lys Glu Asp Gly Leu
                1765                1770                1775
Gly Val Glu Asn Ile His Gly Ser Ala Ala Ile Ala Ser Ala Tyr Ser
            1780                1785                1790
Arg Ala Tyr Glu Glu Thr Phe Thr Leu Thr Phe Val Thr Gly Arg Thr
        1795                1800                1805
Val Gly Ile Gly Ala Tyr Leu Ala Arg Leu Gly Ile Arg Cys Ile Gln
    1810                1815                1820
Arg Ile Asp Gln Pro Ile Ile Leu Thr Gly Phe Ser Ala Leu Asn Lys
1825                1830                1835                1840
Leu Leu Gly Arg Glu Val Tyr Ser Ser His Met Gln Leu Gly Gly Pro
                1845                1850                1855
Lys Ile Met Ala Thr Asn Gly Val Val His Leu Thr Val Pro Asp Asp
            1860                1865                1870
Leu Glu Gly Val Ser Asn Ile Leu Arg Trp Leu Ser Tyr Val Pro Ala
        1875                1880                1885
Asn Ile Gly Gly Pro Leu Pro Ile Thr Lys Ser Leu Asp Pro Ile Asp
    1890                1895                1900
Arg Pro Val Ala Tyr Ile Pro Glu Asn Thr Cys Asp Pro Arg Ala Ala
1905                1910                1915                1920
Ile Ser Gly Ile Asp Asp Ser Gln Gly Lys Trp Leu Gly Gly Met Phe
                1925                1930                1935
Asp Lys Asp Ser Phe Val Glu Thr Phe Glu Gly Trp Ala Lys Thr Val
            1940                1945                1950
Val Thr Gly Arg Ala Lys Leu Gly Gly Ile Pro Val Gly Val Ile Ala
        1955                1960                1965
Val Glu Thr Gln Thr Met Met Gln Leu Val Pro Ala Asp Pro Gly Gln
    1970                1975                1980
Pro Asp Ser His Glu Arg Ser Val Pro Arg Ala Gly Gln Val Trp Phe
1985                1990                1995                2000
Pro Asp Ser Ala Thr Lys Thr Ala Gln Ala Met Leu Asp Phe Asn Arg
                2005                2010                2015
Glu Gly Leu Pro Leu Phe Ile Leu Ala Asn Trp Arg Gly Phe Ser Gly
            2020                2025                2030
Gly Gln Arg Asp Leu Phe Glu Gly Ile Leu Gln Ala Gly Ser Thr Ile
        2035                2040                2045
Val Glu Asn Leu Arg Thr Tyr Asn Gln Pro Ala Phe Val Tyr Ile Pro
    2050                2055                2060
Lys Ala Ala Glu Leu Arg Gly Gly Ala Trp Val Val Ile Asp Ser Lys
2065                2070                2075                2080
Ile Asn Pro Asp Arg Ile Glu Cys Tyr Ala Glu Arg Thr Ala Lys Gly
                2085                2090                2095
Asn Val Leu Glu Pro Gln Gly Leu Ile Glu Ile Lys Phe Arg Ser Glu
            2100                2105                2110
Glu Leu Lys Glu Cys Met Gly Arg Leu Asp Pro Glu Leu Ile Asp Leu
        2115                2120                2125
Lys Ala Arg Leu Gln Gly Ala Asn Gly Ser Leu Ser Asp Gly Glu Ser
    2130                2135                2140
Leu Gln Lys Ser Ile Glu Ala Arg Lys Lys Gln Leu Leu Pro Leu Tyr
2145                2150                2155                2160
Thr Gln Ile Ala Val Arg Phe Ala Glu Leu His Asp Thr Ser Leu Arg
                2165                2170                2175
Met Ala Ala Lys Gly Val Ile Arg Lys Val Val Asp Trp Glu Asp Ser
            2180                2185                2190
Arg Ser Phe Phe Tyr Lys Arg Leu Arg Arg Arg Leu Ser Glu Asp Val
        2195                2200                2205
Leu Ala Lys Glu Ile Arg Gly Val Ile Gly Glu Lys Phe Pro His Lys
    2210                2215                2220
Ser Ala Ile Glu Leu Ile Lys Lys Trp Tyr Leu Ala Ser Glu Ala Ala
2225                2230                2235                2240

Ala Ala Gly Ser Thr Asp Trp Asp Asp Asp Asp Ala Phe Val Ala Trp
                2245                2250                2255
Arg Glu Asn Pro Glu Asn Tyr Lys Glu Tyr Ile Lys Glu Leu Arg Ala
            2260                2265                2270
Gln Arg Val Ser Arg Leu Leu Ser Asp Val Ala Gly Ser Ser Ser Asp
        2275                2280                2285
Leu Gln Ala Leu Pro Gln Gly Leu Ser Met Leu Leu Asp Lys Met Asp
    2290                2295                2300
Pro Ser Lys Arg Ala Gln Phe Ile Glu Glu Val Met Lys Val Leu Lys
2305                2310                2315                2320

<210> 4
<211> 6963
<212> DNA
<213> Alopecurus myosuroides ACCase gene DNA sequence (DNA sequence)
<400> 4
atgggatcca cacatctgcc cattgtcggg tttaatgcat ccacaacacc atcgctatcc 60
actcttcgcc agataaactc agctgctgct gcattccaat cttcgtcccc ttcaaggtca 120
tccaagaaga aaagccgacg tgttaagtca ataagggatg atggcgatgg aagcgtgcca 180
gaccctgcag gccatggcca gtctattcgc caaggtctcg ctggcatcat cgacctccca 240
aaggagggcg catcagctcc agatgtggac atttcacatg ggtctgaaga ccacaaggcc 300
tcctaccaaa tgaatgggat actgaatgaa tcacataacg ggaggcacgc ctctctgtct 360
aaagtttatg aattttgcac ggaattgggt ggaaaaacac caattcacag tgtattagtc 420
gccaacaatg gaatggcagc agctaagttc atgcggagtg tccggacatg ggctaatgat 480
acatttgggt cagagaaggc gattcagttg atagctatgg caactccgga agacatgaga 540
ataaatgcag agcacattag aattgctgat cagtttgttg aagtacctgg tggaacaaac 600
aataacaact atgcaaatgt ccaactcata gtggagatag cagagagaac tggtgtctcc 660
gccgtttggc ctggttgggg ccatgcatct gagaatcctg aacttccaga tgcactaact 720
gcaaaaggaa ttgtttttct tgggccacca gcatcatcaa tgaacgcact aggcgacaag 780
gttggttcag ctctcattgc tcaagcagca ggggttccca ctcttgcttg gagtggatca 840
catgtggaaa ttccattaga actttgtttg gactcgatac ctgaggagat gtataggaaa 900
gcctgtgtta caaccgctga tgaagcagtt gcaagttgtc agatgattgg ttaccctgcc 960
atgatcaagg catcctgggg tggtggtggt aaaggattta gaaaggttaa taatgatgac 1020
gaggtgaaag cactgtttaa gcaagtacag ggtgaagttc ctggctcccc gatatttatc 1080
atgagacttg catctcagag tcgtcatctt gaagtccagc tgctttgtga tgaatatggc 1140
aatgtagcag cacttcacag tcgtgattgc agtgtgcaac gacgacacca aaagattatc 1200
gaggaaggac cagttactgt tgctcctcgt gaaacagtga aagagctaga gcaagcagca 1260
aggaggcttg ctaaggccgt gggttacgtc ggtgctgcta ctgttgaata tctctacagc 1320
atggagactg gtgaatacta ttttctggag cttaatccac ggttgcaggt tgagcaccca 1380
gtcaccgagt cgatagctga agtaaatttg cctgcagccc aagttgcagt tgggatgggt 1440
ataccccttt ggcagattcc agagatcaga cgtttctacg gaatggacaa tggaggaggc 1500
tatgatattt ggaggaaaac agcagctctc gctactccat tcaactttga tgaagtagat 1560
tctcaatggc cgaagggtca ttgtgtggca gttaggataa ccagtgagaa tccagatgat 1620
ggattcaagc ctactggtgg aaaagtaaag gagataagtt ttaaaagtaa gccaaatgtc 1680
tggggatatt tctcagttaa gtctggtgga ggcattcatg aatttgcgga ttctcagttt 1740
ggacacgttt ttgcctatgg agagactaga tcagcagcaa taaccagcat gtctcttgca 1800
ctaaaagaga ttcaaattcg tggagaaatt catacaaacg ttgattacac ggttgatctc 1860
ttgaatgccc cagacttcag agaaaacacg atccataccg gttggctgga taccagaata 1920
gctatgcgtg ttcaagctga gaggcctccc tggtatattt cagtggttgg aggagctcta 1980
tataaaacaa taaccaccaa tgcggagacc gtttctgaat atgttagcta tctcatcaag 2040
ggtcagattc caccaaagca catatccctt gtccattcaa ctatttcttt gaatatagag 2100
gaaagcaaat atacaattga gattgtgagg agtggacagg gtagctacag attgagactg 2160
aatggatcac ttattgaagc caatgtacaa acattatgtg atggaggcct tttaatgcag 2220
ctggatggaa atagccatgt tatttatgct gaagaagaag cgggtggtac acggcttctt 2280
attgatggaa aaacatgctt gctacagaat gaccatgatc cgtcaaggtt attagctgag 2340
acaccctgca aacttcttcg tttcttgatt gccgatggtg ctcatgttga tgctgatgta 2400
ccatacgcgg aagttgaggt tatgaagatg tgcatgcccc tcttgtcgcc tgctgctggt 2460
gtcattaatg ttttgttgtc tgagggccag gcgatgcagg ctggtgatct tatagcgaga 2520
cttgatctcg atgacccttc tgctgtgaag agagccgagc catttgaagg atcttttcca 2580
gaaatgagcc ttcctattgc tgcttctggc caagttcaca aaagatgtgc tgcaagtttg 2640
aacgctgctc gaatggtcct tgcaggatat gaccatgcgg ccaacaaagt tgtgcaagat 2700
ttggtatggt gccttgatac acctgctctt cctttcctac aatgggaaga gcttatgtct 2760
gttttagcaa ctagacttcc aagacgtctt aagagcgagt tggagggcaa atacaatgaa 2820

tacaagttaa atgttgacca tgtgaagatc aaggatttcc ctaccgagat gcttagagag 2880
acaatcgagg aaaatcttgc atgtgtttcc gagaaggaaa tggtgacaat tgagaggctt 2940
gttgaccctc tgatgagcct gctgaagtca tacgagggtg ggagagaaag ccatgcccac 3000
tttattgtca agtccctttt tgaggagtat ctctcggttg aggaactatt cagtgatggc 3060
attcagtctg acgtgattga acgcctgcgc ctacaatata gtaaagacct ccagaaggtt 3120
gtagacattg ttttgtctca ccagggtgtg agaaacaaaa caaagctgat actcgcgctc 3180
atggagaaac tggtctatcc aaaccctgct gcctacagag atcagttgat tcgcttttct 3240
tccctcaacc ataaaagata ttataagttg gctcttaaag ctagtgaact tcttgaacaa 3300
accaagctca gcgaactccg cacaagcatt gcaaggaacc tttcagcgct ggatatgttc 3360
accgaggaaa aggcagattt ctccttgcaa gacagaaaat tggccattaa tgagagcatg 3420
ggagatttag tcactgcccc actgccagtt gaagatgcac ttgtttcttt gtttgattgt 3480
actgatcaaa ctcttcagca gagagtgatt cagacataca tatctcgatt ataccagcct 3540
caacttgtga aggatagcat ccagctgaaa tatcaggatt ctggtgttat tgctttatgg 3600
gaattcactg aaggaaatca tgagaagaga ttgggtgcta tggttatcct gaagtcacta 3660
gaatctgtgt caacagccat tggagctgct ctaaaggatg catcacatta tgcaagctct 3720
gcgggcaaca cggtgcatat tgctttgttg gatgctgata cccaactgaa tacaactgaa 3780
gatagtggtg ataatgacca agctcaagac aagatggata aactttcttt tgtactgaaa 3840
caagatgttg tcatggctga tctacgtgct gctgatgtca aggttgttag ttgcattgtt 3900
caaagagatg gagcaatcat gcctatgcgc cgtaccttcc tcttgtcaga ggaaaaactt 3960
tgttacgagg aagagccgat tcttcggcat gtggagcctc cactttctgc acttcttgag 4020
ttggataaat tgaaagtgaa aggatacaat gagatgaagt atacaccgtc acgtgatcgt 4080
cagtggcata tatacacact tagaaatact gaaaatccaa aaatgctgca cagggtattt 4140
ttccgaacac ttgtcagaca acccagtgca ggcaacaggt ttacatcaga ccatatcact 4200
gatgttgaag taggacacgc agaggaacct ctttcattta cttcaagcag catattaaaa 4260
tcgttgaaga ttgctaaaga agaattggag cttcacgcga tcaggactgg ccattctcat 4320
atgtacttgt gcatattgaa agagcaaaag cttcttgacc ttgttcctgt ttcagggaac 4380
actgttgtgg atgttggtca agatgaagct actgcatgct ctcttttgaa agaaatggct 4440
ttaaagatac atgaacttgt tggtgcaaga atgcatcatc tttctgtatg ccagtgggaa 4500
gtgaaactta agttggtgag cgatggccct gccagtggta gctggagagt tgtaacaacc 4560
aatgttactg gtcacacctg cactgtggat atctaccggg aggtcgaaga tacagaatca 4620
cagaaactag tataccactc caccgcattg tcatctggtc ctttgcatgg tgttgcactg 4680
aatacttcgt atcagccttt gagtgttatt gatttaaaac gttgctctgc caggaacaac 4740
aaaactacat actgctatga ttttccattg acatttgaag ctgcagtgca gaagtcgtgg 4800
tctaacattt ccagtgaaaa caaccaatgt tatgttaaag cgacagagct tgtgtttgct 4860
gaaaagaatg ggtcgtgggg cactcctata attcctatgc agcgtgctgc tgggctgaat 4920
gacattggta tggtagcctg gatcttggac atgtccactc ctgaatttcc cagcggcaga 4980
cagatcattg ttatcgcaaa tgatattaca tttagagctg gatcatttgg cccaagggaa 5040
gatgcatttt tcgaagctgt aaccaacctg gcttgtgaga agaagcttcc acttatctac 5100
ttggctgcaa actctggtgc tcggattggc attgctgatg aagtaaaatc ttgcttccgt 5160
gttggatgga ctgatgatag cagccctgaa cgtggattta ggtacattta tatgactgac 5220
gaagaccatg atcgtattgg ctcttcagtt atagcacaca agatgcagct agatagtggc 5280
gagatcaggt gggttattga ttctgttgtg ggaaaagagg atggactagg tgtggagaac 5340
atacatggaa gtgctgctat tgccagtgcc tattctaggg cgtacgagga gacatttaca 5400
cttacattcg ttactggacg aactgttgga atcggagcct atcttgctcg acttggcata 5460
cggtgcatac agcgtattga ccagcccatt attttgaccg ggttttctgc cctgaacaag 5520
cttcttgggc gggaggtgta cagctcccac atgcagttgg gtggtcccaa aatcatggcg 5580
acgaatggtg ttgtccatct gactgttcca gatgaccttg aaggtgtttc taatatattg 5640
aggtggctca gctatgttcc tgcaaacatt ggtggacctc ttcctattac aaaatctttg 5700
gacccaatag acagacccgt tgcatacatc cctgagaata catgtgatcc tcgtgcagcc 5760
atcagtggca ttgatgacag ccaagggaaa tggttgggtg gcatgtttga caaagacagt 5820
tttgtggaga catttgaagg atgggcgaag acagtagtta ctggcagagc aaaacttgga 5880
gggattcctg ttggtgttat agctgtggag acacagacca tgatgcagct cgtccccgct 5940
gatccaggcc agcctgattc ccacgagcgg tctgttcctc gtgctgggca agtttggttt 6000
ccagattctg ctaccaagac agcgcaggcg atgttggact tcaaccgtga aggattacct 6060
ctgttcatac ttgctaactg gagaggcttc tctggagggc aaagagatct ttttgaagga 6120
attctgcagg ctgggtcaac aattgttgag aaccttagga catacaatca gcctgccttt 6180
gtatatatcc ccaaggctgc agagctacgt ggaggagcct gggtcgtgat tgatagcaag 6240
ataaacccag atcgcatcga gtgctatgct gagaggactg caaagggtaa tgttctcgaa 6300
cctcaagggt tgattgagat caagttcagg tcagaggaac tcaaagaatg catgggtagg 6360
cttgatccag aattgataga tctgaaagca agactccagg gagcaaatgg aagcctatct 6420
gatggagaat cccttcagaa gagcatagaa gctcggaaga aacagttgct gcctctgtac 6480
acccaaatcg cggtacgttt tgcggaattg cacgacactt cccttagaat ggctgctaaa 6540
ggtgtgatca ggaaagttgt agactgggaa gactctcggt ctttcttcta caagagatta 6600

cggaggaggc tatccgagga cgttctggca aaggagatta gaggtgtaat tggtgagaag 6660
tttcctcaca aatcagcgat cgagctgatc aagaaatggt acttggcttc tgaggcagct 6720
gcagcaggaa gcaccgactg ggatgacgac gatgcttttg tcgcctggag ggagaaccct 6780
gaaaactata aggagtatat caaagagctt agggctcaaa gggtatctcg gttgctctca 6840
gatgttgcag gctccagttc ggatttacaa gccttgccgc agggtctttc catgctacta 6900
gataagatgg atccctctaa gagagcacag tttatcgagg aggtcatgaa ggtcctgaaa 6960
tga 6963

<210> 5
<211> 446
<212> PRT
<213> Oryza sativa L. HPPD protein amino acid sequence (amino acid sequence)

<400> 5
Met Pro Pro Thr Pro Thr Pro Thr Ala Thr Thr Gly Ala Val Ser Ala
1               5                   10                  15
Ala Ala Ala Ala Gly Glu Asn Ala Gly Phe Arg Leu Val Gly His Arg
            20                  25                  30
Arg Phe Val Arg Ala Asn Pro Arg Ser Asp Arg Phe Gln Ala Leu Ala
        35                  40                  45
Phe His His Val Glu Leu Trp Cys Ala Asp Ala Ala Ser Ala Ala Gly
    50                  55                  60
Arg Phe Ala Phe Ala Leu Gly Ala Pro Leu Ala Ala Arg Ser Asp Leu
65                  70                  75                  80
Ser Thr Gly Asn Ser Ala His Ala Ser Leu Leu Leu Arg Ser Ala Ser
                85                  90                  95
Val Ala Phe Leu Phe Thr Ala Pro Tyr Gly Gly Asp His Gly Val Gly
            100                 105                 110
Ala Asp Ala Ala Thr Thr Ala Ser Ile Pro Ser Phe Ser Pro Gly Ala
        115                 120                 125
Ala Arg Arg Phe Ala Ala Asp His Gly Leu Ala Val His Ala Val Ala
    130                 135                 140
Leu Arg Val Ala Asp Ala Ala Asp Ala Phe Arg Ala Ser Val Ala Ala
145                 150                 155                 160
Gly Ala Arg Pro Ala Phe Gln Pro Ala Asp Leu Gly Gly Gly Phe Gly
                165                 170                 175
Leu Ala Glu Val Glu Leu Tyr Gly Asp Val Val Leu Arg Phe Val Ser
            180                 185                 190
His Pro Asp Gly Ala Asp Ala Pro Phe Leu Pro Gly Phe Glu Gly Val
        195                 200                 205
Ser Asn Pro Gly Ala Val Asp Tyr Gly Leu Arg Arg Phe Asp His Val
    210                 215                 220
Val Gly Asn Val Pro Glu Leu Ala Pro Val Ala Ala Tyr Ile Ser Gly
225                 230                 235                 240
Phe Thr Gly Phe His Glu Phe Ala Glu Phe Thr Ala Glu Asp Val Gly
                245                 250                 255
Thr Ala Glu Ser Gly Leu Asn Ser Val Val Leu Ala Asn Asn Ala Glu
            260                 265                 270
Thr Val Leu Leu Pro Leu Asn Glu Pro Val His Gly Thr Lys Arg Arg
        275                 280                 285
Ser Gln Ile Gln Thr Tyr Leu Asp His His Gly Gly Pro Gly Val Gln
    290                 295                 300
His Ile Ala Leu Ala Ser Asp Asp Val Leu Gly Thr Leu Arg Glu Met
305                 310                 315                 320
Arg Ala Arg Ser Ala Met Gly Gly Phe Glu Phe Leu Ala Pro Pro Pro
                325                 330                 335
Pro Asn Tyr Tyr Asp Gly Val Arg Arg Arg Ala Gly Asp Val Leu Ser
            340                 345                 350
Glu Glu Gln Ile Asn Glu Cys Gln Glu Leu Gly Val Leu Val Asp Arg
        355                 360                 365
Asp Asp Gln Gly Val Leu Leu Gln Ile Phe Thr Lys Pro Val Gly Asp
    370                 375                 380
Arg Pro Thr Phe Phe Leu Glu Met Ile Gln Arg Ile Gly Cys Met Glu

385                 390                 395                 400
Lys Asp Glu Ser Gly Gln Glu Tyr Gln Lys Gly Gly Cys Gly Gly Phe
                405                 410                 415
Gly Lys Gly Asn Phe Ser Glu Leu Phe Lys Ser Ile Glu Glu Tyr Glu
            420                 425                 430
Lys Ser Leu Glu Ala Lys Gln Ala Pro Thr Val Gln Gly Ser
        435                 440                 445

<210>  6
<211>  2099
<212>  DNA
<213>  Oryza sativa L. HPPD genome DNA sequence (DNA sequence)

<400>  6
atgcctccca ctcccacccc caccgccacc accggcgccg tctcggccgc tgcggcggcg      60
ggggagaacg cggggttccg cctcgtcggg caccgccgct tcgtccgcgc caacccgcgg     120
agcgaccggt tccaggcgct cgcgttccac cacgtcgagc tctggtgcgc cgacgccgcg     180
tccgccgcgg gccggttcgc cttcgccctg ggcgcgccgc tcgccgccag gtccgacctc     240
tccacgggga actccgcgca cgcctccctc ctcctccgct ccgcctccgt cgcgttcctc     300
ttcaccgccc cctacggcgg cgaccacggc gtcggcgcgg acgcggccac caccgcctcc     360
atcccttcct tctccccagg cgccgcgcgg aggttcgccg cggaccacgg cctcgcggtg     420
cacgccgtgg cgctgcgcgt cgccgacgcg gccgacgcct tccgcgccag cgtcgcggcc     480
ggtgcgcgcc cggcgttcca gcccgccgac ctcggcggtg gcttcggcct cgcggaggtg     540
gagctctacg gcgacgtcgt gctccgcttc gtcagccacc cggacggcgc cgacgcgccc     600
ttcctcccgg gtttcgaggg cgtcagcaac ccgggcgccg tggactacgg cctccgccgg     660
ttcgaccacg tcgtcggcaa cgtgccggag ctcgctccgg tagccgcgta catctccggg     720
ttcaccgggt tccacgagtt cgccgagttc accgccgagg acgtgggcac cgccgagagc     780
ggcctcaact cggtggtgct cgccaacaac gcggagaccg tgctgctgcc gctcaacgag     840
ccggtgcacg gcaccaagcg gcggagccag atacagacgt acctggacca ccacggcggc     900
ccgggggtgc agcacatcgc gctggccagc gacgacgtgc tcgggacgct gagggagatg     960
cgggcgcgct ccgccatggg cggcttcgag ttcttggcgc cgccgccgcc caactactac    1020
gacggcgtgc ggcggcgcgc cggggacgtg ctctcggagg agcagatcaa cgagtgccag    1080
gagctcgggg tgctcgtgga cagggatgac cagggggtgt tgctccagat cttcaccaag    1140
ccagtaggag acaggtaaaa tcctcacctc tttcatgatg aaaatggctt atgaattcag    1200
atttgcagtt atttgttggc acatagcatc gattaggcgc agaaaggtgt caagcattat    1260
gaaattaatc cagaatgctt gaataataca gtataatata tgatagtgag ctctgtgata    1320
ctccatggat actctttatg tgtctccatg aatccatgat gcgcctttct gaagattgtg    1380
acactagaaa gggaataaag ctgaatgtgc ataggaaaaa aatgaaaagc caatgtgtgt    1440
ctgtttatgc cttcttgcaa gcatatccca gttccttttt gccggcatgt tgtaatgcag    1500
atagccagcc acatatagct acttaattag tgagtactcc ctctcacaat gtaagtcatt    1560
ctagtatttt ccacattcat attgatgcta atctatctag attcattagc atcaatatga    1620
atatgggaaa tactagaatg acttacattg tgaaacggag gaagtattac ttactacatc    1680
taaggtccat ggattccttt ttttacaaaa gaaagaaaga atcttatggc aactccatca    1740
gcataaacca gcaatgctgc tgggaacaac ttaaacttta ggttcaggag gttgtaattg    1800
tctttaagct taatagtctg attcagtcag tattctaatt tctgctgcat ctttgctatt    1860
gttatttcct ctctgtgact ccaaatctaa ctggatcagc tatttcactc aggccaacct    1920
ttttcttgga gatgatacaa aggattgggt gcatggagaa ggatgagagt gggcaggagt    1980
accagaaggg cggctgcggc gggtttggga agggcaactt ctcggagctg ttcaagtcca    2040
ttgaggagta tgagaaatcc cttgaagcca agcaagcccc tacagttcaa ggatcctag     2099

<210>  7
<211>  536
<212>  PRT
<213>  Oryza sativa L. PPO1 protein amino acid sequence (amino acid sequence)

<400>  7
Met Ala Ala Ala Ala Ala Ala Met Ala Thr Ala Thr Ser Ala Thr Ala
1               5                   10                  15
Ala Pro Pro Leu Arg Ile Arg Asp Ala Ala Arg Arg Thr Arg Arg Arg
            20                  25                  30
Gly His Val Arg Cys Ala Val Ala Ser Gly Ala Ala Glu Ala Pro Ala
        35                  40                  45
Ala Pro Gly Ala Arg Val Ser Ala Asp Cys Val Val Val Gly Gly Gly

50                  55                  60
Ile Ser Gly Leu Cys Thr Ala Gln Ala Leu Ala Thr Lys His Gly Val
65                  70                  75                  80
Gly Asp Val Leu Val Thr Glu Ala Arg Ala Arg Pro Gly Gly Asn Ile
                85                  90                  95
Thr Thr Ala Glu Arg Ala Gly Glu Gly Tyr Leu Trp Glu Glu Gly Pro
            100                 105                 110
Asn Ser Phe Gln Pro Ser Asp Pro Val Leu Thr Met Ala Val Asp Ser
        115                 120                 125
Gly Leu Lys Asp Asp Leu Val Phe Gly Asp Pro Asn Ala Pro Arg Phe
    130                 135                 140
Val Leu Trp Glu Gly Lys Leu Arg Pro Val Pro Ser Lys Pro Gly Asp
145                 150                 155                 160
Leu Pro Phe Phe Asp Leu Met Ser Ile Pro Gly Lys Leu Arg Ala Gly
                165                 170                 175
Leu Gly Ala Leu Gly Val Arg Ala Pro Pro Pro Gly Arg Glu Glu Ser
            180                 185                 190
Val Glu Asp Phe Val Arg Arg Asn Leu Gly Ala Glu Val Phe Glu Arg
        195                 200                 205
Leu Ile Glu Pro Phe Cys Ser Gly Val Tyr Ala Gly Asp Pro Ser Lys
    210                 215                 220
Leu Ser Met Lys Ala Ala Phe Gly Lys Val Trp Arg Leu Glu Asp Thr
225                 230                 235                 240
Gly Gly Ser Ile Ile Gly Gly Thr Ile Lys Thr Ile Gln Glu Arg Gly
                245                 250                 255
Lys Asn Pro Lys Pro Pro Arg Asp Pro Arg Leu Pro Thr Pro Lys Gly
            260                 265                 270
Gln Thr Val Ala Ser Phe Arg Lys Gly Leu Thr Met Leu Pro Asp Ala
        275                 280                 285
Ile Thr Ser Arg Leu Gly Ser Lys Val Lys Leu Ser Trp Lys Leu Thr
    290                 295                 300
Ser Ile Thr Lys Ser Asp Asn Lys Gly Tyr Ala Leu Val Tyr Glu Thr
305                 310                 315                 320
Pro Glu Gly Val Val Ser Val Gln Ala Lys Thr Val Val Met Thr Ile
                325                 330                 335
Pro Ser Tyr Val Ala Ser Asp Ile Leu Arg Pro Leu Ser Ser Asp Ala
            340                 345                 350
Ala Asp Ala Leu Ser Ile Phe Tyr Tyr Pro Pro Val Ala Ala Val Thr
        355                 360                 365
Val Ser Tyr Pro Lys Glu Ala Ile Arg Lys Glu Cys Leu Ile Asp Gly
    370                 375                 380
Glu Leu Gln Gly Phe Gly Gln Leu His Pro Arg Ser Gln Gly Val Glu
385                 390                 395                 400
Thr Leu Gly Thr Ile Tyr Ser Ser Ser Leu Phe Pro Asn Arg Ala Pro
                405                 410                 415
Ala Gly Arg Val Leu Leu Leu Asn Tyr Ile Gly Gly Ser Thr Asn Thr
            420                 425                 430
Gly Ile Val Ser Lys Thr Glu Ser Glu Leu Val Glu Ala Val Asp Arg
        435                 440                 445
Asp Leu Arg Lys Met Leu Ile Asn Pro Lys Ala Val Asp Pro Leu Val
    450                 455                 460
Leu Gly Val Arg Val Trp Pro Gln Ala Ile Pro Gln Phe Leu Ile Gly
465                 470                 475                 480
His Leu Asp His Leu Glu Ala Ala Lys Ser Ala Leu Gly Lys Gly Gly
                485                 490                 495
Tyr Asp Gly Leu Phe Leu Gly Gly Asn Tyr Val Ala Gly Val Ala Leu
            500                 505                 510
Gly Arg Cys Val Glu Gly Ala Tyr Glu Ser Ala Ser Gln Ile Ser Asp
        515                 520                 525
Tyr Leu Thr Lys Tyr Ala Tyr Lys
    530                 535

<210>  8

<211>  3246
<212>  DNA
<213>  Oryza sativa L. PPO1 genome DNA sequence (DNA sequence)

<400>  8
atggccgccg ccgccgcagc catggccacc gccacctccg ccacggcagc gccgccgctc      60
cgcattcgcg acgccgcgag gaggacccgc cgacgcggcc acgttcgctg cgccgtcgcc     120
agcggcgcgg ccgaggcgcc cgcggcgccc ggggcgcggg tgtcggcgga ctgcgtcgtg     180
gtgggcggcg gcatcagcgg gctctgcacc gcgcaggcgc tggccacaaa gcacggcgtc     240
ggcgacgtgc tcgtcacgga ggcccgcgcc cgccccggcg gcaacatcac caccgccgag     300
cgcgccggcg agggctacct ctgggaggag gggcccaaca gcttccagcc ttccgacccc     360
gtcctcacca tggccgtacg tcttcttgct ccccttctct tctgattctc tcgcggcgga     420
gacgacatga atgggaatgg tggtgcatgg attggggcgc gcaggtggac agcgggctca     480
aggacgatct cgtgttcggg gaccccaacg cgccgcggtt cgtgctgtgg gaggggaagc     540
taaggccggt gccgtccaag cccggcgacc tgccgttctt cgacctcatg agcatccccg     600
gcaagctcag ggccggcctt ggcgcgctcg gcgttcgagc gccacctcca gtttgtgtgc     660
tctccccgct gtgcattctt gattcacttg tgaaattcga ttgtgctgag cgtttccggc     720
gaaggtttca ggggcgtgag gagtcggtgg aggacttcgt gcggcgcaac ctcggcgcgg     780
aggtctttga gcgcctcatt gagcctttct gctcaggtgt ttattgtagt gtgcaattgc     840
tgttttgttt ttgatgattc agataagaat acggtgattt cggtgcttag gtgtgtatgc     900
tggtgatcct tcaaagctca gtatgaaggc tgcatttggg aaggtgtgga ggctggagga     960
tactggaggt agcattattg gtggaaccat caaaacaatc caggagaggg ggaaaaaccc    1020
caaaccgccg agggatccgt gagtgagaaa ttgccttctt tgttggatta attgtccatt    1080
gtgttacact gatatgcctt caccattttt agccgccttc caacgccaaa ggggcagaca    1140
gttgcatctt tcaggaaggg tctgactatg ctcccggatg ctattacatc taggtttgtt    1200
atcattgtct ttgtaattta cctagttctt caactatgga tattaggtgc tgtagattgt    1260
tcagatagat gcacattgta caacaatcta ggtagattga ttgctatggc ttgttgaatt    1320
aactgtttca cttgcatcat tgcctcagca catatgaagc atatggatag attcttcaat    1380
catttatccc tcaataacac aattttgaca ccagtgcttc tccttttttt catcctgctt    1440
catctggcta tccacaatat aattaagcat acaaaagagg cactctttga tggacaattc    1500
atagtgttgt gggttaatat tcatttgcat tctttgaggg acaattcgtt acaccctaac    1560
atgaactagt aatgattggg gtgcttaacc atttgttctg catttcctcc attttcaggt    1620
tgggtagcaa agtcaaactt tcatggaagt tgacaagcat tacaaagtca gacaacaaag    1680
gatatgcatt agtgtatgaa acaccagaag gggtggtctc ggtgcaagct aaaactgttg    1740
tcatgaccat cccatcatat gttgctagtg atatcttgcg gccactttca gtaagttata    1800
tatatttaat taactttctg ttcccaaaat acactgcagc acttcattgc ttcctgaggt    1860
cctcgattca tttttcggta gacaggaagt agtattcatt tgcacttttt aagggattaa    1920
ttcaacatat ccactggaaa tatacatatc ctacacatcc tgtcaacata cttgctaaac    1980
agcattttgt ttgagttgac tggcatctca gcagccaatt actatcttta ggggacaagc    2040
cacattctta ataaatcctg tcggaaatca ctttttgatt tttatagatt agtgttgtca    2100
tagaatttgg cttggtgatc tacttggtaa ggttaactga ttcacaagtc ggacaatttc    2160
ttcaccaatc tagcagtatg tttaagtgtg ttggtactta attctaaatg tcctgcgcat    2220
ggtaacatat catatgcaaa aattcctcag taaccgaaat ttatactgta agttttaact    2280
gtctttacac tgttaatttt agacatactt cttccttgct tgttcattga acttgtttcc    2340
cccttccaca gagtgatgca gcagatgctc tgtcaatatt ctattatcca ccagttgctg    2400
ctgtaactgt ttcatatcca aaagaagcaa ttagaaaaga atgcttaatt gacggagagc    2460
tccagggttt cggccagctg catccgcgta gtcagggagt tgagacttta ggtacttata    2520
ggaattcaac cttattattc ttctaacata taaatgaact aatctttctt gtctagtttg    2580
catttattgt ggattaagtt tggttatatt gttcttacaa gtttgtggta ttattttgta    2640
taggaacaat atatagctca tcactctttc caaatcgtgc tccagctgga agggtgttac    2700
ttctgaacta cataggaggt tctacaaata cagggattgt ttccaaggta tcgctgtcaa    2760
gttgtttatt ttgcgactat atgattacag tatcctgttt ttcaactcca gctgctgtta    2820
gactgtcata ataaatctgc tactacatgt ttgcacacta tttgactgca tttaaaaact    2880
cagatagcct atatttttag ttgcctgcta ctgggtgtat ttctaatgat cccatcatgt    2940
ttgcagactg aaagtgagct ggtagaagca gttgaccgtg acctcaggaa gatgctgata    3000
aatcctaaag cagtggaccc tttggtcctt ggcgtccggg tatggccaca agccatacca    3060
cagttcctca ttggccatct tgatcatctt gaggctgcaa aatctgccct gggcaaaggt    3120
ggttatgatg gattgttcct cggagggaac tatgttgcag gagttgccct gggccgatgc    3180
gttgaaggtg catatgagag tgcctcacaa atatctgact acttgaccaa gtacgcctac    3240
aagtga                                                                3246

<210>  9
<211>  587

<212>  PRT
<213>  Oryza sativa L. TIR1 protein amino acid sequence (amino acid sequence)

<400>  9
Met Gly Arg Gly Gly Ser Arg Ala Ala Cys Ala Ala Ala Ala Pro Pro
1               5                   10                  15
Trp His Ser Leu Pro Asp Glu Val Trp Glu His Ala Phe Ser Phe Leu
            20                  25                  30
Pro Ala Ala Ala Asp Arg Gly Ala Ala Ala Gly Ala Cys Ser Ser Trp
        35                  40                  45
Leu Arg Ala Glu Arg Arg Ser Arg Arg Arg Leu Ala Val Ala Asn Cys
    50                  55                  60
Tyr Ala Ala Ala Pro Arg Asp Ala Val Glu Arg Phe Pro Ser Val Arg
65                  70                  75                  80
Ala Ala Glu Val Lys Gly Lys Pro His Phe Ala Asp Phe Gly Leu Val
                85                  90                  95
Pro Pro Ala Trp Gly Ala Ala Ala Ala Pro Trp Ile Ala Ala Ala Ala
            100                 105                 110
Asp Gly Trp Pro Leu Leu Glu Glu Leu Ser Phe Lys Arg Met Val Val
        115                 120                 125
Thr Asp Glu Cys Leu Glu Met Ile Ala Ala Ser Phe Arg Asn Phe Gln
    130                 135                 140
Val Leu Arg Leu Val Ser Cys Asp Gly Phe Ser Thr Ala Gly Leu Ala
145                 150                 155                 160
Ala Ile Ala Ala Gly Cys Arg His Leu Arg Glu Leu Asp Leu Gln Glu
                165                 170                 175
Asn Glu Ile Glu Asp Cys Ser Ile His Trp Leu Ser Leu Phe Pro Glu
            180                 185                 190
Ser Phe Thr Ser Leu Val Thr Leu Asn Phe Ser Cys Leu Glu Gly Glu
        195                 200                 205
Val Asn Ile Thr Val Leu Glu Arg Leu Val Thr Arg Cys His Asn Leu
    210                 215                 220
Lys Thr Leu Lys Leu Asn Asn Ala Ile Pro Leu Asp Lys Leu Ala Ser
225                 230                 235                 240
Leu Leu His Lys Ala Pro Gln Leu Val Glu Leu Gly Thr Gly Lys Phe
                245                 250                 255
Ser Ala Asp Tyr His Ser Asp Leu Phe Ala Lys Leu Glu Ala Ala Phe
            260                 265                 270
Gly Gly Cys Lys Ser Leu Arg Arg Leu Ser Gly Ala Trp Asp Ala Val
        275                 280                 285
Pro Asp Tyr Leu Pro Ala Phe Tyr Cys Val Cys Glu Gly Leu Thr Ser
    290                 295                 300
Leu Asn Leu Ser Tyr Ala Thr Val Arg Gly Pro Glu Leu Ile Lys Phe
305                 310                 315                 320
Ile Ser Arg Cys Arg Asn Leu Gln Gln Leu Trp Val Met Asp Leu Ile
                325                 330                 335
Glu Asp His Gly Leu Ala Val Val Ala Ser Ser Cys Asn Lys Leu Gln
            340                 345                 350
Glu Leu Arg Val Phe Pro Ser Asp Pro Phe Gly Ala Gly Phe Leu Thr
        355                 360                 365
Glu Arg Gly Leu Val Asp Val Ser Ala Ser Cys Pro Met Leu Glu Ser
    370                 375                 380
Val Leu Tyr Phe Cys Arg Arg Met Thr Asn Glu Ala Leu Ile Thr Ile
385                 390                 395                 400
Ala Lys Asn Arg Pro Asn Phe Thr Cys Phe Arg Leu Cys Ile Leu Glu
                405                 410                 415
Pro His Thr Pro Asp Tyr Ile Thr Arg Glu Pro Leu Asp Ala Gly Phe
            420                 425                 430
Ser Ala Ile Val Glu Ser Cys Arg Gly Leu Arg Arg Leu Ser Ile Ser
        435                 440                 445
Gly Leu Leu Thr Asp Leu Val Phe Lys Ser Ile Gly Ala His Ala Asp
    450                 455                 460
Arg Leu Glu Met Leu Ser Ile Ala Phe Ala Gly Asn Ser Asp Leu Gly

465                 470                 475                 480
Leu His Tyr Ile Leu Ser Gly Cys Lys Ser Leu Lys Lys Leu Glu Ile
                485                 490                 495
Arg Asp Cys Pro Phe Gly Asp Lys Pro Leu Leu Ala Asn Ala Ala Lys
            500                 505                 510
Leu Glu Thr Met Arg Ser Leu Trp Met Ser Ser Cys Leu Leu Thr Leu
        515                 520                 525
Gly Ala Cys Arg Gln Leu Ala Arg Lys Met Pro Arg Leu Ser Val Glu
    530                 535                 540
Ile Met Asn Asp Pro Gly Arg Ser Cys Pro Leu Asp Ser Leu Pro Asp
545                 550                 555                 560
Glu Thr Pro Val Glu Lys Leu Tyr Val Tyr Arg Thr Ile Ala Gly Pro
                565                 570                 575
Arg Ser Asp Thr Pro Ala Cys Val Gln Ile Val
            580                 585

<210>  10
<211>  5791
<212>  DNA
<213>  Oryza sativa L. TIR1 genome DNA sequence (DNA sequence)

<400>  10
atggggcgcg gcggctcgcg cgcggcgtgc gccgccgcgg cgccgccgtg gcactcgctc      60
ccggacgagg tctgggagca cgccttctcc ttcctccccg ccgccgcgga caggggcgcc     120
gcggcggggg cgtgcagctc gtggctccgc gccgagcgcc ggtcgcgccg ccgcctcgcc     180
gtcgccaact gctacgccgc cgcgccgcgg gacgccgtcg agcggttccc gtccgtgcgc     240
gccgccgagg tcaagggcaa gccccacttc gccgacttcg gcctcgtccc ccccgcctgg     300
ggcgccgccg cggcgccgtg gatcgccgcc gccgccgacg ggtggccgct gctcgaggag     360
ctcagcttca agcgcatggt cgtcaccgac gagtgcctcg agatgatcgc ccgcgtcctc     420
aggaacttcc aggtgctccg cctcgtctcc tgcgacggct tcagcaccgc gggcctcgcc     480
gccattgctg ccggttgcag gtgaggtgga gagtgttgtt tgggtcaaat ctttttagct     540
aaataagcat gggatctttg ggttgttctt ggatttgaga ggaatctttc acggcccaag     600
ctttgattcg tcttcttttt agctaaaaca tgacagattt cttggtgcca tttctttctt     660
cacagtgttc tttagtgagt tcatggtcta gctgaagtga tttgttcttc catgattcct     720
atattaactt tgttttgaac ttcattgagt agtatgtgga agtagatgtg gctttctgct     780
aagatttgat ctattcttgg tgtttaattt gcaaaggaac agtttttttt tctgtttact     840
tgtgtatgat ctggatttac ttttggtgtt tcctaagttg aagaccaaga agtcttcttg     900
tgacagtatt ttagatgctt ataacgtgtt aatgtcaaag gaatgattcc catgaaaatt     960
ctgctgtgat ttgtacattt tattaggagt taagtaacag agtgaattga gtgatgctca    1020
cgtcttcttt aaatcttagc tatgatttgt tcctaggaag ctttgacttg tatttgagtg    1080
cctggtttgc gaagatgtga ttctcttgca cggatctgtg tgattgacag aagaaatatt    1140
tctacctgtt taagcaatga tggatccatc tctttgtttc tttttgtgct ttttacccaa    1200
aagaatggga tatttcaagt ttacaaacaa ctggtgatgc gcctttataa ttttgggctt    1260
ttttaaaaga atgtattctt cccatcatga gttactagat ctgagttgaa ctgttttcct    1320
ttatattttc ctgatattta agtgaatttg ttgagttcct atcctgattt gaacttccag    1380
attctaaaga accaggtctt ttaagcatgt ttttgtcatg tagattccca gtttatcccc    1440
tccccctcct tttgtgctca agaatctact accttttatc ttgcttttgg tgagggaaag    1500
tgatttttgc tccaatattt gtgatgtcct catgtaatgg acagttaaaa caaacagatt    1560
tgttcttgcg agtatcttct ggactccccc cagaaaagaa ttaggaatgt gtgggattcc    1620
tatcattggg gaatttatat cctaaaccag atagaaggat ctaatagtgg gagtttcgat    1680
cctgattcat ccattagtag tagtttaata tgccaaagca gtatcatttg catcattttg    1740
aggaactcga aacagttgaa ctgtgaagtt taactactgt acacttcaaa caggcagatt    1800
agaataggtt gtgagataac taaatttggt tcggtgatcg tataaggcca tattcttgga    1860
tattaggacg aaatgagcct gagcaccaca tgtgtacttg ctgatggttg agaacttagc    1920
atagatacta gtttttatgc tagactagag ggctactggt acattcactt gtttgataaa    1980
tccagatgta tttaagtttt gattcaaaat attttgactc tagattggag actacatttt    2040
agagcattca aaatttacaa tggaagcagg attttagcaa aactattttc atacatttac    2100
tctgttattg agccagaatc attttttagt tcttgctact tttttgttaa cactgttatg    2160
catgtaaact cagcgttaca tttatttcaa ttacaaggat ctacacttgt agctctgggt    2220
gcagatggta gttaacattg atgaatctta gctatatcga aatactatct tctaagtaca    2280
aacttttctt gtgtgattct tatccttgtt gttactgttg ctacttatat ccattgattg    2340
agcatgcttt gaattacctt aagaatttct ctctccagtc aaatttattt gactttgaga    2400
gaactgtgtt tatttctccc aacgtcaaat gttttgaatt ttgaatggac agagtatgta    2460

gtagtgggca gcacattggt gccctagctg caaataaaac cttttttttt tgttaaaaca 2520
atgattatga ggatgtcggt ttacactgtc cactcagaaa tttgacttga ttatggatag 2580
ggtacacctt gttgttttag atggttcctt cttaacaaga tgctagattt tgatgatttg 2640
attctttaca aggagtagtc taaaatactg gaagtgtcaa cttaaaaagt tcaaacttgc 2700
cctagccata cgtgatacat tagacaactg atgtgctgtg ttatctgcaa ctgacaatta 2760
ttgttgttgc tatctccttt ttttgcttta gagtaggcag gtattttgtg tcaactaaaa 2820
gtgtgtttca cgtgttgcct ttcttaatac cttttgaata atagggtata agaaaatttt 2880
catgaatggt ttgctttcca attgttgatg ttgctttcct tcaccttatt ttttaaacca 2940
ccatgattaa tctatttatc taggtaatat tgctcatgct gattcatgat acgtggtaaa 3000
aaaataggtc ttttcaactt tcagaggctg ctggactcca tttatttagt tgattttgat 3060
ccctacaccg atttatccca ttggtttata ttcactgcta acatatgata ctcgctatca 3120
tttacttcac tgttgatcat caatgtttta attttgacca ttattaactt ttatatgatt 3180
aatttagtat taatgaaagt tgtgcggttg tatttatggc atgacacaca tttctaacat 3240
ggcatacttt taagcataca taactttttt tgtaaaaagg acaagcgatc aaaggtaaaa 3300
aggtgaatag catacagtga gaagtatttg agaccaaagg gagtataatc attatctact 3360
aaacaaatac aataccattc attataagct gagttcgcat agtgtaattg aaagtgggga 3420
atgctagtat agctcacaca tgaaatcaag tccctgtgaa atgatagtaa accctacgtg 3480
ttcattgttt tagtcttatg atatgttcca gttatgtttg ccatggttgt tgaatgcagt 3540
agtcaaagtt tgcttctgtt tgtttgagtg gattctcatt taccttgcct tttccaccat 3600
atactcatat gggctttgtt tcacaagtga taacattggt taatggctat atgctatcct 3660
tacagtttca ttcttctttt aagatatgag tcaccatgca catttcttca acttctgatt 3720
ttgtctaata ttgcagacac ctaagagaac ttgacctgca agagaacgag attgaggatt 3780
gttctattca ttggctcagc ctcttcccgg aatcgttcac ttctctagta actctaaact 3840
tttcatgctt agagggggag gtcaatatca ctgtacttga acggttagtg accagatgtc 3900
acaacctgaa gactcttaag ctcaacaatg ctatccccct tgacaagctt gctagcctcc 3960
ttcataaggc tcctcagcta gttgaactcg gaactggcaa attctctgct gattaccatt 4020
ccgatctgtt tgcaaagctg gaggcggcgt ttggaggttg taaaagcttg agaaggcttt 4080
ctggggcttg ggatgctgtt ccagattatc tgccagcatt ctattgtgta tgtgaaggcc 4140
tcacatcact taatctgagt tatgctactg tgcgaggtcc tgagctcatc aaattcatta 4200
gtagatgcag aaatttgcaa caattatggg tatgcaactc ttgtcaattc ttttttacta 4260
ctatgtattc atcccattcg aatctgttaa cttttgaaaa actgttgcat atctctttgc 4320
aatacaagca cttgattccc tacaagtgga ttttgcaaat tttttctttt cttctgtgac 4380
ctccagtaaa aaagttcctg atgttttctt acttatcatg gtcactagta aaagtccgac 4440
tacatgtatt cttttttcac aatgtagttt tatcaattat attggtatag ggctgcaata 4500
gcattttatg atgaaactga ctgcactttt cccatgacaa caagaatttg tggcttggaa 4560
taatctattg agtgttaaat catgcatatg tttttcatgt ttgtcaatac agttttgtag 4620
gatgcatgtt acattcaatg gcatgctgtt gggtgcctcc aatgaataga ttgaaattac 4680
cactcttatc ttttggggaa gtaggatatt tatatggaaa aaacatagat gacaatggta 4740
tcctatcctt attagaatca ttgtgaaaag actgccagat gaatccattt gctctagggc 4800
agcattcttt gcctttcttg gcaaactgag ttattgtctg aaccctcata gtttccatcc 4860
tttaactgtt aactgctaga tgagttgctt gctctacggc agatatttta aactgaatta 4920
tgatctgaac cccaatggct attaacctgg tcatgttagc tctttatgca atcttttttc 4980
tctatatttt tcttctaaaa gttaagctgc aatttttcag gtgatggacc tcattgagga 5040
tcatggttta gctgttgtgg catcatcttg caataaactt caagagttgc gggtcttccc 5100
ttctgaccct tttggtgcag gattcttgac tgaaagaggt cttgttgatg tctctgcaag 5160
ttgtccaatg ttggagtcag tgctctactt ctgcagacgg atgacaaatg aggcacttat 5220
taccattgca aagaaccgtc ccaacttcac ttgcttccgc ctatgcatcc ttgagccaca 5280
cactccagac tacatcacac gggagcctct tgatgcaggt ttcagcgcca ttgtggagtc 5340
atgcaggggc cttaggcgtc tctctatctc aggccttctc acagatcttg tgtttaaatc 5400
cattggggca catgctgatc gtcttgagat gctttcaatc gccttcgctg ggaacagcga 5460
cttgggcctg cattacatcc tctcaggctg caagagcctg aagaaactgg agatcaggga 5520
ctgcccattt ggtgataagc cattgctggc gaacgcagca aagctggaga caatgcgatc 5580
cctttggatg tcgtcgtgct tgttgaccct gggcgcatgc cgacagcttg cacgcaagat 5640
gccccgcctt agtgtggaga tcatgaacga tcctggaagg tcatgcccct tggattcgct 5700
tccggatgaa acacctgttg agaaactgta cgtctaccgg acgatcgcag gtccaaggtc 5760
cgacacacca gcctgcgtcc agattgtgta g 5791

<210> 11
<211> 644
<212> PRT
<213> Oryza sativa L. ALS protein amino acid sequence (amino acid sequence)

<400> 11

Met Ala Thr Thr Ala Ala Ala Ala Ala Ala Thr Leu Ser Ala Ala Ala
1               5                   10                  15
Thr Ala Lys Thr Gly Arg Lys Asn His Gln Arg His His Val Leu Pro
            20                  25                  30
Ala Arg Gly Arg Val Gly Ala Ala Ala Val Arg Cys Ser Ala Val Ser
        35                  40                  45
Pro Val Thr Pro Pro Ser Pro Ala Pro Pro Ala Thr Pro Leu Arg Pro
    50                  55                  60
Trp Gly Pro Ala Glu Pro Arg Lys Gly Ala Asp Ile Leu Val Glu Ala
65                  70                  75                  80
Leu Glu Arg Cys Gly Val Ser Asp Val Phe Ala Tyr Pro Gly Gly Ala
                85                  90                  95
Ser Met Glu Ile His Gln Ala Leu Thr Arg Ser Pro Val Ile Thr Asn
            100                 105                 110
His Leu Phe Arg His Glu Gln Gly Glu Ala Phe Ala Ala Ser Gly Tyr
        115                 120                 125
Ala Arg Ala Ser Gly Arg Val Gly Val Cys Val Ala Thr Ser Gly Pro
    130                 135                 140
Gly Ala Thr Asn Leu Val Ser Ala Leu Ala Asp Ala Leu Leu Asp Ser
145                 150                 155                 160
Val Pro Met Val Ala Ile Thr Gly Gln Val Pro Arg Arg Met Ile Gly
                165                 170                 175
Thr Asp Ala Phe Gln Glu Thr Pro Ile Val Glu Val Thr Arg Ser Ile
            180                 185                 190
Thr Lys His Asn Tyr Leu Val Leu Asp Val Glu Asp Ile Pro Arg Val
        195                 200                 205
Ile Gln Glu Ala Phe Phe Leu Ala Ser Ser Gly Arg Pro Gly Pro Val
    210                 215                 220
Leu Val Asp Ile Pro Lys Asp Ile Gln Gln Gln Met Ala Val Pro Val
225                 230                 235                 240
Trp Asp Thr Ser Met Asn Leu Pro Gly Tyr Ile Ala Arg Leu Pro Lys
                245                 250                 255
Pro Pro Ala Thr Glu Leu Leu Glu Gln Val Leu Arg Leu Val Gly Glu
            260                 265                 270
Ser Arg Arg Pro Ile Leu Tyr Val Gly Gly Gly Cys Ser Ala Ser Gly
        275                 280                 285
Asp Glu Leu Arg Arg Phe Val Glu Leu Thr Gly Ile Pro Val Thr Thr
    290                 295                 300
Thr Leu Met Gly Leu Gly Asn Phe Pro Ser Asp Asp Pro Leu Ser Leu
305                 310                 315                 320
Arg Met Leu Gly Met His Gly Thr Val Tyr Ala Asn Tyr Ala Val Asp
                325                 330                 335
Lys Ala Asp Leu Leu Leu Ala Phe Gly Val Arg Phe Asp Asp Arg Val
            340                 345                 350
Thr Gly Lys Ile Glu Ala Phe Ala Ser Arg Ala Lys Ile Val His Ile
        355                 360                 365
Asp Ile Asp Pro Ala Glu Ile Gly Lys Asn Lys Gln Pro His Val Ser
    370                 375                 380
Ile Cys Ala Asp Val Lys Leu Ala Leu Gln Gly Leu Asn Ala Leu Leu
385                 390                 395                 400
Asp Gln Ser Thr Thr Lys Thr Ser Ser Asp Phe Ser Ala Trp His Asn
                405                 410                 415
Glu Leu Asp Gln Gln Lys Arg Glu Phe Pro Leu Gly Tyr Lys Thr Phe
            420                 425                 430
Gly Glu Glu Ile Pro Pro Gln Tyr Ala Ile Gln Val Leu Asp Glu Leu
        435                 440                 445
Thr Lys Gly Glu Ala Ile Ile Ala Thr Gly Val Gly Gln His Gln Met
    450                 455                 460
Trp Ala Ala Gln Tyr Tyr Thr Tyr Lys Arg Pro Arg Gln Trp Leu Ser
465                 470                 475                 480
Ser Ala Gly Leu Gly Ala Met Gly Phe Gly Leu Pro Ala Ala Ala Gly
                485                 490                 495
Ala Ser Val Ala Asn Pro Gly Val Thr Val Val Asp Ile Asp Gly Asp

                    500                     505                     510
Gly Ser Phe Leu Met Asn Ile Gln Glu Leu Ala Leu Ile Arg Ile Glu
        515                     520                     525
Asn Leu Pro Val Lys Val Met Val Leu Asn Asn Gln His Leu Gly Met
    530                     535                     540
Val Val Gln Trp Glu Asp Arg Phe Tyr Lys Ala Asn Arg Ala His Thr
545                     550                     555                     560
Tyr Leu Gly Asn Pro Glu Cys Glu Ser Glu Ile Tyr Pro Asp Phe Val
                565                     570                     575
Thr Ile Ala Lys Gly Phe Asn Ile Pro Ala Val Arg Val Thr Lys Lys
            580                     585                     590
Ser Glu Val Arg Ala Ala Ile Lys Lys Met Leu Glu Thr Pro Gly Pro
        595                     600                     605
Tyr Leu Leu Asp Ile Ile Val Pro His Gln Glu His Val Leu Pro Met
    610                     615                     620
Ile Pro Asn Gly Gly Ala Phe Lys Asp Met Ile Leu Asp Gly Asp Gly
625                     630                     635                     640
Arg Thr Met Tyr


<210>  12
<211>  1935
<212>  DNA
<213>  Oryza sativa L. ALS gene DNA sequence (DNA sequence)

<400>  12
atggctacga ccgccgcggc cgcggccgcc accttgtccg ccgccgcgac ggccaagacc      60
ggccgtaaga accaccagcg acaccacgtc cttcccgctc gaggccgggt gggggcggcg     120
gcggtcaggt gctcggcggt gtccccggtc accccgccgt ccccggcgcc gccggccacg     180
ccgctccggc cgtgggggcc ggccgagccc cgcaagggcg cggacatcct cgtggaggcg     240
ctggagcggt gcggcgtcag cgacgtgttc gcctacccgg gcggcgcgtc catggagatc     300
caccaggcgc tgacgcgctc cccggtcatc accaaccacc tcttccgcca cgagcagggc     360
gaggcgttcg cggcgtccgg gtacgcgcgc gcgtccggcc gcgtcggggt ctgcgtcgcc     420
acctccggcc ccggggcaac caacctcgtg tccgcgctcg ccgacgcgct gctcgactcc     480
gtcccgatgg tcgccatcac gggccaggtc ccccgccgca tgatcggcac cgacgccttc     540
caggagacgc ccatagtcga ggtcacccgc tccatcacca agcacaatta ccttgtcctt     600
gatgtggagg acatcccccg cgtcatacag gaagccttct tcctcgcgtc ctcgggccgt     660
cctggcccgg tgctggtcga catccccaag gacatccagc agcagatggc tgtgccagtc     720
tgggacacct cgatgaatct accggggtac attgcacgcc tgcccaagcc acccgcgaca     780
gaattgcttg agcaggtctt gcgtctggtt ggcgagtcac ggcgcccgat tctctatgtc     840
ggtggtggct gctctgcatc tggtgatgaa ttgcgccggt ttgttgagct gaccggcatc     900
ccagttacaa ccactctgat gggcctcggc aatttcccca gtgatgatcc gttgtccctg     960
cgcatgcttg ggatgcatgg cacggtgtac gcaaattatg cggtggataa ggctgacctg    1020
ttgcttgcat ttggcgtgcg gtttgatgat cgtgtgacag ggaaaattga ggcttttgca    1080
agcagggcca agattgtgca cattgacatt gatccagcgg agattggaaa gaacaagcaa    1140
ccacatgtgt caatttgcgc agatgttaag cttgctttac agggcttgaa tgctctgcta    1200
gaccagagca caacaaagac aagttctgat tttagtgcat ggcacaatga gttggaccag    1260
cagaagaggg agtttcctct ggggtacaag acttttggtg aagagatccc accgcaatat    1320
gctattcagg tgctggatga gctgacgaaa ggggaggcaa tcatcgctac tggtgttgga    1380
cagcaccaga tgtgggcggc acaatattac acctacaagc ggccacggca gtggctgtct    1440
tcggctggtc tgggcgcaat gggatttggg ctgcctgctg cagctggtgc ttctgtggct    1500
aacccaggtg tcacagttgt tgatattgat ggggatggta gcttcctcat gaacattcag    1560
gagttggcat tgatccgcat tgagaacctc ccggtgaagg tgatggtgtt gaacaaccaa    1620
catttgggta tggttgtgca atgggaggat aggttttaca aggcaaatag ggcgcataca    1680
tacttgggca acccagaatg tgagagcgag atatatccag attttgtgac tattgctaaa    1740
gggttcaata ttcctgcagt ccgtgtaaca aagaagagtg aagtccgtgc cgccatcaag    1800
aagatgctcg agaccccagg gccatacttg ttggatatca tcgtcccaca ccaggagcat    1860
gtgctgccta tgatcccaaa tgggggcgca ttcaaggaca tgatcctgga tggtgatggc    1920
aggactatgt attaa                                                     1935

<210>  13
<211>  2327
<212>  PRT

<213>  Oryza sativa L. ACCase2 protein amino acid sequence (amino acid
sequence)

<400>  13
Met Thr Ser Thr His Val Ala Thr Leu Gly Val Gly Ala Gln Ala Pro
1               5                   10                  15
Pro Arg His Gln Lys Lys Ser Ala Gly Thr Ala Phe Val Ser Ser Gly
            20                  25                  30
Ser Ser Arg Pro Ser Tyr Arg Lys Asn Gly Gln Arg Thr Arg Ser Leu
        35                  40                  45
Arg Glu Glu Ser Asn Gly Gly Val Ser Asp Ser Lys Lys Leu Asn His
    50                  55                  60
Ser Ile Arg Gln Gly Leu Ala Gly Ile Ile Asp Leu Pro Asn Asp Ala
65                  70                  75                  80
Ala Ser Glu Val Asp Ile Ser His Gly Ser Glu Asp Pro Arg Gly Pro
                85                  90                  95
Thr Val Pro Gly Ser Tyr Gln Met Asn Gly Ile Ile Asn Glu Thr His
            100                 105                 110
Asn Gly Arg His Ala Ser Val Ser Lys Val Val Glu Phe Cys Thr Ala
        115                 120                 125
Leu Gly Gly Lys Thr Pro Ile His Ser Val Leu Val Ala Asn Asn Gly
    130                 135                 140
Met Ala Ala Ala Lys Phe Met Arg Ser Val Arg Thr Trp Ala Asn Asp
145                 150                 155                 160
Thr Phe Gly Ser Glu Lys Ala Ile Gln Leu Ile Ala Met Ala Thr Pro
                165                 170                 175
Glu Asp Leu Arg Ile Asn Ala Glu His Ile Arg Ile Ala Asp Gln Phe
            180                 185                 190
Val Glu Val Pro Gly Gly Thr Asn Asn Asn Asn Tyr Ala Asn Val Gln
        195                 200                 205
Leu Ile Val Glu Ile Ala Glu Arg Thr Gly Val Ser Ala Val Trp Pro
    210                 215                 220
Gly Trp Gly His Ala Ser Glu Asn Pro Glu Leu Pro Asp Ala Leu Thr
225                 230                 235                 240
Ala Lys Gly Ile Val Phe Leu Gly Pro Pro Ala Ser Ser Met His Ala
                245                 250                 255
Leu Gly Asp Lys Val Gly Ser Ala Leu Ile Ala Gln Ala Ala Gly Val
            260                 265                 270
Pro Thr Leu Ala Trp Ser Gly Ser His Val Glu Val Pro Leu Glu Cys
        275                 280                 285
Cys Leu Asp Ser Ile Pro Asp Glu Met Tyr Arg Lys Ala Cys Val Thr
    290                 295                 300
Thr Thr Glu Glu Ala Val Ala Ser Cys Gln Val Val Gly Tyr Pro Ala
305                 310                 315                 320
Met Ile Lys Ala Ser Trp Gly Gly Gly Gly Lys Gly Ile Arg Lys Val
                325                 330                 335
His Asn Asp Asp Glu Val Arg Thr Leu Phe Lys Gln Val Gln Gly Glu
            340                 345                 350
Val Pro Gly Ser Pro Ile Phe Ile Met Arg Leu Ala Ala Gln Ser Arg
        355                 360                 365
His Leu Glu Val Gln Leu Leu Cys Asp Gln Tyr Gly Asn Val Ala Ala
    370                 375                 380
Leu His Ser Arg Asp Cys Ser Val Gln Arg Arg His Gln Lys Ile Ile
385                 390                 395                 400
Glu Glu Gly Pro Val Thr Val Ala Pro Arg Glu Thr Val Lys Glu Leu
                405                 410                 415
Glu Gln Ala Ala Arg Arg Leu Ala Lys Ala Val Gly Tyr Val Gly Ala
            420                 425                 430
Ala Thr Val Glu Tyr Leu Tyr Ser Met Glu Thr Gly Glu Tyr Tyr Phe
        435                 440                 445
Leu Glu Leu Asn Pro Arg Leu Gln Val Glu His Pro Val Thr Glu Trp
    450                 455                 460
Ile Ala Glu Val Asn Leu Pro Ala Ala Gln Val Ala Val Gly Met Gly

465                 470                 475                 480
Ile Pro Leu Trp Gln Ile Pro Glu Ile Arg Arg Phe Tyr Gly Met Asn
                485                 490                 495
His Gly Gly Gly Tyr Asp Leu Trp Arg Lys Thr Ala Ala Leu Ala Thr
            500                 505                 510
Pro Phe Asn Phe Asp Glu Val Asp Ser Lys Trp Pro Lys Gly His Cys
        515                 520                 525
Val Ala Val Arg Ile Thr Ser Glu Asp Pro Asp Asp Gly Phe Lys Pro
    530                 535                 540
Thr Gly Gly Lys Val Lys Glu Ile Ser Phe Lys Ser Lys Pro Asn Val
545                 550                 555                 560
Trp Ala Tyr Phe Ser Val Lys Ser Gly Gly Gly Ile His Glu Phe Ala
                565                 570                 575
Asp Ser Gln Phe Gly His Val Phe Ala Tyr Gly Thr Thr Arg Ser Ala
            580                 585                 590
Ala Ile Thr Thr Met Ala Leu Ala Leu Lys Glu Val Gln Ile Arg Gly
        595                 600                 605
Glu Ile His Ser Asn Val Asp Tyr Thr Val Asp Leu Leu Asn Ala Ser
    610                 615                 620
Asp Phe Arg Glu Asn Lys Ile His Thr Gly Trp Leu Asp Thr Arg Ile
625                 630                 635                 640
Ala Met Arg Val Gln Ala Glu Arg Pro Pro Trp Tyr Ile Ser Val Val
                645                 650                 655
Gly Gly Ala Leu Tyr Lys Thr Val Thr Ala Asn Thr Ala Thr Val Ser
            660                 665                 670
Asp Tyr Val Gly Tyr Leu Thr Lys Gly Gln Ile Pro Pro Lys His Ile
        675                 680                 685
Ser Leu Val Tyr Thr Thr Val Ala Leu Asn Ile Asp Gly Lys Lys Tyr
    690                 695                 700
Thr Ile Asp Thr Val Arg Ser Gly His Gly Ser Tyr Arg Leu Arg Met
705                 710                 715                 720
Asn Gly Ser Thr Val Asp Ala Asn Val Gln Ile Leu Cys Asp Gly Gly
                725                 730                 735
Leu Leu Met Gln Leu Asp Gly Asn Ser His Val Ile Tyr Ala Glu Glu
            740                 745                 750
Glu Ala Ser Gly Thr Arg Leu Leu Ile Asp Gly Lys Thr Cys Met Leu
        755                 760                 765
Gln Asn Asp His Asp Pro Ser Lys Leu Leu Ala Glu Thr Pro Cys Lys
    770                 775                 780
Leu Leu Arg Phe Leu Val Ala Asp Gly Ala His Val Asp Ala Asp Val
785                 790                 795                 800
Pro Tyr Ala Glu Val Glu Val Met Lys Met Cys Met Pro Leu Leu Ser
                805                 810                 815
Pro Ala Ser Gly Val Ile His Val Val Met Ser Glu Gly Gln Ala Met
            820                 825                 830
Gln Ala Gly Asp Leu Ile Ala Arg Leu Asp Leu Asp Asp Pro Ser Ala
        835                 840                 845
Val Lys Arg Ala Glu Pro Phe Glu Asp Thr Phe Pro Gln Met Gly Leu
    850                 855                 860
Pro Ile Ala Ala Ser Gly Gln Val His Lys Leu Cys Ala Ala Ser Leu
865                 870                 875                 880
Asn Ala Cys Arg Met Ile Leu Ala Gly Tyr Glu His Asp Ile Asp Lys
                885                 890                 895
Val Val Pro Glu Leu Val Tyr Cys Leu Asp Thr Pro Glu Leu Pro Phe
            900                 905                 910
Leu Gln Trp Glu Glu Leu Met Ser Val Leu Ala Thr Arg Leu Pro Arg
        915                 920                 925
Asn Leu Lys Ser Glu Leu Glu Gly Lys Tyr Glu Glu Tyr Lys Val Lys
    930                 935                 940
Phe Asp Ser Gly Ile Ile Asn Asp Phe Pro Ala Asn Met Leu Arg Val
945                 950                 955                 960
Ile Ile Glu Glu Asn Leu Ala Cys Gly Ser Glu Lys Glu Lys Ala Thr
                965                 970                 975

Asn Glu Arg Leu Val Glu Pro Leu Met Ser Leu Leu Lys Ser Tyr Glu
            980                 985                 990
Gly Gly Arg Glu Ser His Ala His Phe Val Val Lys Ser Leu Phe Glu
        995                 1000                1005
Glu Tyr Leu Tyr Val Glu Glu Leu Phe Ser Asp Gly Ile Gln Ser Asp
    1010                1015                1020
Val Ile Glu Arg Leu Arg Leu Gln His Ser Lys Asp Leu Gln Lys Val
1025                1030                1035                1040
Val Asp Ile Val Leu Ser His Gln Ser Val Arg Asn Lys Thr Lys Leu
                1045                1050                1055
Ile Leu Lys Leu Met Glu Ser Leu Val Tyr Pro Asn Pro Ala Ala Tyr
            1060                1065                1070
Arg Asp Gln Leu Ile Arg Phe Ser Ser Leu Asn His Lys Ala Tyr Tyr
        1075                1080                1085
Lys Leu Ala Leu Lys Ala Ser Glu Leu Leu Glu Gln Thr Lys Leu Ser
    1090                1095                1100
Glu Leu Arg Ala Arg Ile Ala Arg Ser Leu Ser Glu Leu Glu Met Phe
1105                1110                1115                1120
Thr Glu Glu Ser Lys Gly Leu Ser Met His Lys Arg Glu Ile Ala Ile
                1125                1130                1135
Lys Glu Ser Met Glu Asp Leu Val Thr Ala Pro Leu Pro Val Glu Asp
            1140                1145                1150
Ala Leu Ile Ser Leu Phe Asp Cys Ser Asp Thr Thr Val Gln Gln Arg
        1155                1160                1165
Val Ile Glu Thr Tyr Ile Ala Arg Leu Tyr Gln Pro His Leu Val Lys
    1170                1175                1180
Asp Ser Ile Lys Met Lys Trp Ile Glu Ser Gly Val Ile Ala Leu Trp
1185                1190                1195                1200
Glu Phe Pro Glu Gly His Phe Asp Ala Arg Asn Gly Gly Ala Val Leu
                1205                1210                1215
Gly Asp Lys Arg Trp Gly Ala Met Val Ile Val Lys Ser Leu Glu Ser
            1220                1225                1230
Leu Ser Met Ala Ile Arg Phe Ala Leu Lys Glu Thr Ser His Tyr Thr
        1235                1240                1245
Ser Ser Glu Gly Asn Met Met His Ile Ala Leu Leu Gly Ala Asp Asn
    1250                1255                1260
Lys Met His Ile Ile Gln Glu Ser Gly Asp Asp Ala Asp Arg Ile Ala
1265                1270                1275                1280
Lys Leu Pro Leu Ile Leu Lys Asp Asn Val Thr Asp Leu His Ala Ser
                1285                1290                1295
Gly Val Lys Thr Ile Ser Phe Ile Val Gln Arg Asp Glu Ala Arg Met
            1300                1305                1310
Thr Met Arg Arg Thr Phe Leu Trp Ser Asp Glu Lys Leu Ser Tyr Glu
        1315                1320                1325
Glu Glu Pro Ile Leu Arg His Val Glu Pro Pro Leu Ser Ala Leu Leu
    1330                1335                1340
Glu Leu Asp Lys Leu Lys Val Lys Gly Tyr Asn Glu Met Lys Tyr Thr
1345                1350                1355                1360
Pro Ser Arg Asp Arg Gln Trp His Ile Tyr Thr Leu Arg Asn Thr Glu
                1365                1370                1375
Asn Pro Lys Met Leu His Arg Val Phe Phe Arg Thr Leu Val Arg Gln
            1380                1385                1390
Pro Ser Val Ser Asn Lys Phe Ser Ser Gly Gln Ile Gly Asp Met Glu
        1395                1400                1405
Val Gly Ser Ala Glu Glu Pro Leu Ser Phe Thr Ser Thr Ser Ile Leu
    1410                1415                1420
Arg Ser Leu Met Thr Ala Ile Glu Glu Leu Glu Leu His Ala Ile Arg
1425                1430                1435                1440
Thr Gly His Ser His Met Tyr Leu His Val Leu Lys Glu Gln Lys Leu
                1445                1450                1455
Leu Asp Leu Val Pro Val Ser Gly Asn Thr Val Leu Asp Val Gly Gln
            1460                1465                1470
Asp Glu Ala Thr Ala Tyr Ser Leu Leu Lys Glu Met Ala Met Lys Ile

1475                1480                1485
His Glu Leu Val Gly Ala Arg Met His His Leu Ser Val Cys Gln Trp
    1490                1495                1500
Glu Val Lys Leu Lys Leu Asp Cys Asp Gly Pro Ala Ser Gly Thr Trp
1505                1510                1515                1520
Arg Ile Val Thr Thr Asn Val Thr Ser His Thr Cys Thr Val Asp Ile
                1525                1530                1535
Tyr Arg Glu Met Glu Asp Lys Glu Ser Arg Lys Leu Val Tyr His Pro
            1540                1545                1550
Ala Thr Pro Ala Ala Gly Pro Leu His Gly Val Ala Leu Asn Asn Pro
        1555                1560                1565
Tyr Gln Pro Leu Ser Val Ile Asp Leu Lys Arg Cys Ser Ala Arg Asn
    1570                1575                1580
Asn Arg Thr Thr Tyr Cys Tyr Asp Phe Pro Leu Ala Phe Glu Thr Ala
1585                1590                1595                1600
Val Arg Lys Ser Trp Ser Ser Ser Thr Ser Gly Ala Ser Lys Gly Val
                1605                1610                1615
Glu Asn Ala Gln Cys Tyr Val Lys Ala Thr Glu Leu Val Phe Ala Asp
            1620                1625                1630
Lys His Gly Ser Trp Gly Thr Pro Leu Val Gln Met Asp Arg Pro Ala
        1635                1640                1645
Gly Leu Asn Asp Ile Gly Met Val Ala Trp Thr Leu Lys Met Ser Thr
    1650                1655                1660
Pro Glu Phe Pro Ser Gly Arg Glu Ile Ile Val Val Ala Asn Asp Ile
1665                1670                1675                1680
Thr Phe Arg Ala Gly Ser Phe Gly Pro Arg Glu Asp Ala Phe Phe Glu
                1685                1690                1695
Ala Val Thr Asn Leu Ala Cys Glu Lys Lys Leu Pro Leu Ile Tyr Leu
            1700                1705                1710
Ala Ala Asn Ser Gly Ala Arg Ile Gly Ile Ala Asp Glu Val Lys Ser
        1715                1720                1725
Cys Phe Arg Val Gly Trp Ser Asp Asp Gly Ser Pro Glu Arg Gly Phe
    1730                1735                1740
Gln Tyr Ile Tyr Leu Ser Glu Glu Asp Tyr Ala Arg Ile Gly Thr Ser
1745                1750                1755                1760
Val Ile Ala His Lys Met Gln Leu Asp Ser Gly Glu Ile Arg Trp Val
                1765                1770                1775
Ile Asp Ser Val Val Gly Lys Glu Asp Gly Leu Gly Val Glu Asn Ile
            1780                1785                1790
His Gly Ser Ala Ala Ile Ala Ser Ala Tyr Ser Arg Ala Tyr Lys Glu
        1795                1800                1805
Thr Phe Thr Leu Thr Phe Val Thr Gly Arg Thr Val Gly Ile Gly Ala
    1810                1815                1820
Tyr Leu Ala Arg Leu Gly Ile Arg Cys Ile Gln Arg Leu Asp Gln Pro
1825                1830                1835                1840
Ile Ile Leu Thr Gly Tyr Ser Ala Leu Asn Lys Leu Leu Gly Arg Glu
                1845                1850                1855
Val Tyr Ser Ser His Met Gln Leu Gly Gly Pro Lys Ile Met Ala Thr
            1860                1865                1870
Asn Gly Val Val His Leu Thr Val Ser Asp Asp Leu Glu Gly Val Ser
        1875                1880                1885
Asn Ile Leu Arg Trp Leu Ser Tyr Val Pro Ala Tyr Ile Gly Gly Pro
    1890                1895                1900
Leu Pro Val Thr Thr Pro Leu Asp Pro Pro Asp Arg Pro Val Ala Tyr
1905                1910                1915                1920
Ile Pro Glu Asn Ser Cys Asp Pro Arg Ala Ala Ile Arg Gly Val Asp
                1925                1930                1935
Asp Ser Gln Gly Lys Trp Leu Gly Gly Met Phe Asp Lys Asp Ser Phe
            1940                1945                1950
Val Glu Thr Phe Glu Gly Trp Ala Lys Thr Val Val Thr Gly Arg Ala
        1955                1960                1965
Lys Leu Gly Gly Ile Pro Val Gly Val Ile Ala Val Glu Thr Gln Thr
    1970                1975                1980

Met Met Gln Thr Ile Pro Ala Asp Pro Gly Gln Leu Asp Ser Arg Glu
1985                1990                1995                2000
Gln Ser Val Pro Arg Ala Gly Gln Val Trp Phe Pro Asp Ser Ala Thr
                2005                2010                2015
Lys Thr Ala Gln Ala Leu Leu Asp Phe Asn Arg Glu Gly Leu Pro Leu
            2020                2025                2030
Phe Ile Leu Ala Asn Trp Arg Gly Phe Ser Gly Gly Gln Arg Asp Leu
        2035                2040                2045
Phe Glu Gly Ile Leu Gln Ala Gly Ser Thr Ile Val Glu Asn Leu Arg
    2050                2055                2060
Thr Tyr Asn Gln Pro Ala Phe Val Tyr Ile Pro Met Ala Ala Glu Leu
2065                2070                2075                2080
Arg Gly Gly Ala Trp Val Val Val Asp Ser Lys Ile Asn Pro Asp Arg
                2085                2090                2095
Ile Glu Cys Tyr Ala Glu Arg Thr Ala Lys Gly Asn Val Leu Glu Pro
            2100                2105                2110
Gln Gly Leu Ile Glu Ile Lys Phe Arg Ser Glu Glu Leu Gln Asp Cys
        2115                2120                2125
Met Ser Arg Leu Asp Pro Thr Leu Ile Asp Leu Lys Ala Lys Leu Glu
    2130                2135                2140
Val Ala Asn Lys Asn Gly Ser Ala Asp Thr Lys Ser Leu Gln Glu Asn
2145                2150                2155                2160
Ile Glu Ala Arg Thr Lys Gln Leu Met Pro Leu Tyr Thr Gln Ile Ala
                2165                2170                2175
Ile Arg Phe Ala Glu Leu His Asp Thr Ser Leu Arg Met Ala Ala Lys
            2180                2185                2190
Gly Val Ile Lys Lys Val Val Asp Trp Glu Glu Ser Arg Ser Phe Phe
        2195                2200                2205
Tyr Lys Arg Leu Arg Arg Arg Ile Ser Glu Asp Val Leu Ala Lys Glu
    2210                2215                2220
Ile Arg Ala Val Ala Gly Glu Gln Phe Ser His Gln Pro Ala Ile Glu
2225                2230                2235                2240
Leu Ile Lys Lys Trp Tyr Ser Ala Ser His Ala Ala Glu Trp Asp Asp
                2245                2250                2255
Asp Asp Ala Phe Val Ala Trp Met Asp Asn Pro Glu Asn Tyr Lys Asp
            2260                2265                2270
Tyr Ile Gln Tyr Leu Lys Ala Gln Arg Val Ser Gln Ser Leu Ser Ser
        2275                2280                2285
Leu Ser Asp Ser Ser Ser Asp Leu Gln Ala Leu Pro Gln Gly Leu Ser
    2290                2295                2300
Met Leu Leu Asp Lys Met Asp Pro Ser Arg Arg Ala Gln Leu Val Glu
2305                2310                2315                2320
Glu Ile Arg Lys Val Leu Gly
                2325

<210> 14
<211> 11927
<212> DNA
<213> Oryza sativa L. ACCase2 genome DNA sequence (DNA Sequence)

<400> 14
atgacatcca cacatgtggc gacattggga gttggtgccc aggcacctcc tcgtcaccag 60
aaaaagtcag ctggcactgc atttgtatca tctgggtcat caagaccctc ataccgaaag 120
aatggtcagc gtactcggtc acttagggaa gaaagcaatg gaggagtgtc tgattccaaa 180
aagcttaacc actctattcg ccaaggtgac cactagctac tttacatatg ctataatttg 240
tgccaaacat aaacatgcaa tggctgctat tatttaaacg ttaatgttga aatagctgct 300
ataggataca gcaaaaatat ataattgact gggcaagatg caacaattgt ttttcactaa 360
agttagttat cttttgctgt aaaagacaac tgttttttac ataaaatggt attaataacc 420
ttgtaatatt caatgcaaca tgttctcaag taaaaaaaaa cattgcctgg ttgtataagc 480
aaatgtgtcg ttgtagacat cttattaaac ctttttgtga tatctattac cgtagggaac 540
aggggagctg tttaaatctg ttatcataga gtaatatgag aaaagtggat tgtgcgactt 600
tggcatgtat acctgctcaa tttcaaatat atgtctatgt gcaggtcttg ctggcatcat 660
tgacctccca aatgacgcag cttcagaagt tgatatttca cagtaaggac tttatatttt 720

ataataatta ttatataatt ttctgacatg ttttgagaac ctcaaaacat gtgattgcac 780
cttccttttt tatgtctggt tcagaaactg ataagttttg acagtgttta ggatggatct 840
ttgatgcgca cagtgctttc taatgttttc atttttgaaa gtaatgtttt aggaagaaat 900
atctgattaa atttatactt tatctttaca aaagtcaaat gcgttctgta tcaattgcgg 960
tttgtaatat ggcaagaaca tgctttcaga atttgttcat acaatgcttt ctttctatta 1020
ttatgtagaa caaataccta atactttgtt caccttttat agtggacacc tctcacagct 1080
ttttcagtaa gtgatgcaat tttgtacatt tgtaagatgt gttccagaaa ccttttctcc 1140
tgcaattcta atgtacccac tcaaactggt atcaccaaag atctccatct gattgaaaaa 1200
aagctgcgtg aagtatgctt atttatgcta accatacatg atttatactg ttttatagta 1260
caatgcttat ttatgctaac catacataat tttattctgt tttctagtac attatttgtg 1320
cccctgacca taaatgatcc tttcttttac agtggttccg aagatcccag ggggcctacg 1380
gtcccaggtt cctaccaaat gaatgggatt atcaatgaaa cacataatgg gaggcatgct 1440
tcagtctcca aggttgttga gttttgtacg gcacttggtg gcaaaacacc aattcacagt 1500
gtattagtgg ccaacaatgg aatggcagca gctaagttca tgcggagtgt ccgaacatgg 1560
gctaatgata cttttggatc agagaaggca attcagctga tagctatggc aactccggag 1620
gatctgagga taaatgcaga gcacatcaga attgccgatc aatttgtaga ggtacctggt 1680
ggaacaaaca acaacaacta tgcaaatgtc caactcatag tggaggttag ttcagctcat 1740
ccctcaacac aacattttcg tttctattta agttagggaa aaatctctac gaccctccaa 1800
tttctgaaca tccaattttc accatcaact gcaatcacag atagcagaga gaacaggtgt 1860
ttctgctgtt tggcctggtt ggggtcatgc atctgagaat cctgaacttc cagatgcgct 1920
gactgcaaaa ggaattgttt ttcttgggcc accagcatca tcaatgcatg cattaggaga 1980
caaggttggc tcagctctca ttgctcaagc agctggagtt ccaacacttg cttggagtgg 2040
atcacatgtg agccttgtct tctctttttt agcttatcat cttatctttt cggtgatgca 2100
ttatcccaat gacactaaac cataggtgga agttcctctg gagtgttgct tggactcaat 2160
acctgatgag atgtatagaa aagcttgtgt tactaccaca gaggaagcag ttgcaagttg 2220
tcaggtggtt ggttatcctg ccatgattaa ggcatcttgg ggtggtggtg gtaaaggaat 2280
aaggaaggtt tgttcttctt gtagttatca agagattgtt tggattgcaa gtgtttagtg 2340
cccatagtta actctggtct ttctaacatg agtaactcaa ctttcttgca ggttcataat 2400
gatgatgagg ttaggacatt atttaagcaa gttcaaggcg aagtacctgg ttccccaata 2460
tttatcatga ggctagctgc tcaggtgggg ccttttatgg aagttacacc ttttccctta 2520
atgttgagtt attccggagt tattatggtt atgttctgta tgtttgatct gtaaattatt 2580
gaaattcacc tccattggtt ctccagatta gcagacctac aattctacat atggtttata 2640
ctttataaat actaggattt agggatcttc atatagttta tacatggtat ttagatttca 2700
tttgtaaccc tattgaagac atcctgattg ttgtcttatg tagagtcgac atcttgaagt 2760
tcagttgctt tgtgatcaat atggcaacgt agcagcactt cacagtcgag attgcagtgt 2820
acaacggcga caccaaaagg tctgctgtct cagttaaatc acccctctga atgatctact 2880
tcttgcctgc tgcgttggtc agaggaataa tggttgtatt ctactgaaca gataatcgag 2940
gaaggaccag ttactgttgc tcctcgtgag actgtgaaag agcttgagca ggcagcacgg 3000
aggcttgcta aagctgtggg ttatgttggt gctgctactg ttgaatacct ttacagcatg 3060
gaaactggtg aatattattt tctggaactt aatccacggc tacaggtcgg ctcctttgac 3120
attcttcagg aattaatttc tgttgaccac atgatttaca ttgtcaaatg gtctcacagg 3180
ttgagcatcc tgtcactgag tggatagctg aagtaaattt gcctgcggct caagttgctg 3240
ttggaatggg tatacccctt tggcagattc caggtaatgc ttcttcattt agttcctgct 3300
ctttgttaat tgaatgagct cttatacaga ccatgagaca cattctactg ttaattcata 3360
gtatcccctg acttgttagt gttagagata cagagatgta tcacaaattc attgtatctc 3420
ctcaaggact gtaaaaatcc tataattaaa tttctgaaaa tttgttcttt taagcagaaa 3480
aaaaatctct aaattatctc cctgtataca gagatcaggc gcttctacgg aatgaaccat 3540
ggaggaggct atgacctttg gaggaaaaca gcagctctag cgactccatt taactttgat 3600
gaagtagatt ctaaatggcc aaaaggccac tgcgtagctg ttagaataac tagcgaggat 3660
ccagatgatg ggtttaagcc tactggtgga aaagtaaagg tgcggtttcc tgatgttagg 3720
tgtatgaatt gaacacattg ctatattgca gctagtgaaa tgactggatc atggttctct 3780
tattttcagg agataagttt caagagtaaa ccaaatgttt gggcctattt ctcagtaaag 3840
gtagtcctca atattgttgc actgccacat tatttgagtt gtcctaacaa ttgtgctgca 3900
attgttagtt ttcaactatt tgttgttctg tttggttgac tggtaccctc tctttgcagt 3960
ctggtggagg catccatgaa ttcgctgatt ctcagttcgg tatgtaaagt taaaagagta 4020
atattgtctt tgctatttat gtttgtcctc acttttaaaa gatattgcct tccattacag 4080
gacatgtttt tgcgtatgga actactagat cggcagcaat aactaccatg gctcttgcac 4140
taaaagaggt tcaaattcgt ggagaaattc attcaaacgt agactacaca gttgacctat 4200
taaatgtaag gactaaatat ctgcttattg aaccttgctt tttggttccc taatgccatt 4260
ttagtctggc tactgaagaa cttatccatc atgccatttc tgttatctta aattcaggcc 4320
tcagatttta gagaaaataa gattcatact ggttggctgg ataccaggat agccatgcgt 4380
gttcaagctg agaggcctcc atggtatatt tcagtcgttg gaggggcttt atatgtaaga 4440
caaactatgc cactcattag catttatgtg aagcaaatgc ggaaaacatg atcaatatgt 4500

cgtcttattt aaatttattt atttttgtgc tgcagaaaac agtaactgcc aacacggcca 4560
ctgtttctga ttatgttggt tatcttacca agggccagat tccaccaaag gtactattct 4620
gttttttcag gatatgaatg ctgtttgaat gtgaaaacca ttgaccataa atccttgttt 4680
gcagcatata tcccttgtct atacgactgt tgctttgaat atagatggga aaaaatatac 4740
agtaagtgtg acattcttaa tggggaaact taatttgttg taaataatca atatcatatt 4800
gactcgtgta tgctgcatca tagatcgata ctgtgaggag tggacatggt agctacagat 4860
tgcgaatgaa tggatcaacg gttgacgcaa atgtacaaat attatgtgat ggtgggcttt 4920
taatgcaggt aatatcttct tcctagttaa agaagatata tcttgttcaa agaattctga 4980
ttattgatct tttaatgttt tcagctggat ggaaacagcc atgtaattta tgctgaagaa 5040
gaggccagtg gtacacgact tcttattgat ggaaagacat gcatgttaca ggtaatgata 5100
gccttgttct ttttagttct agtcacggtg tttgcttgct atttgttgta tctatttaat 5160
gcattcacta attactatat tagtttgcat catcaagtta aaatggaact tctttcttgc 5220
agaatgacca tgacccatca aagttattag ctgagacacc atgcaaactt cttcgtttct 5280
tggttgctga tggtgctcat gttgatgctg atgtaccata tgcggaagtt gaggttatga 5340
agatgtgcat gcccctctta tcacccgctt ctggtgtcat acatgttgta atgtctgagg 5400
gccaagcaat gcaggtacat tcctacattc cattcattgt gctgtgctga catgaacatt 5460
tcaagtaaat acctgtaact tgtttattat tctaggctgg tgatcttata gctaggctgg 5520
atcttgatga cccttctgct gttaagagag ctgagccgtt cgaagatact tttccacaaa 5580
tgggtctccc tattgctgct tctggccaag ttcacaaatt atgtgctgca agtctgaatg 5640
cttgtcgaat gatccttgcg gggtatgagc atgatattga caaggtaaac atcatgtcct 5700
cttgtttttt cttttgttta tcatgcattc ttatgttcat catgtcctct ggcaaatcta 5760
gattccgctg tcgtttcaca cagatttttc tcattctcat aatggtgcca aacataaata 5820
tgctgctata ttcatcaatg ttttcactcg atttctaatt ttgcttttga gttttaaact 5880
ttagtacaat ccatatctaa tctcctttgg caacagtgaa tccattatat atatttttat 5940
taaactgctt tctttttcag gttgtgccag agttggtata ctgcctagac actccggagc 6000
ttcctttcct gcagtgggag gagcttatgt ctgttttagc aactagactt ccaagaaatc 6060
ttaaaagtga ggtatattat ggttgacaag atagctagtc tcatgctcta aggacttgta 6120
catttcgcca cataggttaa ttttccatat caagttctaa tgtacgatat aaaagtagta 6180
ctggcctaaa acagtattgg tggttgacta tctttgttgt gtaagatcaa gtatttcttt 6240
ttcatgctta gtttgtcaat acttcacatt tatcactgac ttgtcgagct aaatgagatt 6300
ttatttgatt tctgtgctcc attatttttg tatatatata tatatattta actatgacta 6360
tatgttatgc ctcaaacgtt tcaaactctt tcagttggag ggcaaatatg aggaatacaa 6420
agtaaaattt gactctggga taatcaatga tttccctgcc aatatgctac gagtgataat 6480
tgaggtcagt tattcaattt gttgtgataa tcactgcctt aactgttcgt tcttttaaca 6540
agcggtttta taggaaaatc ttgcatgtgg ttctgagaag gagaaggcta caaatgagag 6600
gcttgttgag cctcttatga gcctactgaa gtcatatgag ggtgggagag aaagtcatgc 6660
tcactttgtt gtcaagtccc tttttgagga gtatctctat gttgaagaat tgttcagtga 6720
tggaattcag gttaacttac ctattcgcat taaacaaatc atcagttgtt ttatgataaa 6780
gtcaaaatgt ttatatttcc cattcttctg tggatcaaat atatcacgga catgatatag 6840
tttccttagg ctatataatg gttcttcatc aaataatatt gcaggaaaca gtatagcaaa 6900
ctatttgtat atactcgaga tggaaattgt tagaaacatc attgactaaa tctgtccttt 6960
gttacgctgt ttttgtagtc tgatgtgatt gagcgtctgc gccttcaaca tagtaaagac 7020
ctacagaagg tcgtagacat tgtgttgtcc caccaggtaa atttcttcat ggtctgatga 7080
cttcactgcg aatggttact gaactgtctt cttgttctga caatgtgact tttctttgta 7140
gagtgttaga aataaaacta agctgatact aaaactcatg gagagtctgg tctatccaaa 7200
tcctgctgcc tacagggatc aattgattcg cttttcttcc cttaatcaca aagcgtatta 7260
caaggtgacc aggataaaca taaataaacg tgaatttttc aatgaccttt tcttctgaca 7320
tctgaatctg atgaatttct tgcatattaa tacagttggc acttaaagct agtgaacttc 7380
ttgaacaaac aaaacttagt gagctccgtg caagaatagc aaggagcctt tcagagctgg 7440
agatgtttac tgaggaaagc aagggtctct ccatgcataa gcgagaaatt gccattaagg 7500
agagcatgga agatttagtc actgctccac tgccagttga agatgcgctc atttctttat 7560
ttgattgtag tgatacaact gttcaacaga gagtgattga gacttatata gctcgattat 7620
accaggtatg agaagaaaga ccttttgaaa ttatttatat taacatatcc tagtaaaaca 7680
gcatgctcat catttcttaa aaaaagttta cagcacctga tgtttggtta ctgaccgcat 7740
cattaaaata aagttacttg ttgtggagag atgtattttg gaacttgtgg cacatgcagt 7800
aacatgctac tgctcgatat gtttgctaac ttgacaacaa tatttttcag cctcatcttg 7860
taaaggacag tatcaaaatg aaatggatag aatcgggtgt tattgcttta tgggaatttc 7920
ctgaagggca ttttgatgca agaaatggag gagcggttct tggtgacaaa agatggggtg 7980
ccatggtcat tgtcaagtct cttgaatcac tttcaatggc cattagattt gcactaaagg 8040
agacatcaca ctacactagc tctgagggca atatgatgca tattgctttg ttgggtgctg 8100
ataataagat gcatataatt caagaaaggt atgttcatat gctatgttgg tgctgaaata 8160
gttatatatg tagttagctg gtggagttct ggtaattaac ctatcccatt gttcagtggt 8220
gatgatgctg acagaatagc caaacttccc ttgatactaa aggataatgt aaccgatctg 8280

catgcctctg gtgtgaaaac aataagtttc attgttcaaa gagatgaagc acggatgaca 8340
atgcgtcgta ccttcctttg gtctgatgaa aagctttctt atgaggaaga gccaattctc 8400
cggcatgtgg aacctcctct ttctgcactt cttgagttgg tacgtgatat catcaaaatg 8460
ataatgtttt ggtatggcat tgattatctt ctatgctctt tgtatttatt cagcctattg 8520
tggatacagg acaagttgaa agtgaaagga tacaatgaaa tgaagtatac cccatcacgg 8580
gatcgtcaat ggcatatcta cacacttaga aatactgaaa accccaaaat gttgcaccgg 8640
gtatttttcc gaacccttgt caggcaaccc agtgtatcca acaagttttc ttcgggccag 8700
attggtgaca tggaagttgg gagtgctgaa gaacctctgt catttacatc aaccagcata 8760
ttaagatctt tgatgactgc tatagaggaa ttggagcttc acgcaattag aactggccat 8820
tcacacatgt atttgcatgt attgaaagaa caaaagcttc ttgatcttgt tccagtttca 8880
gggtaagtgc gcatatttct ttttgggaac atatgcttgc ttatgaggtt ggtcttctca 8940
atgatcttct tatcttactc aggaatacag ttttggatgt tggtcaagat gaagctactg 9000
catattcact tttaaaagaa atggctatga agatacatga acttgttggt gcaagaatgc 9060
accatctttc tgtatgccaa tgggaagtga aacttaagtt ggactgcgat ggtcctgcca 9120
gtggtacctg gaggattgta acaaccaatg ttactagtca cacttgcact gtggatgtaa 9180
gtttaatcct ctagcatttt gttttctttg gaaaagcatg tgattttaag ccggctggtc 9240
ctcataccca gacctagtga tctttatata gtgtagacat ttttctaact gcttttaatt 9300
gttttagatc taccgtgaga tggaagataa agaatcacgg aagttagtat accatcccgc 9360
cactccggcg gctggtcctc tgcatggtgt ggcactgaat aatccatatc agcctttgag 9420
tgtcattgat ctcaaacgct gttctgctag gaataataga actacatact gctatgattt 9480
tccactggtg agttgactgc tcccttatat tcaatgcatt accatagcaa attcatattc 9540
gttcatgttg tcaaaataag ccgatgaaaa ttcaaaactg taggcatttg aaactgcagt 9600
gaggaagtca tggtcctcta gtacctctgg tgcttctaaa ggtgttgaaa atgcccaatg 9660
ttatgttaaa gctacagagt tggtatttgc ggacaaacat gggtcatggg gcactccttt 9720
agttcaaatg gaccggcctg ctgggctcaa tgacattggt atggtagctt ggaccttgaa 9780
gatgtccact cctgaatttc ctagtggtag ggagattatt gttgttgcaa atgatattac 9840
gttcagagct ggatcatttg gcccaaggga agatgcattt tttgaagctg ttaccaacct 9900
agcctgtgag aagaaacttc ctcttattta tttggcagca aattctggtg ctcgaattgg 9960
catagcagat gaagtgaaat cttgcttccg tgttgggtgg tctgatgatg gcagccctga 10020
acgtgggttt cagtacattt atctaagcga agaagactat gctcgtattg gcacttctgt 10080
catagcacat aagatgcagc tagacagtgg tgaaattagg tgggttattg attctgttgt 10140
gggcaaggaa gatggacttg gtgtggagaa tatacatgga agtgctgcta ttgccagtgc 10200
ttattctagg gcatataagg agacatttac acttacattt gtgactggaa gaactgttgg 10260
aataggagct tatcttgctc gacttggcat ccggtgcata cagcgtcttg accagcctat 10320
tattcttaca ggctattctg cactgaacaa gcttcttggg cgggaagtgt acagctccca 10380
catgcagttg ggtggtccca aaatcatggc aactaatggt gttgtccatc ttactgtttc 10440
agatgacctt gaaggcgttt ctaatatatt gaggtggctc agttatgttc ctgcctacat 10500
tggtggacca cttccagtaa caacaccgtt ggacccaccg gacagacctg ttgcatacat 10560
tcctgagaac tcgtgtgatc ctcgagcggc tatccgtggt gttgatgaca gccaagggaa 10620
atggttaggt ggtatgtttg ataaagacag ctttgtggaa acatttgaag gttgggctaa 10680
gacagtggtt actggcagag caaagcttgg tggaattcca gtgggtgtga tagctgtgga 10740
gactcagacc atgatgcaaa ctatccctgc tgaccctggt cagcttgatt cccgtgagca 10800
atctgttcct cgtgctggac aagtgtggtt tccagattct gcaaccaaga ctgcgcaggc 10860
attgctggac ttcaaccgtg aaggattacc tctgttcatc ctcgctaact ggagaggctt 10920
ctctggtgga caaagagatc tttttgaagg aattcttcag gctggctcga ctattgttga 10980
gaaccttagg acatacaatc agcctgcctt tgtctacatt cccatggctg cagagctacg 11040
aggaggggct tgggttgtgg ttgatagcaa gataaaccca gaccgcattg agtgctatgc 11100
tgagaggact gcaaaaggca atgttctgga accgcaaggg ttaattgaga tcaagttcag 11160
gtcagaggaa ctccaggatt gcatgagtcg gcttgaccca acattaattg atctgaaagc 11220
aaaactcgaa gtagcaaata aaaatggaag tgctgacaca aaatcgcttc aagaaaatat 11280
agaagctcga acaaaacagt tgatgcctct atatactcag attgcgatac ggtttgctga 11340
attgcatgat acatccctca gaatggctgc gaaaggtgtg attaagaaag ttgtggactg 11400
ggaagaatca cgatctttct tctataagag attacggagg aggatctctg aggatgttct 11460
tgcaaaagaa attagagctg tagcaggtga gcagttttcc caccaaccag caatcgagct 11520
gatcaagaaa tggtattcag cttcacatgc agctgaatgg gatgatgacg atgcttttgt 11580
tgcttggatg gataaccctg aaaactacaa ggattatatt caatatctta aggctcaaag 11640
agtatcccaa tccctctcaa gtctttcaga ttccagctca gatttgcaag ccctgccaca 11700
gggtctttcc atgttactag ataaggtaat tagcttactg atgcttatat aaattctttt 11760
tcattacata tggctggaga actatctaat caaataatga ttataattcc aatcgttctt 11820
tttatgccat tatgatcttc tgaaatttcc ttctttggac acttattcag atggatccct 11880
ctagaagagc tcaacttgtt gaagaaatca ggaaggtcct tggttga 11927

<210> 15

<211>   4203
<212>   DNA
<213>   SpCas9 DNA sequence (DNA sequence)

<400>   15
atggctccga agaagaagag gaaggttggc atccacgggg tgccagctgc tgacaagaag      60
tactcgatcg gcctcgatat tgggactaac tctgttggct gggccgtgat caccgacgag     120
tacaaggtgc cctcaaagaa gttcaaggtc ctgggcaaca ccgatcggca ttccatcaag     180
aagaatctca ttggcgctct cctgttcgac agcggcgaga cggctgaggc tacgcggctc     240
aagcgcaccg cccgcaggcg gtacacgcgc aggaagaatc gcatctgcta cctgcaggag     300
attttctcca acgagatggc gaaggttgac gattctttct tccacaggct ggaggagtca     360
ttcctcgtgg aggaggataa gaagcacgag cggcatccaa tcttcggcaa cattgtcgac     420
gaggttgcct accacgagaa gtaccctacg atctaccatc tgcggaagaa gctcgtggac     480
tccacagata aggcggacct ccgcctgatc tacctcgctc tggcccacat gattaagttc     540
aggggccatt tcctgatcga gggggatctc aacccggaca atagcgatgt tgacaagctg     600
ttcatccagc tcgtgcagac gtacaaccag ctcttcgagg agaaccccat taatgcgtca     660
ggcgtcgacg cgaaggctat cctgtccgct aggctctcga agtctcggcg cctcgagaac     720
ctgatcgccc agctgccggg cgagaagaag aacggcctgt tcgggaatct cattgcgctc     780
agcctggggc tcacgcccaa cttcaagtcg aatttcgatc tcgctgagga cgccaagctg     840
cagctctcca aggacacata cgacgatgac ctggataacc tcctggccca gatcggcgat     900
cagtacgcgg acctgttcct cgctgccaag aatctgtcgg acgccatcct cctgtctgat     960
attctcaggg tgaacaccga gattacgaag gctccgctct cagcctccat gatcaagcgc    1020
tacgacgagc accatcagga tctgaccctc ctgaaggcgc tggtcaggca gcagctcccc    1080
gagaagtaca aggagatctt cttcgatcag tcgaagaacg gctacgctgg gtacattgac    1140
ggcggggcct ctcaggagga gttctacaag ttcatcaagc cgattctgga gaagatggac    1200
ggcacggagg agctgctggt gaagctcaat cgcgaggacc tcctgaggaa gcagcggaca    1260
ttcgataacg gcagcatccc acaccagatt catctcgggg agctgcacgc tatcctgagg    1320
aggcaggagg acttctaccc tttcctcaag gataaccgcg agaagatcga gaagattctg    1380
actttcagga tcccgtacta cgtcggccca ctcgctaggg gcaactcccg cttcgcttgg    1440
atgacccgca agtcagagga gacgatcacg ccgtggaact tcgaggaggt ggtcgacaag    1500
ggcgctagcg ctcagtcgtt catcgagagg atgacgaatt tcgacaagaa cctgccaaat    1560
gagaaggtgc tccctaagca ctcgctcctg tacgagtact tcacagtcta caacgagctg    1620
actaaggtga agtatgtgac cgagggcatg aggaagccgg ctttcctgtc tggggagcag    1680
aagaaggcca tcgtggacct cctgttcaag accaaccgga aggtcacggt taagcagctc    1740
aaggaggact acttcaagaa gattgagtgc ttcgattcgg tcgagatctc tggcgttgag    1800
gaccgcttca acgcctccct ggggacctac cacgatctcc tgaagatcat taaggataag    1860
gacttcctgg acaacgagga gaatgaggat atcctcgagg acattgtgct gacactcact    1920
ctgttcgagg accgggagat gatcgaggag cgcctgaaga cttacgccca tctcttcgat    1980
gacaaggtca tgaagcagct caagaggagg aggtacaccg gctgggggag gctgagcagg    2040
aagctcatca acggcattcg ggacaagcag tccgggaaga cgatcctcga cttcctgaag    2100
agcgatggct tcgcgaaccg caatttcatg cagctgattc acgatgacag cctcacattc    2160
aaggaggata tccagaaggc tcaggtgagc ggccaggggg actcgctgca cgagcatatc    2220
gcgaacctcg ctggctcgcc agctatcaag aaggggattc tgcagaccgt gaaggttgtg    2280
gacgagctgg tgaaggtcat gggcaggcac aagcctgaga acatcgtcat tgagatggcc    2340
cgggagaatc agaccacgca gaagggccag aagaactcac gcgagaggat gaagaggatc    2400
gaggagggca ttaaggagct ggggtcccag atcctcaagg agcacccggt ggagaacacg    2460
cagctgcaga atgagaagct ctacctgtac tacctccaga atggccgcga tatgtatgtg    2520
gaccaggagc tggatattaa caggctcagc gattacgacg tcgatcatat cgttccacag    2580
tcattcctga aggatgactc cattgacaac aaggtcctca ccaggtcgga caagaaccgg    2640
ggcaagtctg ataatgttcc ttcagaggag gtcgttaaga agatgaagaa ctactggcgc    2700
cagctcctga atgccaagct gatcacgcag cggaagttcg ataacctcac aaaggctgag    2760
aggggcgggc tctctgagct ggacaaggcg ggcttcatca agaggcagct ggtcgagaca    2820
cggcagatca ctaagcacgt tgcgcagatt ctcgactcac ggatgaacac taagtacgat    2880
gagaatgaca agctgatccg cgaggtgaag gtcatcaccc tgaagtcaaa gctcgtctcc    2940
gacttcagga aggatttcca gttctacaag gttcgggaga tcaacaatta ccaccatgcc    3000
catgacgcgt acctgaacgc ggtggtcggc acagctctga tcaagaagta cccaaagctc    3060
gagagcgagt tcgtgtacgg ggactacaag gtttacgatg tgaggaagat gatcgccaag    3120
tcggagcagg agattggcaa ggctaccgcc aagtacttct tctactctaa cattatgaat    3180
ttcttcaaga cagagatcac tctggccaat ggcgagatcc ggaagcgccc cctcatcgag    3240
acgaacggcg agacggggga gatcgtgtgg gacaagggca gggatttcgc gaccgtcagg    3300
aaggttctct ccatgccaca agtgaatatc gtcaagaaga cagaggtcca gactggcggg    3360
ttctctaagg agtcaattct gcctaagcgg aacagcgaca agctcatcgc ccgcaagaag    3420
gactgggatc cgaagaagta cggcgggttc gacagcccca ctgtggccta ctcggtcctg    3480

gttgtggcga aggttgagaa gggcaagtcc aagaagctca agagcgtgaa ggagctgctg 3540
gggatcacga ttatggagcg ctccagcttc gagaagaacc cgatcgattt cctggaggcg 3600
aagggctaca aggaggtgaa gaaggacctg atcattaagc tccccaagta ctcactcttc 3660
gagctggaga acggcaggaa gcggatgctg gcttccgctg gcgagctgca gaaggggaac 3720
gagctggctc tgccgtccaa gtatgtgaac ttcctctacc tggcctccca ctacgagaag 3780
ctcaagggca gccccgagga caacgagcag aagcagctgt tcgtcgagca gcacaagcat 3840
tacctcgacg agatcattga gcagatttcc gagttctcca agcgcgtgat cctggccgac 3900
gcgaatctgg ataaggtcct ctccgcgtac aacaagcacc gcgacaagcc aatcagggag 3960
caggctgaga atatcattca tctcttcacc ctgacgaacc tcggcgcccc tgctgctttc 4020
aagtacttcg acacaactat cgatcgcaag aggtacacaa gcactaagga ggtcctggac 4080
gcgaccctca tccaccagtc gattaccggc ctctacgaga cgcgcatcga cctgtctcag 4140
ctcgggggcg acaagcggcc agcggcgacg aagaaggcgg ggcaggcgaa gaagaagaag 4200
tga 4203

<210> 16
<211> 4203
<212> DNA
<213> NGA-Cas9 DNA sequence (DNA sequence)

<400> 16
atggctccga agaagaagag gaaggttggc atccacgggg tgccagctgc tgacaagaag 60
tactcgatcg gcctcgatat tgggactaac tctgttggct gggccgtgat caccgacgag 120
tacaaggtgc cctcaaagaa gttcaaggtc ctgggcaaca ccgatcggca ttccatcaag 180
aagaatctca ttggcgctct cctgttcgac agcggcgaga cggctgaggc tacgcggctc 240
aagcgcaccg cccgcaggcg gtacacgcgc aggaagaatc gcatctgcta cctgcaggag 300
attttctcca acgagatggc gaaggttgac gattctttct tccacaggct ggaggagtca 360
ttcctcgtgg aggaggataa gaagcacgag cggcatccaa tcttcggcaa cattgtcgac 420
gaggttgcct accacgagaa gtaccctacg atctaccatc tgcggaagaa gctcgtggac 480
tccacagata aggcggacct ccgcctgatc tacctcgctc tggcccacat gattaagttc 540
aggggccatt tcctgatcga gggggatctc aacccggaca atagcgatgt tgacaagctg 600
ttcatccagc tcgtgcagac gtacaaccag ctcttcgagg agaaccccat taatgcgtca 660
ggcgtcgacg cgaaggctat cctgtccgct aggctctcga agtctcggcg cctcgagaac 720
ctgatcgccc agctgccggg cgagaagaag aacggcctgt tcgggaatct cattgcgctc 780
agcctggggc tcacgcccaa cttcaagtcg aatttcgatc tcgctgagga cgccaagctg 840
cagctctcca aggacacata cgacgatgac ctggataacc tcctggccca gatcggcgat 900
cagtacgcgg acctgttcct cgctgccaag aatctgtcgg acgccatcct cctgtctgat 960
attctcaggg tgaacaccga gattacgaag gctccgctct cagcctccat gatcaagcgc 1020
tacgacgagc accatcagga tctgaccctc ctgaaggcgc tggtcaggca gcagctcccc 1080
gagaagtaca aggagatctt cttcgatcag tcgaagaacg gctacgctgg gtacattgac 1140
ggcggggcct ctcaggagga gttctacaag ttcatcaagc cgattctgga gaagatggac 1200
ggcacggagg agctgctggt gaagctcaat cgcgaggacc tcctgaggaa gcagcggaca 1260
ttcgataacg gcagcatccc acaccagatt catctcgggg agctgcacgc tatcctgagg 1320
aggcaggagg acttctaccc tttcctcaag gataaccgcg agaagatcga gaagattctg 1380
actttcagga tcccgtacta cgtcggccca ctcgctaggg gcaactcccg cttcgcttgg 1440
atgacccgca agtcagagga gacgatcacg ccgtggaact tcgaggaggt ggtcgacaag 1500
ggcgctagcg ctcagtcgtt catcgagagg atgacgaatt tcgacaagaa cctgccaaat 1560
gagaaggtgc tccctaagca ctcgctcctg tacgagtact tcacagtcta caacgagctg 1620
actaaggtga agtatgtgac cgagggcatg aggaagccgg ctttcctgtc tggggagcag 1680
aagaaggcca tcgtggacct cctgttcaag accaaccgga aggtcacggt taagcagctc 1740
aaggaggact acttcaagaa gattgagtgc ttcgattcgg tcgagatctc tggcgttgag 1800
gaccgcttca acgcctccct ggggacctac cacgatctcc tgaagatcat taaggataag 1860
gacttcctgg acaacgagga gaatgaggat atcctcgagg acattgtgct gacactcact 1920
ctgttcgagg accgggagat gatcgaggag cgcctgaaga cttacgccca tctcttcgat 1980
gacaaggtca tgaagcagct caagaggagg aggtacaccg gctgggggag gctgagcagg 2040
aagctcatca acggcattcg ggacaagcag tccgggaaga cgatcctcga cttcctgaag 2100
agcgatggct tcgcgaaccg caatttcatg cagctgattc acgatgacag cctcacattc 2160
aaggaggata tccagaaggc tcaggtgagc ggccaggggg actcgctgca cgagcatatc 2220
gcgaacctcg ctggctcgcc agctatcaag aaggggattc tgcagaccgt gaaggttgtg 2280
gacgagctgg tgaaggtcat gggcaggcac aagcctgaga acatcgtcat tgagatggcc 2340
cgggagaatc agaccacgca gaagggccag aagaactcac gcgagaggat gaagaggatc 2400
gaggagggca ttaaggagct ggggtcccag atcctcaagg agcacccggt ggagaacacg 2460
cagctgcaga atgagaagct ctacctgtac tacctccaga atggccgcga tatgtatgtg 2520
gaccaggagc tggatattaa caggctcagc gattacgacg tcgatcatat cgttccacag 2580

tcattcctga aggatgactc cattgacaac aaggtcctca ccaggtcgga caagaaccgg 2640
ggcaagtctg ataatgttcc ttcagaggag gtcgttaaga agatgaagaa ctactggcgc 2700
cagctcctga atgccaagct gatcacgcag cggaagttcg ataacctcac aaaggctgag 2760
aggggcgggc tctctgagct ggacaaggcg ggcttcatca agaggcagct ggtcgagaca 2820
cggcagatca ctaagcacgt tgcgcagatt ctcgactcac ggatgaacac taagtacgat 2880
gagaatgaca agctgatccg cgaggtgaag gtcatcaccc tgaagtcaaa gctcgtctcc 2940
gacttcagga aggatttcca gttctacaag gttcgggaga tcaacaatta ccaccatgcc 3000
catgacgcgt acctgaacgc ggtggtcggc acagctctga tcaagaagta cccaaagctc 3060
gagagcgagt tcgtgtacgg ggactacaag gtttacgatg tgaggaagat gatcgccaag 3120
tcggagcagg agattggcaa ggctaccgcc aagtacttct tctactctaa cattatgaat 3180
ttcttcaaga cagagatcac tctggccaat ggcgagatcc ggaagcgccc cctcatcgag 3240
acgaacggcg agacggggga gatcgtgtgg gacaaggcca gggatttcgc gaccgtcagg 3300
aaggttctct ccatgccaca agtgaatatc gtcaagaaga cagaggtcca gactggcggg 3360
ttctctaagg agtcaattct gcctaagcgg aacagcgaca agctcatcgc ccgcaagaag 3420
gactgggatc cgaagaagta cggcgggttc gtcagcccca ctgtggccta ctcggtcctg 3480
gttgtggcga aggttgagaa gggcaagtcc aagaagctca agagcgtgaa ggagctgctg 3540
gggatcacga ttatggagcg ctccagcttc gagaagaacc cgatcgattt cctggaggcg 3600
aagggctaca aggaggtgaa gaaggacctg atcattaagc tccccaagta ctcactcttc 3660
gagctggaga acggcaggaa gcggatgctg gcttccgctg gcgagctgca gaaggggaac 3720
gagctggctc tgccgtccaa gtatgtgaac ttcctctacc tggcctccca ctacgagaag 3780
ctcaagggca gccccgagga caacgagcag aagcagctgt tcgtcgagca gcacaagcat 3840
tacctcgacg agatcattga gcagatttcc gagttctcca agcgcgtgat cctggccgac 3900
gcgaatctgg ataaggtcct ctccgcgtac aacaagcacc gcgacaagcc aatcagggag 3960
caggctgaga atatcattca tctcttcacc ctgacgaacc tcggcgcccc tgctgctttc 4020
aagtacttcg acacaactat cgatcgcaag cagtaccgga gcactaagga ggtcctggac 4080
gcgaccctca tccaccagtc gattaccggc ctctacgaga cgcgcatcga cctgtctcag 4140
ctcgggggcg acaagcggcc agcggcgacg aagaaggcgg ggcaggcgaa gaagaagaag 4200
tga 4203

<210> 17
<211> 5860
<212> DNA
<213> Oryza sativa L. OsBADH2 genome DNA sequence (DNA sequence)

<400> 17
atggccacgg cgatcccgca gcggcagctc ttcgtcgccg gcgagtggcg cgcccccgcg 60
ctcggccgcc gcctccccgt cgtcaacccc gccaccgagt cccccatcgg taccctcctc 120
ttcaccctct ccaccctctg cttctgcctc tgattagcct ttttgttgtt gttgttgttg 180
ctgctgtttt ttgcgtgtcg gtgcgcaggc gagatcccgg cgggcacggc ggaggacgtg 240
gacgcggcgg tggcggcggc gcgggaggcg ctgaagagga accggggccg cgactgggcg 300
cgcgcgccgg gcgccgtccg ggccaagtac ctccgcgcaa tcgcggccaa ggtagggtgg 360
tgactacccc cacccccccc ccccccccca acgcgacccg cgtgcgtgtg ttccgtacag 420
ggggaggagc tccgcgtggc tctccagtag gtttttgagc cccaaatcga tcgatatgct 480
ctagttttaa gtttgctgct taaattcctc aagggtttag tttgcaacca aatccttatt 540
ttagcttcgg tataagcccc ccatatgatg tgcgtgcgtc ggcatcggaa gtgcgtatcc 600
tctgttctgg actaggaatt ggccataggt tgatcgacag ttcgagtatt ctgcttctgt 660
ttggaataag ttggaagcat ggctgattgt gtatctggat gctgtttttg tggtgattcg 720
tttcaagctc ttgttaattg atgggttcaa gcggagaggg tgcgcaacaa caagtgtata 780
tggctcacgg ccatgggtgt gcacatttga ttggtgcgca acaacaagtg tatattgttt 840
gtgtgcttcg ttagttggca ggtcctagtc actaaatcac tattggattg gtactagtta 900
cttttgtgcc ttgacgatgg gactggatta ctagcctttt ggttgccttt gtggtattcc 960
gttgttatgg gcctgttgat ggatggatcc ctttaatttc tagtgccaaa tgcatgctag 1020
atttctcaca gtttttctct tcaggttata tttctcgtat ttccttttcc taaaggattg 1080
ctttttcatg tattttctgg catatatagg ttattattat tattattctc cagaacaaga 1140
ttacccatat tatggatcac tagtgtacac ttttttggat gaaaaaccta cttactgaaa 1200
gtaaaacagt gaccagtgca cactttactt gaactgtcaa accatcaatt ttctagcaaa 1260
gcaggggatg ctagccttcc agtctaaatg acagtaaact actatacttt tgtccgtagg 1320
tttggaaata tgctaatttc tatcataaaa attttcatgg catatgcgag cattttatga 1380
tcaccttttc cctttttctt cagataatcg agaggaaatc tgagctggct agactagaga 1440
cgcttgattg tgggaagcct cttgatgaag cagcatggga catggtatgt ggccagttat 1500
ccactgtatg aatatgtagt tgcctacaca gcaatctttc ctgaacatga atcctgatgt 1560
atgatattcc atttgtcagg acgatgttgc tggatgcttt gagtactttg cagatcttgc 1620
agaatccttg gacaaaaggc aaaatgcacc tgtctctctt ccaatggaaa actttaaatg 1680

ctatcttcgg aaagagccta tcggtgtagt tgggttgatc acaccttggt atttcacatt 1740
tttctctcat cctgcgctta tatttattta tgacccaagc atggtactaa atagtactag 1800
taacatgcat atactgaatg agtttacaac tttacatgat ttttttgaac tatgaaagtt 1860
gaagacattt gagattttat tcctcttctc ttgtgcaaac atattattgt ctcacaaatt 1920
gtacctagca gctactctct ccgtttcata ttataagtcg tttgactttt ttcctagtca 1980
aaatgtgtta agtttgacca agtttataga aaaatttagc aacatctaaa atatcaaagt 2040
catgttttag tgttttttca ggctctcatg taagcaattt tgatgtgccc tctcctttct 2100
tcttaatata atgatacaca gctcttgtgt attcaaagga aaatatatat atatataatg 2160
atacacacct ctcctccgtg ttaatgcagc tcatttgttc tgtcccggtt caaatatcta 2220
tttttctcat atgttgtcag catgattcac ttaatttagt atatagaaga tgccattatt 2280
tatgtctgga atcttactgc agaaggaaaa acaattgata acggaattga ttgcattcta 2340
atttgttgtt tctttgttat gttcttatcg acaattacaa atttgattct gagaatcatg 2400
ttcgggatgt gtatttctac tgcaggaact atcctctcct gatggcaaca tggaaggtag 2460
ctcctgccct ggctgctggc tgtacagctg tactaaaacc atctgaattg gcttccgtgt 2520
aagtttaaca tgttaacttg ttaatgtcat acccatgcta gttgcaatga catttgattt 2580
taaaatgttg tggcatgtcc atgctgcaag caatgtaatt tgaaatctct ctctatcatt 2640
aattaccagg acttgtttgg agcttgctga tgtgtgtaaa gaggttggtc ttccttcagg 2700
tgtgctaaac atagtgactg gattaggttc tgaagccggt gctcctttgt catcacaccc 2760
tggtgtagac aaggtacagc tattcctcct gtaatcatgt ataccccatc aatggaaatg 2820
atattcctct caatacatgg tttatgtttt ctgttaggtt gcatttactg ggagttatga 2880
aactggtaaa aagattatgg cttcagctgc tcctatggtt aaggtttgtt tccaaatttc 2940
tgtggatatt ttttgttctc tttctactaa ctctctatta tcaattctca atgttgtcct 3000
tttcttttaa ctcctttact ttttagaatt gtgatcaaga cactttgagc atcattctag 3060
tagccagttc tatcctgttt cttacctttt tatggttcgt cttttcttga cagcctgttt 3120
cactggaact tggtggaaaa agtcctatag tggtgtttga tgatgttgat gttgaaaaag 3180
gtacatgcca cttgctatga ttaactaatt ctgaagtgcg ggactttgta aagcacttaa 3240
ctgagctgga tgctagaccc ccaaaagccc tttttggtgt cttgggcttg ttgcagaaat 3300
actggtccca gacgagcagg atgcaagaaa attaactact tttgccactg attagtattt 3360
cttagaagtt acacctcaag gattagcaat actttcttaa aatgtgctat tgattaaaaa 3420
gatgtcctgt attatttgaa gcagatcttg tactggttga tcggcttgca tgaaaatatt 3480
gttgaggatt ataatgccat gccaactgag taaagaaaag agttgtaaaa tatgttatgc 3540
aacatgaata tatatgtgat ttcatttttc ctttttcttt tcgtggcaag gaaggcagtt 3600
aggaaggact gatgtgaaaa gcacaagtac tattcttagt tctggaaaac tgtgttcttt 3660
attttcctaa ctacaattca ccttgattag tcagtaactt gatattggca attctagctg 3720
attatgaatt ctgtttatat ttcactaatt ttgaatcttt aattacattt tatggttgaa 3780
atttaacgtt ttgtctggtt atggactctg tttgtattca ctcaatttgg atcttccatt 3840
agatttcatt gttggtcctt cttcttgtac agctgttgag tggactctct ttggttgctt 3900
ttggaccaat ggccagattt gcagtgcaac atcgcgtctt attcttcatg taagcattga 3960
atatatccgt caatcataat ctattgttgt acttgatttt ttttctgatc aactcctgag 4020
ttcagattat tatatgatgc cattactatt gcacagagcg aataaaattg tatttatgca 4080
cagcatgtat tttgagtaat atatgcattg cctattattt aatatataga ttgtagcact 4140
taattttgtg tccatgtctc tatgatgttt attactttat tattgccggc atgaagcaac 4200
tttgaactct atgttgatct tgaactaaaa ttgaaattaa ttggcttatt gctattaatg 4260
atatagcttt cagcttcttg ctcctgacca tgaaagtttt gcagaaaaaa atcgctaaag 4320
aatttcaaga aaggatggtt gcatgggcca aaaatattaa ggtgtcagat ccacttgaag 4380
agggttgcag gcttgggccc gttgttagtg aaggacaggt accacatgta aactttttct 4440
aaattcaaaa aagaaatgcc actgatcaat ggtaggtcct tccaagcctt attgctggat 4500
tgttgcactg ttttgtcaat tttgtgtaat atagttctga atgaattagt cggtgtatgc 4560
tcttgctagt tgctagtatg tggtacaggg tcttcctact ttgagcaaat tcgtgttaaa 4620
atgcattgat gaaaaggcca cctttccgta ggtttatctt gtcataattt aaaccccaat 4680
aaaattttaa ttttttgttt tgaccccatg gcactttaat gaaatcactt agccatgagc 4740
ttttgtatat attttcaaag caccagaatg tttagatggt ttgttggaaa tcttacacat 4800
cctattgcct tgtgtcagta tgagaagatt aagcaatttg tatctaccgc caaaagccaa 4860
ggtgctacca ttctgactgg tggggttaga cccaaggtaa taatctacta cacggttgta 4920
tatataggta cccacatatc attatgaagt agaaataatc ttgtatgttt ttgtcagcat 4980
ctggagaaag gtttctatat tgaacccaca atcattactg atgtcgatac atcaatgcaa 5040
atttggaggg aagaagtttt tggtccagtg ctctgtgtga aagaatttag cactgaagaa 5100
gaagccattg aattggccaa cgatactcag tgagtttttt ttttaataca gttcattgtc 5160
ctgttcaatc ttgcagcata tgtatatact ctgtggcata tgaacttatt ctgctactac 5220
tacttttgat agttatggtc tggctggtgc tgtgctttcc ggtgaccgcg agcgatgcca 5280
gagattaact gaggtatatc caagtgaagg gggttggcat tgtttgattc atatgacatg 5340
gttgcatcaa gctgatattc aagaatctca tttattactt gcattctatg catctccagt 5400
tcttccctgg actccggtca atgttaatat agtttgtttg ctagtagtat gctactccaa 5460

ttaagttgct cttcacttcc acatcatctg atccatgact ttatatttga cccctttttt 5520
ttgcaaaaga aagggaaata cttaacgaaa atttcctact gcaggagatc gatgccggaa 5580
ttatctgggt gaactgctcg caaccctgct tctgccaagc tccatggggc gggaacaagc 5640
gcagcggctt tggacgcgag ctcggagaag ggtgggtagc acacaacaat ctcactttaa 5700
aacaccattt cgatcgtctg atgatctcga cctgacatca tgcctttggt attttcattc 5760
acttttcagg ggcattgaca actacctaag cgtcaagcaa gtgacggagt acgcctccga 5820
tgagccgtgg ggatggtaca aatccccttc caagctgtaa 5860

<210> 18
<211> 2192
<212> DNA
<213> Oryza sativa L.OsSWEET14 genome DNA sequence (DNA sequence)

<400> 18
atggctggca tgtctcttca gcatccctgg gccttcgcct ttggtctcct aggtgtgttg 60
cctttgatct gatccaagga attctcttga gaattaatct tgcatggtta tttacttttg 120
ttgttattat tctctacatt tttaatcatg tacttttcca tgttccactt ttgttgccaa 180
agctactata tttttcctac caattcatcc aaaactacta tattatagca aggcagctag 240
tggatcgact ttgcactttt ggatgcaatt gtgagtggtt tacattagag gggccatgca 300
tagccaaata aattgtttgg caaaatatga tttcccttgt agttagcaca atattgtgat 360
ctttcattcc tttactcttt ttgttccacc accctcatta ttgcaaagcc ccctctttta 420
gaaccaaact aaagataaga aacatttgat tcatcattag aggagattag atatacatac 480
atactacaca tgcattgctg tgataagcct agccaggcac agaaagagcc aagacaaaag 540
gagctcaaaa gaaaaggggc tccaatatct tgcccggcct gatcagtgta acatgacgtc 600
acccacacaa ttattaaaag gatagatgca tatgtgttcg ttacgtatct ccttgtgtca 660
cgttgctgtg catggttcac tcgcttaatt ataacgtccg tcgcatttta tgcatgcact 720
tcgtttttag ttaatcaaat taaacaagat cctaacatat gatttctatt ttattctctt 780
tgcaggcaac atcatctcct tcatgaccta cctggcccca ctgtaagtcc cacacatccg 840
gatattttct tttctttagc gaaattttac attgctccag aaagtacctt ttacactaaa 900
atttcttata caccaccata aaattaatgt ggtactattg gtaccttatc aaaaatgata 960
aaattacctt tttcttcaga taaaaacact ccccgtctct gtataactaa gatgtagaca 1020
accaatacac atgtctagag ctaatcctat caattaacat tgctagtaaa tacaaggaaa 1080
aatataaaga attaaccaat tgcttggatg tcttgcaggc cgacgttcta caggatctac 1140
aagagcaagt cgacgcaggg gttccagtcg gtaccctacg tggtggcgct gttcagcgcg 1200
atgctgtgga tctactacgc gctgctcaag tccgacgagt gcctcctcat caccatcaac 1260
tccgctggct gcgtcatcga gaccatctac atcgccgtct acctcgtcta cgcccccaag 1320
aaggccaaga tgttcaccgc caagctcctc ctcctcgtca acgtcggcgt cttcggcctc 1380
atcctcctcc tcaccctcct cctctccgcc ggcgaccgcc gcatcgtggt tcttggttgg 1440
gtctgcgttg gcttctccgt cagcgtcttc gtcgcccccc ttagcatcat cgtaagaaac 1500
cctagaattg cctgtaaaca ataataactt tcggtctctg caccaactaa ggatgcacat 1560
atttacgcac cgcttttgct atctctgcag aggctggtgg tgcgcaccaa gagcgtggag 1620
ttcatgccgt tctcgctctc cttctccctc accatcagcg ccgtcgtctg gttcctctac 1680
ggcctcctca tcaaggacaa atatgtcgct gtgagtagct ccgattcgac ccgttcttct 1740
tcctaaattt tccgctgctc gatttaattt ctatttctaa ttgtggaaga ttgtttaatt 1800
atagtgatta tggctagctg ataaccgatt ttaatttctg atggtgatcc aaaatggaac 1860
agcttcccaa cgtgctgggc ttctccttcg gcgtcatcca gatggggctg tacgccatgt 1920
acaggaactc gacgcccaag gccgtgctga ccaaggaggt cgaggcggcg acggccaccg 1980
gcgacgacga ccactccgcc gccggcgtca aggagcacgt cgtcaacatc gccaagctct 2040
ctgccgccgt cgacgtcgtc aagacccgcg aggtgcaccc cgtcgacgtc gagtccccgc 2100
cggcagaggc gccgcctgag gaggacgaca aggccgccgc cgccaccgcc gccgccgtcg 2160
ccggcgccgg cgagaagaag gtagctgcat ga 2192

<210> 19
<211> 657
<212> PRT
<213> Solanum tuberosum L. StALS2 protein amino acid sequence (amino acid
sequence)

<400> 19
Met Ala Ala Ala Ser Pro Ser Pro Cys Phe Ser Lys Asn Leu Pro Pro
1               5                   10                  15
Ser Ser Ser Lys Ser Ser Ile Leu Leu Pro Lys Ser Thr Phe Thr Phe
            20                  25                  30

His Asn His Pro Lys Asn Thr Ser Pro Leu His Leu Thr His Thr Gln
            35                  40                  45
His His Ser Arg Phe Thr Val Ser Asn Val Ile Leu Ser Thr Thr Thr
    50                  55                  60
His Asn Asp Val Ser Glu Pro Glu Ile Phe Val Ser Arg Phe Ala Pro
65                  70                  75                  80
Asp Glu Pro Arg Lys Gly Cys Asp Val Leu Val Glu Ala Leu Glu Arg
                85                  90                  95
Glu Gly Val Lys Asp Val Phe Ala Tyr Pro Gly Gly Ala Ser Met Glu
            100                 105                 110
Ile His Gln Ala Leu Thr Arg Ser Asn Ile Ile Arg Asn Val Leu Pro
        115                 120                 125
Arg His Glu Gln Gly Gly Val Phe Ala Ala Glu Gly Tyr Ala Arg Ala
    130                 135                 140
Thr Gly Phe Pro Gly Val Cys Ile Ala Thr Ser Gly Pro Gly Ala Thr
145                 150                 155                 160
Asn Leu Val Ser Gly Leu Ala Asp Ala Leu Leu Asp Ser Ile Pro Ile
                165                 170                 175
Val Ala Ile Thr Gly Gln Val Pro Arg Arg Met Ile Gly Thr Asp Ala
            180                 185                 190
Phe Gln Glu Thr Pro Ile Val Glu Val Thr Arg Ser Ile Thr Lys His
        195                 200                 205
Asn Tyr Leu Val Met Asp Val Glu Asp Ile Pro Arg Val Val Arg Glu
    210                 215                 220
Ala Phe Phe Leu Ala Lys Ser Gly Arg Pro Gly Pro Val Leu Ile Asp
225                 230                 235                 240
Val Pro Lys Asp Ile Gln Gln Gln Leu Val Ile Pro Asn Trp Asp Gln
                245                 250                 255
Pro Met Arg Leu Pro Gly Tyr Ile Ser Arg Leu Pro Lys Leu Pro Asn
            260                 265                 270
Glu Met Leu Leu Glu Gln Ile Val Arg Leu Ile Ser Glu Ser Lys Lys
        275                 280                 285
Pro Val Leu Tyr Val Gly Gly Gly Cys Thr Gln Ser Ser Glu Glu Leu
    290                 295                 300
Arg Arg Phe Val Glu Leu Thr Gly Ile Pro Val Ala Ser Thr Leu Met
305                 310                 315                 320
Gly Leu Gly Thr Phe Pro Cys Gly Asp Glu Leu Ser Leu Gln Met Leu
                325                 330                 335
Gly Met His Gly Thr Val Tyr Ala Asn Tyr Ala Val Asp Ser Ser Asp
            340                 345                 350
Leu Leu Leu Ala Phe Gly Val Arg Phe Asp Asp Arg Val Thr Gly Lys
        355                 360                 365
Leu Glu Ala Phe Ala Ser Arg Ala Lys Ile Val His Ile Asp Ile Asp
    370                 375                 380
Ser Ala Glu Ile Gly Lys Asn Lys Gln Pro His Val Ser Ile Cys Ala
385                 390                 395                 400
Asp Ile Lys Leu Ala Leu Gln Gly Leu Asn Ser Ile Leu Glu Gly Lys
                405                 410                 415
Glu Gly Lys Leu Lys Leu Asp Phe Ser Ala Trp Arg Gln Glu Leu Thr
            420                 425                 430
Glu Gln Lys Val Lys Tyr Pro Leu Asn Tyr Lys Thr Phe Gly Glu Ala
        435                 440                 445
Ile Pro Pro Gln Tyr Ala Ile Gln Val Leu Asp Glu Leu Thr Asn Gly
    450                 455                 460
Asn Ala Ile Ile Ser Thr Gly Val Gly Gln His Gln Met Trp Ala Ala
465                 470                 475                 480
Gln Tyr Tyr Lys Tyr Lys Lys Pro Arg Gln Trp Leu Thr Ser Gly Gly
                485                 490                 495
Leu Gly Ala Met Gly Phe Gly Leu Pro Ala Ala Ile Gly Ala Ala Val
            500                 505                 510
Gly Arg Pro Gly Glu Ile Val Val Asp Ile Asp Gly Asp Gly Ser Phe
        515                 520                 525
Ile Met Asn Val Gln Glu Leu Ala Thr Ile Lys Val Glu Asn Leu Pro

530                 535                 540
Val Lys Ile Met Leu Leu Asn Asn Gln His Leu Gly Met Val Val Gln
545                 550                 555                 560
Trp Glu Asp Arg Phe Tyr Lys Ala Asn Arg Ala His Thr Tyr Leu Gly
                565                 570                 575
Asn Pro Ala Asn Glu Glu Glu Ile Phe Pro Asn Met Leu Lys Phe Ala
            580                 585                 590
Glu Ala Cys Gly Val Pro Ala Ala Arg Val Ser His Arg Asp Asp Leu
        595                 600                 605
Arg Ala Ala Ile Gln Lys Met Leu Asp Thr Pro Gly Pro Tyr Leu Leu
    610                 615                 620
Asp Val Ile Val Pro His Gln Glu His Val Leu Pro Met Ile Pro Ser
625                 630                 635                 640
Gly Gly Ala Phe Lys Asp Val Ile Thr Glu Gly Asp Gly Arg Arg Ser
                645                 650                 655
Tyr

<210>  20
<211>  1974
<212>  DNA
<213>  Solanum tuberosum L. StALS2 gene DNA sequence (DNA sequence)

<400>  20
atggcggctg catctccatc tccttgtttt tccaaaaacc tacctccatc ttcatcaaaa      60
tcttccatcc ttcttcccaa atctaccttt actttccaca atcaccccaa aaatacctca     120
ccccttcacc ttacccacac ccaacatcat agccgtttca ctgtctcaaa tgtcatccta     180
tcaaccacga cccataacga cgtttctgaa cccgaaatct tcgtttcacg tttcgcccct     240
gacgaaccca gaaagggttg tgatgttctt gtggaggcac ttgaaaggga aggggttaag     300
gatgtatttg catacccagg aggtgcttcc atggagattc atcaggcttt gacacgttcc     360
aatattattc gtaatgtgct gccacgtcat gaacagggtg gtgtgtttgc tgcagagggt     420
tacgcacggg ccactgggtt ccctggtgtt tgcattgcta catctggtcc gggagctacg     480
aatcttgtta gcggtcttgc ggatgctttg ttggatagta ttccgattgt tgctattacg     540
ggtcaagtgc cgaggaggat gattggtact gatgcgtttc aggaaactcc tattgttgag     600
gtaacgagat ccattacgaa gcataattat cttgttatgg atgtagagga tattcctagg     660
gttgttcgtg aagcgttttt tctagcgaaa tcgggacggc ctggaccggt tctgattgat     720
gttcctaagg atattcagca acaattggtg atacctaatt gggatcagcc aatgaggttg     780
cctggttaca tctctaggtt gcctaaattg cctaatgaga tgcttttgga acaaattgtt     840
aggctgattt cggagtcgaa gaagcctgtt ttgtatgtgg gtggtgggtg tacacaatcg     900
agtgaggagc tgagacgatt tgtggagctt acgggtattc ctgtggcgag tactttgatg     960
ggtcttggaa cttttccatg tggggatgag ctttctcttc aaatgttggg tatgcatggg    1020
actgtgtatg ctaattatgc ggtggatagt agtgatttgt tgcttgcatt tggggtgagg    1080
tttgatgatc gagttactgg taaattggaa gcttttgcta gccgagcgaa aattgtccac    1140
attgatattg attcggctga gattggaaag aacaagcaac ctcatgtttc catttgtgca    1200
gatatcaagt tggcattaca gggtttgaat tccatattgg agggtaaaga aggtaagctg    1260
aagttggact tttctgcttg gaggcaggag ttaacggagc agaaggtgaa gtacccattg    1320
aattataaga cttttggtga agccatccct ccacaatatg ctattcaggt tcttgatgag    1380
ttaactaacg gaaatgccat tattagtact ggtgtggggc aacaccaaat gtgggctgcc    1440
caatactata agtacaaaaa gccacgccaa tggttgacat ctggtggatt aggagcaatg    1500
ggatttggtt tgcctgctgc tataggtgcg gctgttggaa gaccgggtga gattgtggtt    1560
gacattgatg gtgacgggag ttttatcatg aatgtgcagg agttagcaac aattaaggtg    1620
gagaatctcc cagttaagat tatgttactg aataatcaac acttgggaat ggtggttcag    1680
tgggaggatc gattctataa ggctaacaga gcacacactt acttgggtaa tcctgctaat    1740
gaggaagaaa tcttccctaa tatgctgaaa tttgcagagg cttgtggcgt acctgctgca    1800
agagtgtcac acagggatga tcttagagct gccattcaaa agatgttaga cactcctggg    1860
ccatacttgt tggatgtgat tgtacctcat caggagcacg ttctacctat gattcccagt    1920
ggtggcgctt tcaaagatgt gattacggag ggtgatggga gacgttccta ttga          1974

<210>  21
<211>  1424
<212>  DNA
<213>  HBB gene DNA sequence (comprising intron)(DNA sequence)

<400>  21
atggtgcacc tgactcctga ggagaagtct gccgttactg ccctgtgggg caaggtgaac      60
gtggatgaag ttggtggtga ggccctgggc aggttggtat caaggttaca agacaggttt     120
aaggagacca atagaaactg ggcatgtgga gacagagaag actcttgggt ttctgatagg     180
cactgactct ctctgcctat tggtctattt tcccaccctt aggctgctgg tggtctaccc     240
ttggacccag aggttctttg agtcctttgg ggatctgtcc actcctgatg ctgttatggg     300
caaccctaag gtgaaggctc atggcaagaa agtgctcggt gcctttagtg atggcctggc     360
tcacctggac aacctcaagg gcacctttgc cacactgagt gagctgcact gtgacaagct     420
gcacgtggat cctgagaact tcagggtgag tctatgggac gcttgatgtt ttctttcccc     480
ttcttttcta tggttaagtt catgtcatag gaaggggata agtaacaggg tacagtttag     540
aatgggaaac agacgaatga ttgcatcagt gtggaagtct caggatcgtt ttagtttctt     600
ttatttgctg ttcataacaa ttgttttctt ttgtttaatt cttgctttct ttttttttct     660
tctccgcaat ttttactatt atacttaatg ccttaacatt gtgtataaca aaaggaaata     720
tctctgagat acattaagta acttaaaaaa aaactttaca cagtctgcct agtacattac     780
tatttggaat atatgtgtgc ttatttgcat attcataatc tccctacttt attttctttt     840
atttttaatt gatacataat cattatacat atttatgggt taaagtgtaa tgttttaata     900
tgtgtacaca tattgaccaa atcagggtaa ttttgcattt gtaattttaa aaaatgcttt     960
cttcttttaa tatacttttt tgtttatctt atttctaata ctttccctaa tctctttctt    1020
tcagggcaat aatgatacaa tgtatcatgc ctctttgcac cattctaaag aataacagtg    1080
ataatttctg ggttaaggca atagcaatat ctctgcatat aaatatttct gcatataaat    1140
tgtaactgat gtaagaggtt tcatattgct aatagcagct acaatccagc taccattctg    1200
cttttatttt atggttggga taaggctgga ttattctgag tccaagctag gcccttttgc    1260
taatcatgtt catacctctt atcttcctcc cacagctcct gggcaacgtg ctggtctgtg    1320
tgctggccca tcactttggc aaagaattca ccccaccagt gcaggctgcc tatcagaaag    1380
tggtggctgg tgtggctaat gccctggccc acaagtatca ctaa                      1424

<210>  22
<211>  444
<212>  DNA
<213>  HBB gene CDS sequence (DNA sequence)

<400>  22
atggtgcacc tgactcctga ggagaagtct gccgttactg ccctgtgggg caaggtgaac      60
gtggatgaag ttggtggtga ggccctgggc aggctgctgg tggtctaccc ttggacccag     120
aggttctttg agtcctttgg ggatctgtcc actcctgatg ctgttatggg caaccctaag     180
gtgaaggctc atggcaagaa agtgctcggt gcctttagtg atggcctggc tcacctggac     240
aacctcaagg gcacctttgc cacactgagt gagctgcact gtgacaagct gcacgtggat     300
cctgagaact tcaggctcct gggcaacgtg ctggtctgtg tgctggccca tcactttggc     360
aaagaattca ccccaccagt gcaggctgcc tatcagaaag tggtggctgg tgtggctaat     420
gccctggccc acaagtatca ctaa                                            444

<210>  23
<211>  147
<212>  PRT
<213>  HBB gene amino acid sequence (amino acid sequence)

<400>  23

## Description

### Priority information

The present application claims the benefits of the Chinese invention patent application No. 201911081617.X filed on November 7, 2019, the Chinese invention patent application No. 202010821877.2 filed on August 15, 2020, and the Chinese invention patent application No. 202010974151.2 filed on September 16, 2020. The above applications are incorporated herein by reference in their entireties.

### Technical Field

The present invention pertains to the technical field of genetic engineering, and specifically relates to a method for generating a site-specific mutation in an organism in the absence of an artificial DNA template and a use thereof.

### Background Art

The genetic engineering technology for modifying genome of organisms has been widely used in industrial and agricultural production, such as genetically modified microorganisms commonly used in the pharmaceutical and chemical fields, and genetically modified crops with insect-resistance and herbicide-resistance in the agricultural field. With the advent of site-specific nucleases, by introducing a targeted fragmentation into the genome of recipient organism and causing spontaneous repair, it has been possible to achieve site-specific editing of genome and more precise modification of genome.

Gene editing tools mainly include three types of sequence-specific nuclease (SSN): zinc finger nuclease (ZFN), transcription activator-like effector nuclease (TALEN) and clustered regularly interspaced short palindromic repeats (CRISPR) associated Cas system (CRISPR/Cas system). Sequence-specific nucleases are programmable nucleases that can generate DNA double-strand breaks (DSBs) at specific sites in genome. DNA double-strand breaks activate the endogenous DNA repair pathway to repair DNA damage in a cell, but the repair process easily leads to changes in the DNA sequence at target sites, thereby achieving the introduction of mutations at interesting sites. This technology enables biologists to accurately target a target gene and edit it. Among them, both ZFN and TALEN need to design specific recognition protein modules for the target sequence, thereby having low throughput and complex operations. However, Cas protein is universal in the CRISPR/Cas system, in which a guide RNA (gRNA) can be formed by a specific CRISPR-RNA (crRNA) designed for a target site alone or in conjunction with transactivating RNA (tracrRNA), or a single guide RNA (sgRNA) alone is enough, the crRNA and tracrRNA together or sgRNA alone can be assembled with the Cas protein to form a ribonucleoprotein complex (RNP), the target sequence is identified on the basis of protospacer adjacent motif (PAM) in genome, thereby realizing site-specific editing. And thus, it has become a main gene editing tool because of its simple operation, wide application range and high throughput.

Sequence-specific nuclease can produce DNA double-strand breaks at specific sites in the genome. These DNA double-strand breaks can be repaired into a variety of different repair types, which are mainly base insertions or deletions. For example, the two most common types of CRISPR/Cas9 editing events are inserting a base at the break or deleting a base at the break (Shen et al. 2018. Predictable and precise template-free CRISPR editing of pathogenic variants. Nature. DOI: 10.1038/s41586-018-0686-x). The insertion or deletion of bases in coding region will cause frameshift mutations, leading to loss of gene function. Therefore, the main purpose of the above gene editing tools is still to perform gene knockout.

It has been considered all the time that the use of sequence-specific nuclease alone cannot achieve mutations in type of base substitution. To this end, the prior art proposes three solutions: 1) adding an exogenous DNA fragment as a repair template to initiate a homologous recombination repair pathway; 2) fusing deaminase with Cas9 to sequentially develop single-base editing tools for C to T and A to G; 3) fusing reverse transcriptase with Cas9, using pegRNA to guide the synthesis and substitution of small DNA strand. However, the editing efficiency of these three solutions is significantly lower than the efficiency of gene knockout, and the simultaneously introduced exogenous DNA fragment and reverse transcriptase may readily cause concerns about biological safety. The off-target effect of single-base editing also restricts its potential application in cell therapy. Especially for long-term plant breeding projects, how to improve base substitution efficiency at target site while reducing the regulatory authorities' concerns about biosafety is a problem that needs to be resolved in the application of gene editing technology.

In summary, there are urgent technical needs in the fields of cell therapy and biological breeding for site-specific base substitution by using only targeted knockout of sequence-specific nuclease without introducing a foreign DNA fragment, especially through the non-transgenic transient editing system to efficiently complete site-specific base substitution editing.

### Summary of Invention

The invention provides a method for generating a site-specific mutation in an organism only by generating double-strand breaks on a genome and without providing an artificial DNA template, and use of the method.

The technical solutions adopted by the present invention are as follows:
A method for generating a new mutation in an organism, which comprises the following steps: sequentially generating two or more DNA breaks at a specific site in a genome of the organism and spontaneously repairing them respectively, wherein a later DNA break is generated based on a new sequence generated from a previous DNA break repair.

In a specific embodiment, the "DNA break" is achieved by delivering a nuclease with targeting property into a cell of an organism to contact with a specific site of genomic DNA.

In a specific embodiment, the "nuclease with targeting property" is a ZFN, TALEN or CRISPR/Cas system.

In a specific embodiment, the "sequentially generating two or more DNA breaks at a specific site" refers to that based on a new sequence generated from a previous DNA break repair event caused by ZFN or TALEN editing, a new ZFN or TALEN protein is designed to cut the site again.

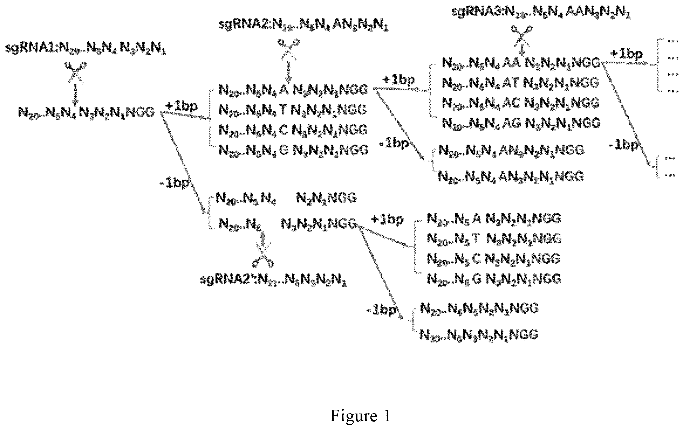

In another specific embodiment, "sequentially generating two or more DNA breaks at a specific site" refers to that based on a new sequence generated from a previous DNA break repair event caused by a CRISPR/Cas system, a new target RNA is designed to cut the site again. For example, a second cutting is performed at the site again by designing a new target RNA on the basis of a new sequence generated from a first break repair event of Cas9 editing. In a similar way, a third cutting is performed at the site by designing a new target RNA on the basis of a new sequence generated from a second break repair event and so on, as shown in Figure 1.

In a specific embodiment, the "two or more DNA breaks" are generated by sequentially delivering different targeted nucleases into recipient cells of different generations, wherein a mutant cell that has completed the previous editing is used as a recipient to receive the delivery of the targeted nuclease for the later editing, thereby performing second editing to generate site-specific mutation. This method is preferably used for ZFN and TALEN editing systems.

In another specific embodiment, the "two or more DNA breaks" are generated by delivering different targeted nucleases for different targets into a same recipient cell. This method is preferably used for CRISPR/Cas editing system.

In a specific embodiment, the "two or more DNA breaks" are generated when RNP complexes formed by a same CRISPR/Cas nuclease respectively with different gRNAs or sgRNAs sequentially cut corresponding target sequences.

In another specific embodiment, the "two or more DNA breaks" are generated when RNP complexes formed by each of two or more CRISPR/Cas nucleases that recognize different PAM sequences with respective gRNA or sgRNA, sequentially cut corresponding target sequences. For example, the PAM sequence recognized by Cas9 from *Streptococcus pyogenes* is "NGG" or "NAG" (Jinek et al., "A programmable dual-RNA-guided DNA endonuclease in adaptive bacterial immunity", Science 2012, 337:816- 821), the PAM sequence recognized by Cas9 of *Staphylococcus aureus* is "NNGRRT" or "NNGRR(N)", the PAM sequence recognized by *Neisseria meningitidis* Cas9 is NNNNGATT, and the PAM sequence recognized by *Streptococcus thermophilus* Cas9 is NNAGAAW. In this way, the editable window of a DNA molecule is larger.

In a specific embodiment, the targeted nuclease is any CRISPR/Cas nuclease capable of achieving genome editing.

In a specific embodiment, the targeted nuclease is in a form of DNA.

In another specific embodiment, the targeted nuclease is in a form of mRNA or protein instead of DNA. The protein form is preferred.

In a specific embodiment, the method for delivering targeted nucleases into a cell is selected from, but not limited to: 1) a PEG-mediated cell transfection method; 2) a liposome-mediated cell transfection method; 3) an electroporation transformation method; 4) a microinjection; 5) a gene gun bombardment; 6) an *Agrobacterium-*mediated transformation method.

In the method, a new target is designed based on a new sequence generated from the previous DNA break repair, and thus, mutations can be sequentially formed for many times at a specific site in the genome, thereby exponentially enriching types of repair events after DNA breaks, and generating new types of base substitution, deletion and insertion mutations that cannot be obtained by a single gene editing, so that the method is suitable to be used as a tool to create new mutations. This method can be briefly described as a method of programmed sequential cutting/editing or successive cutting/editing.

In a specific embodiment, a new target is designed based on a new specific sequence predicted to be generated from previous break repair at a specific site of organism genome, sequential editing is then performed, and thus a final possible mutation at the site can be designed in advance to achieve an expected editing.

In another specific embodiment, a new target is designed based on a new sequence predicted to be generated from previous break repair at a specific site of organism genome, sequential editing is then performed, and in addition to an expected editing event, a variety of different mutations can be generated eventually at the site, so that the method can be used as a tool to create various different mutations.

In another aspect, the present invention further provides a method for generating a new mutation in an organism, which comprises the following step: sequentially generating two or more DNA breaks at a specific site in a gene at the level of genome or chromosome of the organism, thereby achieving a precise base substitution, deletion or insertion.

In a specific embodiment, the "sequentially generating two or more DNA breaks at a specific site" refers to that a new target RNA designed based on a new sequence generated from a previous break repair event, and a cutting is performed again at the same site.

In a specific embodiment, the "DNA break" is achieved by a nuclease with targeting property.

The present invention further provides a new mutation obtained by the aforementioned method.

The present invention further provides a protein or biologically active fragment thereof that has the aforementioned new mutation.

The present invention further provides a nucleic acid, which comprises a nucleic acid sequence or complementary sequence thereof that encodes the protein or biologically active fragment thereof.

The present invention further provides a nucleic acid, which comprises:
(a) a nucleotide sequence encoding a target RNA, wherein
   the target RNA comprises at least two target RNAs, in which a first target RNA targets a DNA to cause a break in the DNA, and a latter target RNA targets a sequence generated from a previous break repair event and generates a break again.

In a specific embodiment, the nucleic acid further comprises (b) a nucleotide sequence encoding a Cas polypeptide.

In a specific embodiment, the target RNA is a sgRNA or gRNA.

In a specific embodiment, the Cas polypeptide and the target RNA are present in an in vitro cell or an ex vivo cell.

The present invention further provides a recombinant expression vector, which comprises the aforementioned nucleic acid and a promoter operably linked thereto.

The present invention further provides an expression cassette, which comprises the aforementioned nucleic acid.

The present invention further provides a host cell, which comprises the aforementioned expression cassette.

The present invention further provides an organism that is regenerated by using the aforementioned host cell.

The present invention further provides a method for lysing a target DNA, which comprises contacting the target DNA with a complex, wherein the complex comprises:
(a) a Cas polypeptide; and
(b) at least two target RNAs, in which a first targets RNA targets the DNA to cause a break in the DNA, and a latter target RNA targets a sequence generated from a previous break repair event and generates a break again.

In a specific embodiment, the target RNA is a sgRNA or gRNA.

In a specific embodiment, the target DNA is present in a bacterial cell, eukaryotic cell, plant cell or animal cell.

In a specific embodiment, the target DNA is a chromosomal DNA.

In a specific embodiment, the Cas polypeptide and the target RNA are present in an in vitro cell or an ex vivo cell.

In a specific embodiment, the contacting comprises introducing the following into a cell: (a) the Cas polypeptide or a polynucleotide encoding the Cas polypeptide, and (b) the target RNA or a DNA polynucleotide encoding the target RNA.

The present invention further provides a composition, which comprises:
(a) a Cas polypeptide, or a polynucleotide encoding the Cas polypeptide; and
(b) at least two target RNAs, or DNA polynucleotides encoding the target RNAs, wherein a first RNA targets a DNA to cause a break in the DNA, and a latter target RNA targets a sequence generated from a previous break repair event and generates a break again.

In a specific embodiment, the target RNA is a sgRNA or gRNA.

In a specific embodiment, the Cas polypeptide and the target RNA are present in an in vitro cell or an ex vivo cell.

The invention further provides a use of the composition in manufacture of a medicament for treatment of a disease.

The disease that can be treated with the composition of the present invention includes, but is not limited to, a disease caused by single gene mutation, such as genetic tyrosinemia type 1, phenylketonuria, progeria, sickle cell disease, etc. A spontaneous cell repair is induced by delivering into a cell the Cas protein and the crRNA or sgRNA composition that is expected to repair a pathogenic mutation site to produce a normal functional protein, and thus a therapeutic effect is obtained.

The present invention further provides a kit, which comprises:
(a) a Cas polypeptide, or a nucleic acid comprising a nucleotide sequence encoding the Cas polypeptide; and
(b) at least two target RNAs, or nucleic acids comprising nucleotide sequences encoding the target RNAs, wherein a first target RNA targets a DNA to cause a break in the DNA, and a latter target RNA targets a sequence generated from a previous break repair event and generates a break again;
   wherein (a) and (b) are in a same or separate containers.

In a specific embodiment, the target RNA is a sgRNA or gRNA.

In a specific embodiment, the target RNAs in (b) are in a same or separate containers.

The present invention further provides a method for screening editing events independent of exogenous transgenic markers, comprising the following steps:
1) two or more DNA breaks are sequentially generated in sequence at a specific site of a first target gene of a recipient cell and spontaneously repaired respectively, wherein a later DNA break is generated based on a new sequence generated from a repair of a previous DNA break;
2) certain editing events are generated after the specific site of the first target gene is sequentially cut and repaired, which can confer a mutant cell with a resistance to a certain selection pressure to produce a phenotypic selectable trait, a corresponding selection pressure is applied to make a selection for the trait, and a cell, tissue, organ or complete organism that contains such editing events is isolated;
3) optionally, in addition to the first target gene, a targeted nuclease for at least one second target gene is used to edit another target site at the same time, and an editing event of the second target gene is enriched and screened synchronously through the screening of the selectable trait generated by mutations of the first target gene, and a cell, tissue, organ or complete organism that simultaneously contains the editing events of the first target gene and of the at least one second target gene is isolated.

In a specific embodiment, the "first target gene" is a gene locus encoding at least one phenotypic selectable trait, wherein the at least one phenotypic selectable trait is a resistance/tolerance trait or a growth advantage trait.

In a specific embodiment, the "specific site of a first target gene" refers to a site at which a certain type of mutation is generated after sequential cuttings and repairs, which is capable of conferring the recipient cell with a resistance to a certain selection pressure to produce at least one phenotypic selectable resistance/tolerance trait or growth advantage trait.

In a specific embodiment, the "certain type of mutation" comprises substitution of single base, substitution of a plurality of bases, or insertion or deletion of an unspecified number of bases.

In a specific embodiment, the "certain selection pressure" may be an environmental pressure or a pressure resulted from an added compound; for example, the environmental pressure is high temperature, low temperature or hypoxia and the like; the pressure resulted from an added compound may be a pressure resulted from a salt ion concentration, antibiotic, cytotoxin, herbicide, etc.

In a specific embodiment, the "DNA break" is achieved by delivering a nuclease with a targeting property into a cell of an organism to contact with a specific site of genomic DNA.

In a specific embodiment, the "nuclease with a targeting property" is any CRISPR/Cas nuclease capable of performing genome editing.

In a specific embodiment, the feature, "two or more DNA breaks are sequentially generated in sequence at a specific site", refers to that based on a new sequence formed by a previous DNA break repair event generated by a CRISPR/Cas system, a new target RNA is designed to cut the site again.

In a specific embodiment, the "two or more DNA breaks" are generated when RNP complexes formed by a same CRISPR/Cas nuclease respectively with different gRNAs or sgRNAs sequentially cut corresponding target sequences.

In another specific embodiment, the "two or more DNA breaks" are generated when RNP complexes respectively formed by each of two or more CRISPR/Cas nucleases that recognize different PAM sequences with respective gRNA or sgRNA, sequentially cut corresponding target sequences. In this way, the editable window of a DNA molecule is larger.

In a specific embodiment, the "second target gene" refers to another gene that is different in coding from the first target gene.

In a specific embodiment, the "targeted nuclease for at least one second target gene" and the CRISPR/Cas nuclease used for generating DNA break at a specific site of the first target gene are the same.

In another specific embodiment, the "targeted nuclease for at least one second target gene" and the CRISPR/Cas nuclease used for generating DNA break at a specific site of the first target gene are different. In this way, there are more selectable editing sites on the second target gene.

In a specific embodiment, the targeted nuclease is in a form of DNA.

In another specific embodiment, the targeted nuclease is in a form of mRNA or protein instead of DNA. The protein form is preferred.

In a specific embodiment, the method for delivering targeted nuclease into cell is selected from, but not limited to: 1) a PEG-mediated cell transfection method; 2) a liposome-mediated cell transfection method; 3) an electroporation transformation method; 4) a microinjection; 5) a gene gun bombardment; or 6) an *Agrobacterium-*mediated transformation method.

The present invention further provides a method for non-transgenic transient editing of an organism genome, comprising the following steps:
1) a combination of at least two crRNA fragments or a combination of at least two sgRNA fragments is designed and synthesized for a specific site of a first target gene of a recipient cell, wherein the crRNA combination combined with tracrRNA or the sgRNA combination alone is capable of guiding a corresponding Cas protein to sequentially generate two or more DNA breaks at the specific site in the first target gene of the recipient cell and to spontaneously repair them respectively, wherein a later DNA break is generated based on a new sequence generated from a previous DNA break repair;
2) an appropriate amount of CRISPR/Cas protein or corresponding mRNA thereof is mixed with the combination of crRNA fragments and tracrRNA fragment or with the combination of sgRNA fragments alone as above designed and synthesized in advance that is capable of guiding a site-specific editing of the first target gene to generate endogenous selection markers, optionally, at least one of artificially synthesized crRNA and tracrRNA fragments or artificially synthesized sgRNA fragments targeting a second, third or more target genes is further added, and incubation is carried out in vitro to form an RNP complex;
3) the above RNP complex is delivered into the recipient cell and contacts with the specific site of genomic DNA to achieve gene editing;
4) according to a phenotypic selectable trait generated by a site-specific editing of the first target gene by the RNP complex, a corresponding selection pressure is applied to make a selection for the trait, and a cell, tissue, organ or complete organism that contains the editing event is isolated, and optionally, a cell, tissue, organ or complete organism that simultaneously contains the editing events of the first target gene and of the at least one of a second, third or more target genes is isolated.

In a specific embodiment, the "first target gene" is a gene locus encoding at least one phenotypic selectable trait, wherein the at least one phenotypic selectable trait is a resistance/tolerance trait or a growth advantage trait.

In a specific embodiment, the "specific site of the first target gene" refers to a site at which a certain type of mutation is generated after sequential cuttings and repairs, which is capable of conferring the recipient cell with a resistance to a certain selection pressure to produce at least one phenotypic selectable resistance/tolerance trait or growth advantage trait.

In a specific embodiment, the "certain type of mutation" comprises substitution of single base, substitution of a plurality of bases, or insertion or deletion of an unspecified number of bases.

In a specific embodiment, the "certain selection pressure" may be an environmental pressure or a pressure resulted from an added compound; for example, the environmental pressure is high temperature, low temperature or hypoxia and the like; the pressure resulted from an added compound may be a pressure resulted from a salt ion concentration, antibiotic, cytotoxin or herbicide, and the like.

In a specific embodiment, the CRISPR/Cas protein is any CRISPR/Cas nuclease capable of performing genome editing.

In a specific embodiment, the feature "to sequentially generate two or more DNA breaks at the specific site" refers to that based on a new sequence formed by a previous DNA break repair event generated by a CRISPR/Cas system, a new target RNA is designed to cut the site again.

In a specific embodiment, the "two or more DNA breaks" are generated when RNP complexes formed by a same CRISPR/Cas nuclease respectively with different gRNAs or sgRNAs sequentially cut corresponding target sequences.

In another specific embodiment, the "two or more DNA breaks" are generated when RNP complexes respectively formed by each of two or more CRISPR/Cas nucleases that recognize different PAM sequences with respective gRNA or sgRNA, sequentially cut corresponding target sequences. In this way, the editable window of a DNA molecule is larger.

In a specific embodiment, the "second, third or more target genes" refer to other genes that are different in coding from the first target gene.

In a specific embodiment, the "at least one of artificially synthesized crRNA and tracrRNA fragments or artificially synthesized sgRNA fragments targeting a second, third or more target genes" shares the same Cas protein with the crRNA or sgRNA targeting the first target gene.

In another specific embodiment, the "at least one of artificially synthesized crRNA and tracrRNA fragments or artificially synthesized sgRNA fragments targeting a second, third or more target genes" and the crRNA or sgRNA targeting the first target gene use Cas proteins that recognize different PAM sequences. In this way, there are more selectable editing sites on the second target gene.

In a specific embodiment, the method for delivering the RNP complex into cells is selected from, but not limited to: 1) a PEG-mediated cell transfection method; 2) a liposome-mediated cell transfection method; 3) an electroporation transformation method; 4) a microinjection; 5) a gene gun bombardment; and so on.

The present invention further provides a method for non-transgenic transient editing of a plant genome, comprising the following steps:
1) a combination of at least two crRNA fragments or a combination of at least two sgRNA fragments is designed and synthesized for a specific site of a first target gene of a recipient plant cell or tissue, the crRNA combination combined with tracrRNA or the sgRNA combination alone is capable of guiding a corresponding Cas protein to sequentially generate two or more DNA breaks at the specific site in the first target gene of the recipient cell and to spontaneously repair them respectively, wherein a later DNA break is generated based on a new sequence generated from a previous DNA break repair;
2) an appropriate amount of CRISPR/Cas protein or corresponding mRNA thereof is mixed with the combination of crRNA fragments and tracrRNA fragment or with the combination of sgRNA fragments alone as above designed and synthesized in advance that is capable of guiding the site-specific editing of the first target gene to generate endogenous selection markers, optionally, at least one of artificially synthesized crRNA and tracrRNA fragments or artificially synthesized sgRNA fragments targeting a second, third or more target genes is further added, and incubation is carried out in vitro to form an RNP complex;
3) the above RNP complex is delivered into the recipient plant cell or tissue and contacts with the specific site of genomic DNA to achieve gene editing;
4) according to a phenotypic selectable trait generated by a site-specific editing of the first target gene by the RNP complex, a corresponding selection pressure is applied to make a selection for the trait, and a cell, tissue, organ or complete plant that contains the editing event is isolated, and optionally, a cell, tissue, organ or complete plant that simultaneously contains the editing events of the first target gene and of the at least one of second, third or more target genes is isolated.

In a specific embodiment, the "first target gene" is a gene locus encoding at least one phenotypic selectable trait, wherein the at least one phenotypic selectable trait is a resistance/tolerance trait or a growth advantage trait.

In a specific embodiment, the "specific site of the first target gene" refers to a site at which a certain type of mutation is generated after sequential cuttings and repairs at the site, which can confer the recipient cell with a resistance to a certain selection pressure to produce at least one phenotypic selectable resistance/tolerance trait or growth advantage trait.

In a specific embodiment, the "certain type of mutation" comprises substitution of single base, substitution of a plurality of bases, or insertion or deletion of an unspecified number of bases.

In a specific embodiment, the "certain selection pressure" may be an environmental pressure or a pressure resulted from an added compound; for example, the environmental pressure is preferably high temperature, low temperature or hypoxia and the like; the pressure resulted from an added compound may be a pressure resulted from a salt ion concentration, antibiotic, cytotoxin, herbicide, etc.

In a specific embodiment, the "recipient plant cell or tissue" is any cell or tissue that can serve as a recipient for transient expression and can be regenerated into a complete plant through tissue culture. Specifically, the cell is a protoplast cell or a suspension cell; the tissue is preferably a callus, immature embryo, mature embryo, leaf, shoot tip, young spike, hypocotyl, etc.

In a specific embodiment, the CRISPR/Cas protein is any CRISPR/Cas nuclease capable of performing genome editing.

In a specific embodiment, the feature "to sequentially generate two or more DNA breaks at the specific site" refers to that based on a new sequence formed by a previous DNA break repair event generated by a CRISPR/Cas system, a new target RNA is designed to cut the site again.

In a specific embodiment, the "two or more DNA breaks" are generated when RNP complexes formed by a same CRISPR/Cas nuclease respectively with different gRNAs or sgRNAs sequentially cut corresponding target sequences.

In another specific embodiment, the "two or more DNA breaks" are generated when RNP complexes respectively formed by each of two or more CRISPR/Cas nucleases that recognize different PAM sequences with respective gRNA or sgRNA, sequentially cut corresponding target sequences. In this way, the editable window of a DNA molecule is larger.

In a specific embodiment, the "second, third or more target genes" refer to other genes that are different in coding from the first target gene.

In a specific embodiment, the "at least one of artificially synthesized crRNA and tracrRNA fragments or artificially synthesized sgRNA fragments targeting a second, third or more target genes" shares the same Cas protein with the crRNA or sgRNA targeting the first target gene.

In another specific embodiment, the "at least one of artificially synthesized crRNA and tracrRNA fragments or artificially synthesized sgRNA fragments targeting a second, third or more target genes" and the crRNA or sgRNA targeting the first target gene use Cas proteins that recognize different PAM sequences. In this way, there are more selectable editing sites on the second target gene.

In a specific embodiment, the method for delivering the RNP complex into plant cells is selected from, but not limited to: 1) a PEG-mediated protoplast transformation method; 2) a microinjection; 3) a gene gun bombardment; 4) a silicon carbide fiber-mediated method; 5) a vacuum infiltration method, or any other transient introduction method. The gene gun bombardment is preferred.

In a specific embodiment, the "first target gene" is at least one endogenous gene that encodes at least one phenotypic selectable trait selected from herbicide resistance/tolerance, wherein the herbicide resistance/tolerance is selected from the group consisting of resistance/tolerance to EPSPS inhibitor (including glyphosate); resistance/tolerance to glutamine synthesis inhibitor (including glufosinate); resistance/tolerance to ALS or AHAS inhibitor (including imidazoline or sulfonylurea); resistance/tolerance to ACCase inhibitor (including aryloxyphenoxypropionic acid (FOP)); resistance/tolerance to carotenoid biosynthesis inhibitor, including carotenoid biosynthesis inhibitors of phytoene desaturase (PDS) step, 4-hydroxyphenylpyruvate dioxygenase (HPPD) inhibitors or other carotenoid biosynthesis target inhibitors; resistance/tolerance to cellulose inhibitor; resistance/tolerance to lipid synthesis inhibitor; resistance/tolerance to long-chain fatty acid inhibitor; resistance/tolerance to microtubule assembly inhibitor; resistance/tolerance to photosystem I electron shunting agent; resistance/tolerance to photosystem II inhibitor (including carbamates, triazines and triazones); resistance/tolerance to PPO inhibitor; and resistance/tolerance to synthetic growth hormone (including dicamba, 2,4-D (i.e., 2,4-dichlorophenoxyacetic acid)). Wherein, the first target gene is selected from PsbA, ALS, EPSPS, ACCase, PPO, HPPD, PDS, GS, DOXPS, TIR1, AFB5, and some types of mutations generated after sequential cuttings and repairs at specific sites of these herbicide target genes may confer the recipient plant cells with resistance/tolerance to the corresponding herbicides.

In a specific embodiment, the "first target gene" is ALS, and the "specific site of gene" refers to site A122, P197, R198, D204, A205, D376, R377, W574, S653 or G654 in an *Arabidopsis* AtALS protein amino acid sequence (e.g., as shown in SEQ ID NO:1), and amino acid sites in an ALS protein of another plant which correspond to the above-mentioned amino acid sites by using the AtALS amino acid sequence as reference standard. The crRNA or sgRNA targets a target sequence comprising a sequence encoding an AtALS protein amino acid sequence site selected from the group consisting of A122, P197, R198, D204, A205, D376, R377, W574, S653, G654 or any combination thereof, and a target sequence comprising a sequence encoding an amino acid site in an ALS protein of another plant which corresponds to the above-mentioned amino acid sites, and any combination thereof, by using the AtALS amino acid sequence as reference standard. The ALS W574 site is preferred. The selection pressure is preferably a treatment with pyroxsulam or nicosulfuron.

In a specific embodiment, the "first target gene" is ACCase, and the "specific site of gene" refers to site 11781, E1874, N1878, W1999, W2027, 12041, D2078, C2088 or G2096 in an *Alopecurus myosuroides* AmACCase protein amino acid sequence (e.g., as shown in SEQ ID NO: 3, and the gene sequence is as shown in SEQ ID NO: 4), and amino acid sites in an ACCase protein of another monocotyledonous plant which correspond to the above-mentioned amino acid sites by using the AmACCase amino acid sequence as reference standard. The crRNA or sgRNA targets a target sequence comprising a sequence encoding an AmACCase amino acid sequence site selected from the group consisting of 11781, E1874, N1878, W1999, W2027, 12041, D2078, C2088, G2096 or any combination thereof, and a target sequence comprising a sequence encoding an amino acid site in an ACCase protein of another monocotyledonous plant which corresponds to the above-mentioned amino acid site, and any combination thereof, by using the AmACCase amino acid sequence as reference standard. ACCase W2027 site is preferred. The selection pressure is preferably a treatment with quizalofop-p-ethyl.

In a specific embodiment, the "first target gene" is HPPD, and the "specific site of gene" refers to site H141, L276, P277, N338, G342, R346, D370, P386, K418 or G419 in an *Oryza sativa* OsHPPD protein amino acid sequence (as shown in SEQ ID NO: 5, and the genome sequence is as shown in SEQ ID NO: 6), and amino acid sites in an HPPD protein of another plant which correspond to the above-mentioned amino acid sites by using the OsHPPD amino acid sequence as reference standard. The crRNA or sgRNA targets a target sequence comprising a sequence encoding an OsHPPD amino acid sequence site selected from the group consisting of H141, L276, P277, N338, G342, R346, D370, P386, K418, G419 or any combination thereof, and a target sequence comprising a sequence encoding an amino acid site in an HPPD protein of another plant which corresponds to the above-mentioned amino acid site, and any combination thereof, by using the OsHPPD amino acid sequence as reference standard. The selection pressure is preferably a treatment with biscarfentrazone.

In a specific embodiment, the "first target gene" is PPO, and the "specific site of gene" refers to site S128, V217, S223, V364, K373, L423, Y425 or W470 in an *Oryza sativa* OsPPO1 protein amino acid sequence (as shown in SEQ ID NO: 7, and the genome sequence is as shown in SEQ ID NO: 8), and amino acid sites in a PPO protein of another plant which correspond to the above-mentioned amino acid sites by using the amino acid sequence of OsPPO1 as reference standard. The crRNA or sgRNA targets a target sequence comprising a sequence encoding an OsPPO1 amino acid sequence site selected from the group consisting of S128, V217, S223, V364, K373, L423, Y425, W470 or any combination thereof, and a target sequence comprising a sequence of the above-mentioned amino acid sites corresponding to a PPO protein of another plant and any combination thereof using the OsPPO1 amino acid sequence as reference standard. The selection pressure is preferably a treatment with saflufenacil.

In a specific embodiment, the "first target gene" is TIR1, and the "specific site of gene" refers to site F93, F357, C413 or S448 in an *Oryza sativa* OsTIR1 protein amino acid sequence (as shown in SEQ ID NO: 9, and the genome sequence is as shown in SEQ ID NO: 10), and amino acid sites in a TIR1 protein of another plant which correspond to the above-mentioned amino acid sites by using the OsTIR1 amino acid sequence as reference standard. The crRNA or sgRNA targets a target sequence comprising a sequence encoding an OsTIR1 amino acid sequence site selected from the group consisting of F93, F357, C413, S448 or any combination thereof, and a target sequence comprising a sequence encoding an amino acid site in a TIR1 protein of another plant which corresponds to the above-mentioned amino acid site, and any combination thereof, by using the OsTIR1 amino acid sequence as reference standard. The selection pressure is preferably 2,4-D treatment.

The present invention further provides a non-transgenic transient editing system using the aforementioned method.

The present invention additionally provides a use of the aforementioned non-transgenic transient editing system as a selection marker.

The present invention additionally provides a use of the aforementioned non-transgenic transient editing system in treatment of a disease.

The present invention additionally provides a use of the aforementioned non-transgenic transient editing system in biological breeding.

The present invention additionally provides a genetically modified plant obtained by the aforementioned method, the genome of which contains an editing event of a first target gene, and the genetically modified plant is obtained in a non-transgenic manner.

The present invention additionally provides a genetically modified plant obtained by the aforementioned method, the genome of which contains an editing event of a first target gene, and further contains at least one second target gene editing event, and the genetically modified plant is obtained in a non-transgenic manner.

The present invention additionally provides a genetically modified plant obtained by the aforementioned method, the genome of which contains at least one second target gene editing event, and the genetically modified plant is obtained in a non-transgenic manner, wherein the first target gene editing event has been removed by genetic separation.

The present invention further provides a genome of the genetically modified plant obtained by the aforementioned method, the genome comprising: 1) an editing event of a first target gene; 2) an editing event of the first target gene and an editing event of at least one second target gene; or 3) at least one second target gene editing event, wherein the editing event of the first target gene has been removed by genetic separation; wherein the genetically modified plant is obtained in a non-transgenic manner.

Another aspect of the present invention provides a new plant gene mutation obtained by the aforementioned method.

The present invention also provides a new mutation generated in a plant, which comprises one or a combination of two or more of the following types:
substitution of aspartic acid at a site corresponding to *Arabidopsis* ALS376 with any other amino acid, substitution of tryptophan at a site corresponding to *Arabidopsis* ALS574 with any other amino acid, substitution of serine at a site corresponding to *Arabidopsis* ALS653 with any other amino acid, or substitution of serine at a site corresponding to *Arabidopsis* ALS654 with any other amino acid; or substitution of tryptophan at a site corresponding to *Alopecurus myosuroides* ACCase2027 with any other amino acid.

In a specific embodiment, wherein the aspartic acid at a site corresponding to *Arabidopsis* ALS376 is substituted by glutamic acid (D376E), the tryptophan at a site corresponding to *Arabidopsis* ALS574 is substituted by leucine or methionine (W574L or W574M), the serine at a site corresponding to *Arabidopsis* ALS653 is substituted by asparagine or arginine (S653N or S653R), or the glycine at a site corresponding to *Arabidopsis* ALS654 is substituted by aspartic acid (G654D), wherein the sites of amino acids are mentioned by using the sites of corresponding amino acids in *Arabidopsis thalianan* as reference; or, the tryptophan at a site corresponding to *Alopecurus myosuroides* ACCase2027 is substituted by leucine or cysteine (W2027L or W2027C), wherein the site of amino acid is mentioned by using the site of corresponding amino acid in *Alopecurus myosuroides* as reference.

In another specific embodiment, the mutation type is S653R/G654D, wherein the sites of amino acids are mentioned by using the sites of corresponding amino acids in *Arabidopsis thalianan* as reference.

In a specific embodiment, the aspartic acid at site 350 of *Oryza sativa* ALS is substituted by any other amino acid, the tryptophan at site 548 of *Oryza sativa* ALS is substituted by any other amino acid, or the tryptophan at site 561 of *Solanum tuberosum L.* ALS2 is substituted by any other amino acid; or, the tryptophan at site 2038 of *Oryza sativa* ACCase2 is substituted by any other amino acid.

In another specific embodiment, the aspartic acid at site 350 of *Oryza sativa* ALS is substituted by glutamic acid (D350E), the tryptophan at site 548 of *Oryza sativa* ALS is substituted by leucine or methionine (W548L or W548M), or the tryptophan at site 561 of *Solanum tuberosum L.* ALS2 is substituted by leucine or methionine (W561L or W561M); or, the tryptophan at site 2038 of *Oryza sativa* ACCase2 is substituted by leucine or cysteine (W2038L or W2038C), wherein the amino acid sequence of the *Oryza sativa* ALS protein is shown in SEQ ID NO: 11, the amino acid sequence of the *Solanum tuberosum L.* StALS2 protein is shown in SEQ ID NO: 19, and the amino acid sequence of the *Oryza sativa* ACCase2 protein is shown in SEQ ID NO: 13.

The present invention additionally provides a protein or biologically active fragment thereof that has the aforementioned new mutation.

The present invention also provides a nucleic acid, which comprises a nucleic acid sequence or complementary sequence thereof that encodes the protein or biologically active fragment thereof.

The present invention additionally provides a recombinant expression vector, which comprises the nucleic acid and a promoter operably linked thereto.

The present invention further provides an expression cassette, which comprises the nucleic acid.

The present invention further provides a plant cell, which comprises the expression cassette.

The present invention further provides a plant regenerated by using the plant cell.

Another aspect of the present invention provides a method for producing a plant with improved resistance or tolerance to herbicides, which comprises regenerating the plant cell into a plant.

Another aspect of the present invention provides a method for controlling weeds in a plant cultivation site, wherein the plant includes the aforementioned plant or a plant produced by the aforementioned method, wherein the method comprises applying to the cultivation site one or more herbicides in an effective amount to control the weeds.

Another aspect of the present invention also provides a use of the new mutation, the protein or biologically active fragment thereof, the nucleic acid, the recombinant expression vector or the expression cassette in improving resistance or tolerance of a plant cell, a plant tissue, a plant part or a plant to herbicides.

The present invention has the following excellent technical effects:
Based on sequences generated from new repair event generated by sequential editing, new targets can be designed, which can sequentially form mutations for many times at a specific site in the genome, thereby exponentially enriching the types of repair events after DNA breaks, and realizing new types of base substitution, deletion and insertion mutations that cannot be obtained by a single gene editing. That is, the programmed sequential cutting/editing scheme adopted by the present invention, which uses the sequence generated from a previous gene editing repair as the later gene editing target, can endow CRISPR/Cas with new functions of single-base editing and site-precise deletion and insertion through simple knockout.

The invention can realize the screening of gene editing events in the absence of exogenous markers, further realize the non-transgenic gene editing and can effectively screen editing events, and can greatly reduce the biological safety concerns of the method in cell therapy and biological breeding.

In particular, the plant non-transgenic transient editing method provided by the present invention only involves Cas protein and artificially synthesized small fragments of gRNA or sgRNA, without the participation of exogenous DNA in the whole process, and produces endogenous resistance selection markers by editing the first target gene via continuous targeting, so that the editing event can be effectively screened, actually without genetic modification operations involved, and thus the method is equivalent to chemical mutagenesis or radiation-induced breeding, also does not require continuous multiple generations of separation and detection of exogenous transgenic components, thereby shortening the breeding cycle, ensuring the biological safety, saving supervision and approval cost, and providing great application prospects in precise breeding of plants.

### Detailed description of invention

In the present invention, unless otherwise specified, the scientific and technical terms used herein have the meanings commonly understood by those skilled in the art. In addition, protein and nucleic acid chemistry, molecular biology, cell and tissue culture, microbiology, immunology related terms and laboratory procedures used herein are all terms and routine procedures widely used in the corresponding fields. At the same time, in order to better understand the present invention, definitions and explanations of related terms are provided below.

The term "genome" as used herein refers to all complements of genetic material (genes and non-coding sequences) present in each cell or virus or organelle of an organism, and/or complete genome inherited from a parent as a unit (haploid).

The term "gene editing" refers to strategies and techniques for targeted specific modification of any genetic information or genome of living organisms. Therefore, the term includes editing of gene coding regions, but also includes editing of regions other than gene coding regions of the genome. It also includes editing or modifying other genetic information of nuclei (if present) and cells.

The term "CRISPR/Cas nuclease" may be a CRISPR-based nuclease or a nucleic acid sequence encoding the same, including but not limited to: 1) Cas9, including SpCas9, ScCas9, SaCas9, xCas9, VRER-Cas9, EQR-Cas9, SpG-Cas9, SpRY-Cas9, SpCas9-NG, NG-Cas9, NGA-Cas9 (VQR), etc.; 2) Cas12, including LbCpf1, FnCpf1, AsCpf1, MAD7, etc., or any variant or derivative of the aforementioned CRISPR-based nuclease; preferably, wherein the at least one CRISPR-based nuclease comprises a mutation compared to the corresponding wild-type sequence, so that the obtained CRISPR-based nuclease recognizes a different PAM sequence. As used herein, "CRISPR-based nuclease" is any nuclease that has been identified in a naturally occurring CRISPR system, which is subsequently isolated from its natural background, and has preferably been modified or combined into a recombinant construct of interest, suitable as a tool for targeted genome engineering. As long as the original wild-type CRISPR-based nuclease provides DNA recognition, i.e., binding properties, any CRISPR-based nuclease can be used and optionally reprogrammed or otherwise mutated so as to be suitable for various embodiments of the invention.

The term "CRISPR" refers to a sequence-specific genetic manipulation technique that relies on clustered regularly interspaced short palindromic repeats, which is different from RNA interference that regulates gene expression at the transcriptional level.

"Cas9 nuclease" and "Cas9" are used interchangeably herein, and refer to RNA-guided nuclease comprising Cas9 protein or fragment thereof (for example, a protein containing the active DNA cleavage domain of Cas9 and/or the gRNA binding domain of Cas9). Cas9 is a component of the CRISPR/Cas (clustered regularly interspaced short palindrome repeats and associated systems) genome editing system. It can target and cut DNA target sequences under the guidance of guide RNA to form DNA double-strand breaks (DSB).

"Cas protein" or "Cas polypeptide" refers to a polypeptide encoded by Cas (CRISPR-associated) gene. Cas protein includes Cas endonuclease. Cas protein can be a bacterial or archaeal protein. For example, the types I to III CRISPR Cas proteins herein generally originate from prokaryotes; the type I and type III Cas proteins can be derived from bacteria or archaea species, and the type II Cas protein (i.e., Cas9) can be derived from bacterial species. "Cas proteins" include Cas9 protein, Cpf1 protein, C2c1 protein, C2c2 protein, C2c3 protein, Cas3, Cas3-HD, Cas5, Cas7, Cas8, Cas10, Cas12a, Cas12b, or a combination or complex thereof.

"Cas9 variant" or "Cas9 endonuclease variant" refers to a variant of the parent Cas9 endonuclease, wherein when associated with crRNA and tracRNA or with sgRNA, the Cas9 endonuclease variant retains the abilities of recognizing, binding to all or part of a DNA target sequence and optionally unwinding all or part of a DNA target sequence, nicking all or part of a DNA target sequence, or cutting all or part of a DNA target sequence. The Cas9 endonuclease variants include the Cas9 endonuclease variants described herein, wherein the Cas9 endonuclease variants are different from the parent Cas9 endonuclease in the following manner: the Cas9 endonuclease variants (when complexed with gRNA to form a polynucleotide-directed endonuclease complex capable of modifying a target site) have at least one improved property, such as, but not limited to, increased transformation efficiency, increased DNA editing efficiency, decreased off-target cutting, or any combination thereof, as compared to the parent Cas9 endonuclease (complexed with the same gRNA to form a polynucleotide-guided endonuclease complex capable of modifying the same target site).

The Cas9 endonuclease variants described herein include variants that can bind to and nick double-stranded DNA target sites when associated with crRNA and tracrRNA or with sgRNA, while the parent Cas endonuclease can bind to the target site and result in double strand break (cleavage) when associated with crRNA and tracrRNA or with sgRNA.

"Guide RNA" and "gRNA" are used interchangeably herein, and refer to a guide RNA sequence used to target a specific gene for correction using CRISPR technology, which usually consists of crRNA and tracrRNA molecules that are partially complementary to form a complex, wherein crRNA contains a sequence that has sufficient complementarity with the target sequence so as to hybridize with the target sequence and direct the CRISPR complex (Cas9+crRNA+tracrRNA) to specifically bind to the target sequence. However, it is known in the art that a single guide RNA (sgRNA) can be designed, which contains both the properties of crRNA and tracrRNA.

The terms "single guide RNA" and "sgRNA" are used interchangeably herein, and refer to the synthetic fusion of two RNA molecules, which comprises a fusion of a crRNA (CRISPR RNA) of a variable targeting domain (linked to a tracr pairing sequence hybridized to tracrRNA) and a tracrRNA (trans-activating CRISPR RNA). The sgRNA may comprise crRNA or crRNA fragments and tracrRNA or tracrRNA fragments of the type II CRISPR/Cas system that can form a complex with the type II Cas endonuclease, wherein the guide RNA/Cas endonuclease complex can guide the Cas endonuclease to a DNA target site so that the Cas endonuclease can recognize, optionally bind to the DNA target site, and optionally nick the DNA target site or cut (introduce a single-strand or double-strand break) the DNA target site.

In certain embodiments, the guide RNA(s) and Cas9 can be delivered to a cell as a ribonucleoprotein (RNP) complex. RNP is composed of purified Cas9 protein complexed with gRNA, and it is well known in the art that RNP can be effectively delivered to many types of cells, including but not limited to stem cells and immune cells (Addgene, Cambridge, MA, Mirus Bio LLC, Madison, WI).

The protospacer adjacent motif (PAM) herein refers to a short nucleotide sequence adjacent to a (targeted) target sequence (prespacer) recognized by the gRNA/Cas endonuclease system. If the target DNA sequence is not adjacent to an appropriate PAM sequence, the Cas endonuclease may not be able to successfully recognize the target DNA sequence. The sequence and length of PAM herein can be different depending on the Cas protein or Cas protein complex in use. The PAM sequence can be of any length, but is typically in length of 1, 2, 3, 4, 5, 6, 7, 8, 9, 10, 11, 12, 13, 14, 15, 16, 17, 18, 19 or 20 nucleotides.

As used herein, the term "organism" or "living body" includes animals, plants, fungi, bacteria, and the like.

As used herein, the term "host cell" includes plant cells, animal cells, fungal cells, bacterial cells, and the like.

In the present invention, "animal" includes but is not limited to vertebrates, such as humans, non-human mammals, birds, fish, reptiles, amphibians, etc., as well as invertebrates, such as insects.

In the present invention, the "plant" should be understood to mean any differentiated multicellular organism capable of performing photosynthesis, in particular monocotyledonous or dicotyledonous plants, for example, (1) food crops: Oryza spp., like Oryza sativa, Oryza latifolia, Oryza sativa, Oryza glaberrima; Triticum spp., like Triticum aestivum, T. Turgidumssp. durum; Hordeum spp., like Hordeum vulgare, Hordeum arizonicum; Secale cereale; Avena spp., like Avena sativa, Avena fatua, Avena byzantine, Avena fatua var.sativa, Avena hybrida; Echinochloa spp., like Pennisetum glaucum, Sorghum, Sorghum bicolor, Sorghum vulgare, Triticale, Zea mays or Maize, Millet, Rice, Foxtail millet, Proso millet, Sorghum bicolor, Panicum, Fagopyrum spp., Panicum miliaceum, Setaria italica, Zizania palustris, Eragrostis tef, Panicum miliaceum, Eleusine coracana; (2) legume crops: Glycine spp. like Glycine max, Soja hispida, Soja max, Vicia spp., Vigna spp., Pisum spp., field bean, Lupinus spp., Vicia, Tamarindus indica, Lens culinaris, Lathyrus spp., Lablab, broad bean, mung bean, red bean, chickpea; (3) oil crops: Arachis hypogaea, Arachis spp, Sesamum spp., Helianthus spp. like Helianthus annuus, Elaeis like Eiaeis guineensis, Elaeis oleifera, soybean, Brassicanapus, Brassica oleracea, Sesamum orientale, Brassica juncea, Oilseed rape, Camellia oleifera, oil palm, olive, castor-oil plant, Brassica napus L., canola; (4) fiber crops: Agave sisalana, Gossypium spp. like Gossypium, Gossypium barbadense, Gossypium hirsutum, Hibiscus cannabinus, Agave sisalana, Musa textilis Nee, Linum usitatissimum, Corchorus capsularis L, Boehmeria nivea (L.), Cannabis sativa, Cannabis sativa; (5) fruit crops: Ziziphus spp., Cucumis spp., Passiflora edulis, Vitis spp., Vaccinium spp., Pyrus communis, Prunus spp., Psidium spp., Punica granatum, Malus spp., Citrullus lanatus, Citrus spp., Ficus carica, Fortunella spp., Fragaria spp., Crataegus spp., Diospyros spp., Eugenia unifora, Eriobotrya japonica, Dimocarpus longan, Carica papaya, Cocos spp., Averrhoa carambola, Actinidia spp., Prunus amygdalus, Musa spp. (musa acuminate), Persea spp. (Persea Americana), Psidium guajava, Mammea Americana, Mangifera indica, Canarium album (Oleaeuropaea), Caricapapaya, Cocos nucifera, Malpighia emarginata, Manilkara zapota, Ananas comosus, Annona spp., Citrus reticulate (Citrus spp.), Artocarpus spp., Litchi chinensis, Ribes spp., Rubus spp., pear, peach, apricot, plum, red bayberry, lemon, kumquat, durian, orange, strawberry, blueberry, hami melon, muskmelon, date palm, walnut tree, cherry tree; (6) rhizome crops: Manihot spp., Ipomoea batatas, Colocasia esculenta, tuber mustard, Allium cepa (onion), eleocharis tuberose (water chestnut), Cyperus rotundus, Rhizoma dioscoreae; (7) vegetable crops: Spinacia spp., Phaseolus spp., Lactuca sativa, Momordica spp, Petroselinum crispum, Capsicum spp., Solanum spp. (such as Solanum tuberosum, Solanum integrifolium, Solanum lycopersicum), Lycopersicon spp. (such as Lycopersicon esculentum, Lycopersicon lycopersicum, Lycopersicon pyriforme), Macrotyloma spp., Kale, Luffa acutangula, lentil, okra, onion, potato, artichoke, asparagus, broccoli, Brussels sprouts, cabbage, carrot, cauliflower, celery, collard greens, squash, Benincasa hispida, Asparagus officinalis, Apium graveolens, Amaranthus spp., Allium spp., Abelmoschus spp., Cichorium endivia, Cucurbita spp., Coriandrum sativum, B.carinata, Rapbanus sativus, Brassica spp. (such as Brassica napus, Brassica rapa ssp., canola, oilseed rape, turnip rape, turnip rape, leaf mustard, cabbage, black mustard, canola (rapeseed), Brussels sprout, Solanaceae (eggplant), Capsicum annuum (sweet pepper), cucumber, luffa, Chinese cabbage, rape, cabbage, calabash, Chinese chives, lotus, lotus root, lettuce; (8) flower crops: Tropaeolum minus, Tropaeolum majus, Canna indica, Opuntia spp., Tagetes spp., Cymbidium (orchid), Crinum asiaticum L., Clivia, Hippeastrum rutilum, Rosa rugosa, Rosa Chinensis, Jasminum sambac, Tulipa gesneriana L., Cerasus sp., Pharbitis nil (L.) Choisy, Calendula officinalis L., Nelumbo sp., Bellis perennis L., Dianthus caryophyllus, Petunia hybrida, Tulipa gesneriana L., Lilium brownie, Prunus mume, Narcissus tazetta L., Jasminum nudiflorum Lindl., Primula malacoides, Daphne odora, Camellia japonica, Michelia alba, Magnolia liliiflora, Viburnum macrocephalum, Clivia miniata, Malus spectabilis, Paeonia suffruticosa, Paeonia lactiflora, Syzygium aromaticum, Rhododendron simsii, Rhododendron hybridum, Michelia figo (Lour.) Spreng., Cercis chinensis, Kerria japonica, Weigela florida, Fructus forsythiae, Jasminum mesnyi, Parochetus communis, Cyclamen persicum Mill., Phalaenophsis hybrid, Dendrobium nobile, Hyacinthus orientalis, Iris tectorum Maxim, Zantedeschia aethiopica, Calendula officinalis, Hippeastrum rutilum, Begonia semperflorenshybr, Fuchsia hybrida, Begonia maculataRaddi, Geranium, Epipremnum aureum; (9) medicinal crops: Carthamus tinctorius, Mentha spp., Rheum rhabarbarum, Crocus sativus, Lycium chinense, Polygonatum odoratum, Polygonatum Kingianum, Anemarrhena asphodeloides Bunge, Radix ophiopogonis, Fritillaria cirrhosa, Curcuma aromatica, Amomum villosum Lour., Polygonum multiflorum, Rheum officinale, Glycyrrhiza uralensis Fisch, Astragalus membranaceus, Panax ginseng, Panax notoginseng, Acanthopanax gracilistylus, Angelica sinensis, Ligusticum wallichii, Bupleurum sinenses DC., Datura stramonium Linn., Datura metel L., Mentha haplocalyx, Leonurus sibiricus L., Agastache rugosus, Scutellaria baicalensis, Prunella vulgaris L., Pyrethrum carneum, Ginkgo biloba L., Cinchona ledgeriana, Hevea brasiliensis (wild), Medicago sativa Linn, Piper Nigrum L., Radix Isatidis, Atractylodes macrocephala Koidz; (10) raw material crops: Hevea brasiliensis, Ricinus communis, Vernicia fordii, Morus alba L., Hops Humulus lupulus, Betula, Alnus cremastogyne Burk., Rhus verniciflua stokes; (11) pasture crops: Agropyron spp., Trifolium spp., Miscanthus sinensis, Pennisetum sp., Phalaris arundinacea, Panicum virgatum, prairiegrasses, Indiangrass, Big bluestem grass, Phleum pratense, turf, cyperaceae (Kobresia pygmaea, Carex pediformis, Carex humilis), Medicago sativa Linn, Phleum pratense L., Medicago sativa, Melilotus suavcolen, Astragalus sinicus, Crotalaria juncea, Sesbania cannabina, Azolla imbircata, Eichhornia crassipes, Amorpha fruticosa, Lupinus micranthus, Trifolium, Astragalus adsurgens pall, Pistia stratiotes linn, Alternanthera philoxeroides, Lolium; (12) sugar crops: Saccharum spp., Beta vulgaris; (13) beverage crops: Camellia sinensis, Camellia Sinensis, tea, Coffee (Coffea spp.), Theobroma cacao, Humulus lupulus Linn.; (14) lawn plants: Ammophila arenaria, Poa spp.(Poa pratensis (bluegrass)), Agrostis spp. (Agrostis matsumurae, Agrostis palustris), Lolium spp. (Lolium), Festuca spp. (Festuca ovina L.), Zoysia spp. (Zoysiajaponica), Cynodon spp. (Cynodon dactylon/bermudagrass), Stenotaphrum secunda tum (Stenotaphrum secundatum), Paspalum spp., Eremochloa ophiuroides (centipedegrass), Axonopus spp. (carpetweed), Bouteloua dactyloides (buffalograss), Bouteloua var. spp. (Bouteloua gracilis), Digitaria sanguinalis, Cyperusrotundus, Kyllingabrevifolia, Cyperusamuricus, Erigeron canadensis, Hydrocotylesibthorpioides, Kummerowiastriata, Euphorbia humifusa, Viola arvensis, Carex rigescens, Carex heterostachya, turf; (15) tree crops: Pinus spp., Salix spp., Acer spp., Hibiscus spp., Eucalyptus spp., Ginkgo biloba, Bambusa sp., Populus spp., Prosopis spp., Quercus spp., Phoenix spp., Fagus spp., Ceiba pentandra, Cinnamomum spp., Corchorus spp., Phragmites australis, Physalis spp., Desmodium spp., Populus, Hedera helix, Populus tomentosa Carr, Viburnum odoratissinum, Ginkgo biloba L., Quercus, Ailanthus altissima, Schima superba, Ilex pur-purea, Platanus acerifolia, ligustrum lucidum, Buxus megistophylla Levl., Dahurian larch, Acacia mearnsii, Pinus massoniana, Pinus khasys, Pinus yunnanensis, Pinus finlaysoniana, Pinus tabuliformis, Pinus koraiensis, Juglans nigra, Citrus limon, Platanus acerifolia, Syzygium jambos, Davidia involucrate, Bombax malabarica L., Ceiba pentandra (L.), Bauhinia blakeana, Albizia saman, Albizzia julibrissin, Erythrina corallodendron, Erythrina indica, Magnolia gradiflora, Cycas revolute, Lagerstroemia indica, coniferous, macrophanerophytes, Frutex; (16) nut crops: Bertholletia excelsea, Castanea spp., Corylus spp., Carya spp., Juglans spp., Pistacia vera, Anacardium occidentale, Macadamia (Macadamia integrifolia), Carya illinoensis Koch, Macadamia, Pistachio, Badam, other plants that produce nuts; (17) others: arabidopsis thaliana, Bra chiaria eruciformis, Cenchrus echinatus, Setaria faberi, eleusine indica, Cadaba farinose, algae, Carex elata, ornamental plants, Carissa macrocarpa, Cynara spp., Daucus carota, Dioscorea spp., Erianthus sp., Festuca arundinacea, Hemerocallis fulva, Lotus spp., Luzula sylvatica, Medicago sativa, Melilotus spp., Morus nigra, Nicotiana spp., Olea spp., Ornithopus spp., Pastinaca sativa, Sambucus spp., Sinapis sp., Syzygium spp., Tripsacum dactyloides, Triticosecale rimpaui, Viola odorata, and the like.

In a specific embodiment, the plant is selected from rice, corn, wheat, soybean, sunflower, sorghum, rape, alfalfa, cotton, barley, millet, sugarcane, tomato, tobacco, cassava, potato, sweet potato, Chinese cabbage, cabbage, cucumber, Chinese rose, *Scindapsus aureus*, watermelon, melon, strawberry, blueberry, grape, apple, citrus, peach, pear, banana, etc.

As used herein, the term "plant" includes a whole plant and any progeny, cell, tissue or part of plant. The term "plant part" includes any part of a plant, including, for example, but not limited to: seed (including mature seed, immature embryo without seed coat, and immature seed); plant cutting; plant cell; plant cell culture; plant organ (e.g., pollen, embryo, flower, fruit, bud, leaf, root, stem, and related explant). Plant tissue or plant organ can be a seed, callus tissue, or any other plant cell population organized into a structural or functional unit. The plant cell or tissue culture can regenerate a plant that has the physiological and morphological characteristics of the plant from which the cell or tissue is derived, and can regenerate a plant that has substantially the same genotype as the plant. In contrast, some plant cells cannot regenerate plants. The regenerable cells in plant cells or tissue cultures can be embryos, protoplasts, meristematic cells, callus, pollen, leaves, anthers, roots, root tips, silk, flowers, kernels, spikes, cobs, husks, or stems.

The plant parts comprise harvestable parts and parts that can be used to propagate offspring plants. The plant parts that can be used for propagation include, for example, but not limited to: seeds; fruits; cuttings; seedlings; tubers; and rootstocks. The harvestable parts of plants can be any of useful parts of plants, including, for example, but not limited to: flowers; pollen; seedlings; tubers; leaves; stems; fruits; seeds; and roots.

The plant cells are the structural and physiological units of plants. As used herein, the plant cells include protoplasts and protoplasts with partial cell walls. The plant cells may be in a form of isolated single cells or cell aggregates (e.g., loose callus and cultured cells), and may be part of higher order tissue units (e.g., plant tissues, plant organs, and plants). Therefore, the plant cells can be protoplasts, gamete-producing cells, or cells or collection of cells capable of regenerating a whole plant. Therefore, in the embodiments herein, a seed containing a plurality of plant cells and capable of regenerating into a whole plant is considered as a "plant part".

As used herein, the term "protoplast" refers to a plant cell whose cell wall is completely or partially removed and whose lipid bilayer membrane is exposed. Typically, the protoplast is an isolated plant cell without cell wall, which has the potential to regenerate a cell culture or a whole plant.

The plant "offspring" includes any subsequent generations of the plant.

The term "bacteria" means all prokaryotes, including all organisms in the Kingdom Procaryotae. The term "bacteria" includes all microorganisms considered to be bacteria, including Mycoplasma, Chlamydia, Actinomyces, Streptomyce, and Rickettsia. All forms of bacteria are included in this definition, including cocci, bacilli, spirilla, spheroplasts, protoplasts, etc. The term also includes prokaryotes that are Gram-negative or Gram-positive. "Gram-negative" and "Gram-positive" mean a staining pattern using Gram staining methods well known in the art (see, for example, Finegold and Martin, Diagnostic Microbiology, 6th Ed., CV Mosby St. Louis, pp. 13 -15[1982]). "Gram-positive bacteria" are bacteria that can retain the original dye used for Gram staining, causing the stained cells to appear dark blue to purple under a microscope. "Gram-negative bacteria" do not retain the original dye used for Gram staining, but can be stained with a counter stain. Therefore, Gram-negative bacteria appear red after the Gram staining reaction.

As used herein, the term "fungi" refers to eukaryotic organisms such as molds and yeasts, including dimorphic fungi.

The terms "herbicide tolerance" and "herbicide resistance" can be used interchangeably, and both refer to herbicide tolerance and herbicide resistance. "Improvement in herbicide tolerance" and "improvement in herbicide resistance" mean that the tolerance or resistance to the herbicide is improved compared to a plant containing wild-type gene.

The term "wild-type" refers to a nucleic acid molecule or protein that can be found in nature.

In the present invention, the term "cultivation site" comprises a site where the plant of the present invention is cultivated, such as soil, and also comprises, for example, plant seeds, plant seedlings and grown plants. The term "weed-controlling effective amount" refers to an amount of herbicide that is sufficient to affect the growth or development of the target weed, for example, to prevent or inhibit the growth or development of the target weed, or to kill the weed. Advantageously, the weed-controlling effective amount does not significantly affect the growth and/or development of the plant seeds, plant seedlings or plants of the present invention. Those skilled in the art can determine such weed-controlling effective amount through routine experiments.

The term "Target DNA" as used herein refers to a DNA polynucleotide comprising a "target site" or "target sequence".

The term "lysis" means the cleavage of the covalent backbone of a DNA molecule. The lysis can be initiated by a variety of methods, including but not limited to enzymatic or chemical hydrolysis of phosphodiester bonds. Both single-strand and double-strand lysis is possible, and double-strand lysis may occur due to two distinct single-strand lysis events. The DNA lysis may result in blunt or staggered ends. In certain embodiments, a complex comprising DNA-targeting RNA and a site-specific modification polypeptide is used for a targeted double-strand DNA lysis.

The term "gene" comprises a nucleic acid fragment expressing a functional molecule (such as, but not limited to, specific protein), including regulatory sequences before (5' non-coding sequences) and after (3' non-coding sequences) a coding sequence.

The DNA sequence that "encodes" a specific RNA is a DNA nucleic acid sequence that can be transcribed into RNA. The DNA polynucleotides can encode a RNA (mRNA) that can be translated into a protein, or the DNA polynucleotides can encode a RNA that cannot be translated into a protein (for example, tRNA, rRNA, or DNA-targeting RNA; which are also known as "non-coding" RNA or " ncRNA").

The terms "polypeptide", "peptide" and "protein" are used interchangeably in the present invention, and refer to a polymer of amino acid residues. The terms are applied to amino acid polymers in which one or more amino acid residues are artificially chemical analogs of corresponding and naturally occurring amino acids, as well as to naturally occurring amino acid polymers. The terms "polypeptide", "peptide", "amino acid sequence" and "protein" may also include their modification forms, including but not limited to glycosylation, lipid linkage, sulfation, γ-carboxylation of glutamic acid residue, hydroxylation and ADP-ribosylation.

The term "biologically active fragment" refers to a fragment that has one or more amino acid residues deleted from the N and/or C-terminus of a protein while still retaining its functional activity.

For the terms related to amino acid substitution used in the description, the first letter represents a naturally occurring amino acid at a certain position in a specific sequence, the following number represents the position in the corresponding sequence, and the second letter represents a different amino acid for substituting the naturally occurring amino acid. For example, W574L means that tryptophan at position 574 is substituted by leucine. For double or multiple mutations, each mutation is separated by "/".

The terms "polynucleotide" and "nucleic acid" are used interchangeably and comprise DNA, RNA or hybrids thereof, which may be double-stranded or single-stranded.

The terms "nucleotide sequence" and "nucleic acid sequence" both refer to the sequence of bases in DNA or RNA.

As used in the present invention, "expression cassette", "expression vector" and "expression construct" refer to a vector such as a recombinant vector suitable for expression of a nucleotide sequence of interest in a plant. The term "expression" refers to the production of a functional product. For example, the expression of a nucleotide sequence may refer to the transcription of the nucleotide sequence (such as transcription to generate mRNA or functional RNA) and/or the translation of RNA into a precursor or mature protein.

The "expression construct" of the present invention can be a linear nucleic acid fragment, a circular plasmid, a viral vector, or, in some embodiments, can be an RNA (such as mRNA) that can be translated.

The "expression construct" of the present invention may comprise regulatory sequences and nucleotide sequences of interest from different sources, or regulatory sequences and nucleotide sequences of interest from the same source but arranged in a way different from those normally occurring in nature.

The terms "recombinant expression vector" or "DNA construct" are used interchangeably herein and refer to a DNA molecule comprising a vector and at least one insert. Recombinant expression vectors are usually produced for the purpose of expression and/or propagation of the insert or for the construction of other recombinant nucleotide sequences. The insert may be operably or may be inoperably linked to a promoter sequence and may be operably or may be inoperably linked to a DNA regulatory sequence.

The terms "regulatory sequence" and "regulatory element" can be used interchangeably and refer to a nucleotide sequence that is located at the upstream (5' non-coding sequence), middle or downstream (3' non-coding sequence) of a coding sequence, and affects the transcription, RNA processing, stability or translation of a related coding sequence. Plant expression regulatory elements refer to nucleotide sequences that can control the transcription, RNA processing or stability or translation of a nucleotide sequence of interest in plants.

The regulatory sequences may include, but are not limited to, promoters, translation leader sequences, introns, and polyA recognition sequences.

The term "promoter" refers to a nucleic acid fragment capable of controlling the transcription of another nucleic acid fragment. In some embodiments of the present invention, the promoter is a promoter capable of controlling gene transcription in plant cells, regardless of whether it is derived from plant cells. The promoter can be a constitutive promoter or a tissue-specific promoter or a developmentally regulated promoter or an inducible promoter.

The term "constitutive promoter" refers to a promoter that will generally cause gene expression in most cell types in most cases. "Tissue-specific promoter" and "tissue-preferred promoter" are used interchangeably, and refer to a promoter that is mainly but not necessarily exclusively expressed in a tissue or organ, and also expressed in a specific cell or cell type. "Developmentally regulated promoter" refers to a promoter whose activity is determined by a developmental event. "Inducible promoter" responds to an endogenous or exogenous stimulus (environment, hormone, chemical signal, etc.) to selectively express an operably linked DNA sequence.

As used herein, the term "operably linked" refers to a connection of a regulatory element (for example, but not limited to, promoter sequence, transcription termination sequence, etc.) to a nucleic acid sequence (for example, a coding sequence or open reading frame) such that the transcription of the nucleotide sequence is controlled and regulated by the transcription regulatory element. The techniques for operably linking regulatory element region to nucleic acid molecule are known in the art.

The "introducing" a nucleic acid molecule (such as a plasmid, linear nucleic acid fragment, RNA, etc.) or protein into a plant refers to transforming a cell of the plant with the nucleic acid or protein so that the nucleic acid or protein can function in the plant cell. The term "transformation" used in the present invention comprises stable transformation and transient transformation.

The term "stable transformation" refers to that the introduction of an exogenous nucleotide sequence into a plant genome results in a stable inheritance of the exogenous gene. Once stably transformed, the exogenous nucleic acid sequence is stably integrated into the genome of the plant and any successive generations thereof.

The term "transient transformation" refers to that the introduction of a nucleic acid molecule or protein into a plant cell to perform function does not result in a stable inheritance of the foreign gene. In transient transformation, the exogenous nucleic acid sequence is not integrated into the genome of the plant.

Unless otherwise defined, all technical and scientific terms used herein have the same meanings as commonly understood by those of ordinary skill in the art to which the present invention pertains. Although any methods and materials similar or equivalent to those described herein can also be used in the practice or testing of the present invention, the preferred methods and materials are now described.

All publications and patents cited in this description are incorporated herein by reference as if each individual publication or patent is exactly and individually indicated to be incorporated by reference, and is incorporated herein by reference to disclose and describe methods and/or materials related to the publications cited. The citation of any publication which it was published before the filing date should not be interpreted as an admission that the present invention is not eligible to precede the publications of the existing invention. In addition, the publication date provided may be different from the actual publication date, which may require independent verification.

Unless specifically stated or implied, as used herein, the terms "a", "a/an" and "the" mean "at least one." All patents, patent applications, and publications mentioned or cited herein are incorporated herein by reference in their entirety, with the same degree of citation as if they were individually cited.

### Brief Description of Drawings

Figure 1 shows a schematic diagram of the method for generating new mutations in organisms according to the present invention. In the figure, only Cas9 with NGG as PAM is taken as an example; in the same way, other Cas9 variants with different PAM (such as NG) can also be used.

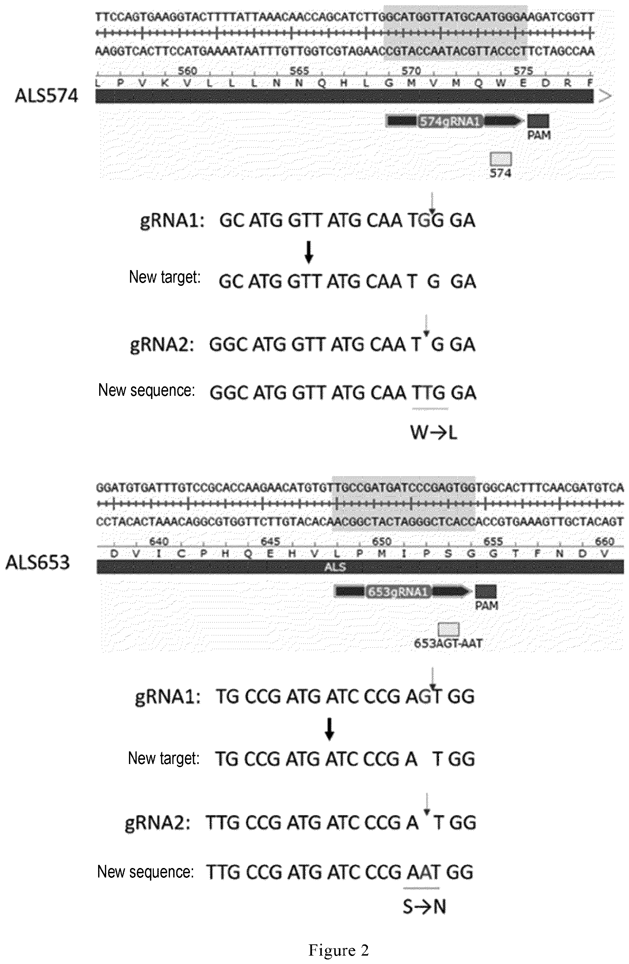

Figure 2 shows a schematic diagram of gRNA design at the ALS gene sites W574 and S653 of *Arabidopsis thaliana.*

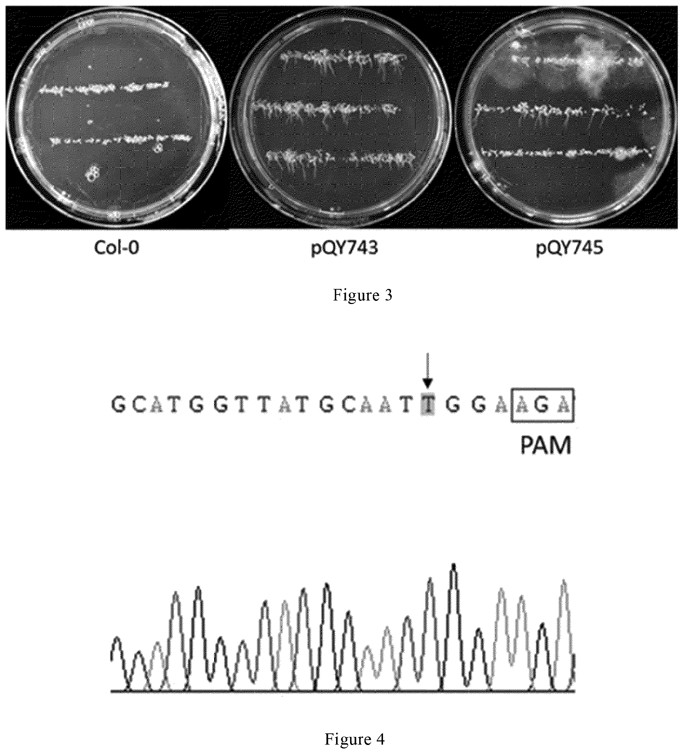

Figure 3 shows the *Arabidopsis thaliana* T2 generation herbicide-resistant strains transformed with two different programmed sequential cutting/editing vectors. pQY743 and pQY745 are vector numbers. Resistant strains were capable of rooting normally, but the wild-type Col-0 and non-resistant strains were not.
Figure 4 shows a diagram of sequencing peaks of the ALS gene of the T2 generation strain of *Arabidopsis thaliana* resistant to imazapic, in which the T indicated by the arrow was mutated from G, resulting in a W574L mutation.

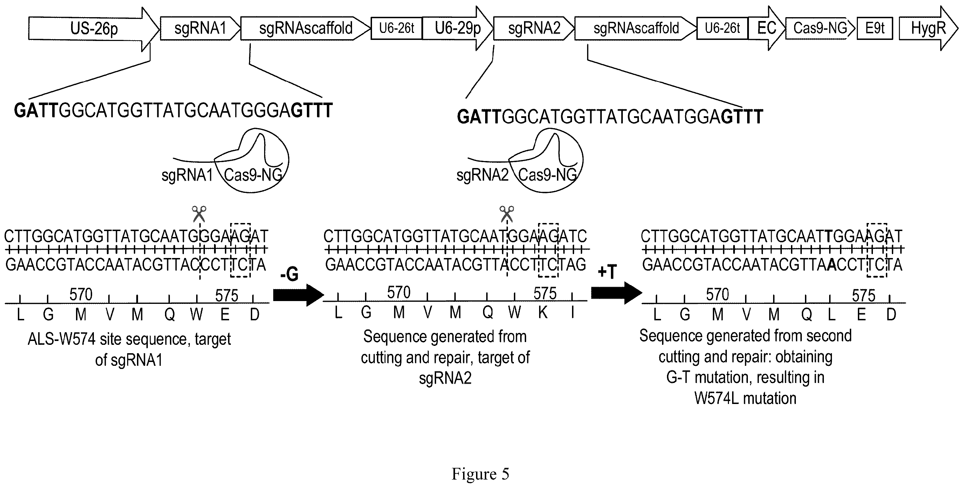

Figure 5 shows a design of the programmed sequential cutting/editing scheme at W574 site of ALS gene of *Arabidopsis thaliana.* The T-DNA sequence expressed four genes, sgRNA1, sgRNA2, Cas9 and HygR. Among them, sgRNA1 and Cas9 formed a complex, which cut the W574 codon of ALS in the genome, and was expected to form -G genotype through cellular spontaneous repair. This new sequence could be recognized and cut by the complex formed by sgRNA2 and Cas9, and formed +T genotype through cellular spontaneous repair, leading to W574L.

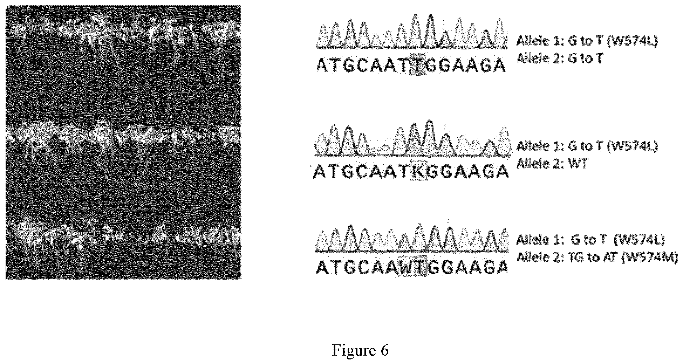

Figure 6 shows the resistant seedlings of *Arabidopsis thaliana* screened by imazapic and the sequencing results of ALS W574 site.

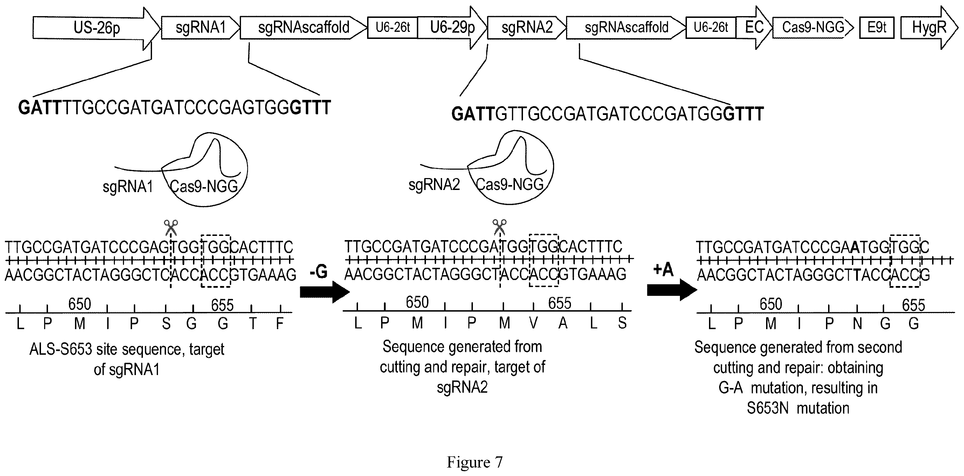

Figure 7 shows a design of the programmed sequential cutting/editing scheme at S653 site of ALS gene of *Arabidopsis thaliana.* The T-DNA sequence expressed four genes, sgRNA1, sgRNA2, Cas9 and HygR. Among them, sgRNA1 and Cas9 formed a complex, which cut the S653 codon of ALS in the genome. After cellular spontaneous repair, a -G genotype was formed. This sequence could be recognized and cut by the complex formed by sgRNA2 and Cas9. After cellular spontaneous repair, a +A genotype was formed, resulting in S653N.

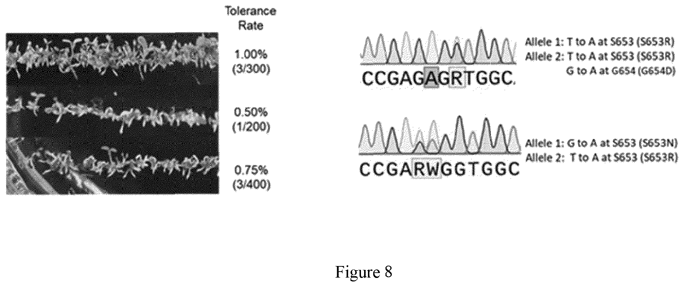

Figure 8 shows the resistant seedlings of *Arabidopsis thaliana* screened by imazapic and the sequencing results of ALS S653 site. The left panel shows the screening results of resistant seedlings and the ratio of resistant seedlings, and the right panel shows a diagram of sequencing peaks and mutation type of S653 site.

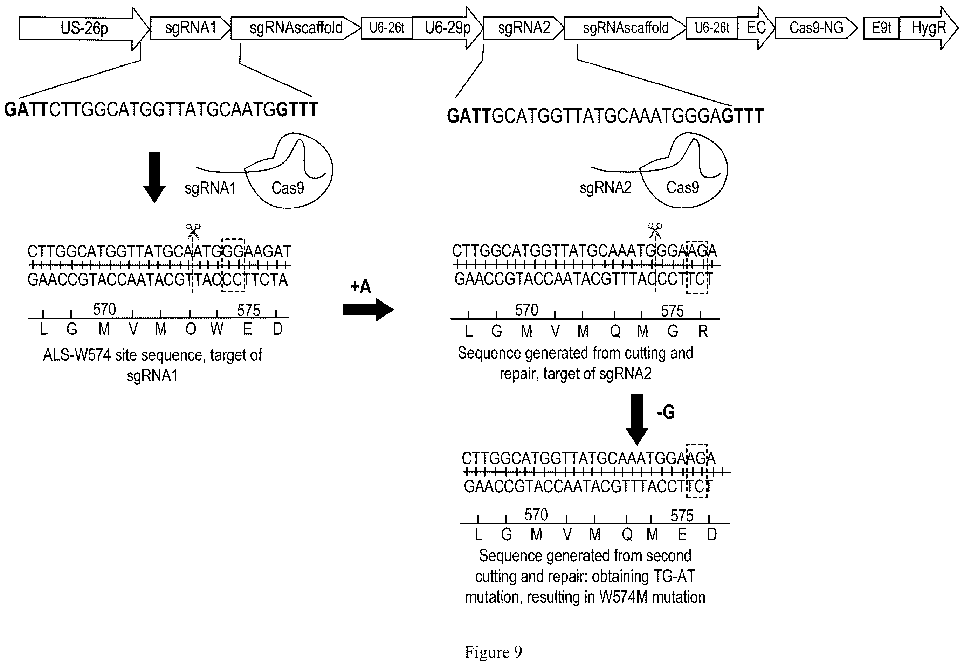

Figure 9 shows a design of the programmed sequential cutting/editing scheme at W574 site of ALS gene of *Arabidopsis thaliana.* The T-DNA sequence expressed four genes, sgRNA1, sgRNA2, Cas9 and HygR. Among them, sgRNA1 and Cas9 formed a complex, which cut the W574 codon of ALS in the genome. After cellular spontaneous repair, the +A genotype was formed. This sequence could be recognized and cut by the complex formed by sgRNA2 and Cas9. After cellular spontaneous repair, the -G genotype was formed, resulting in W574M. The two cuttings used different PAM sites.

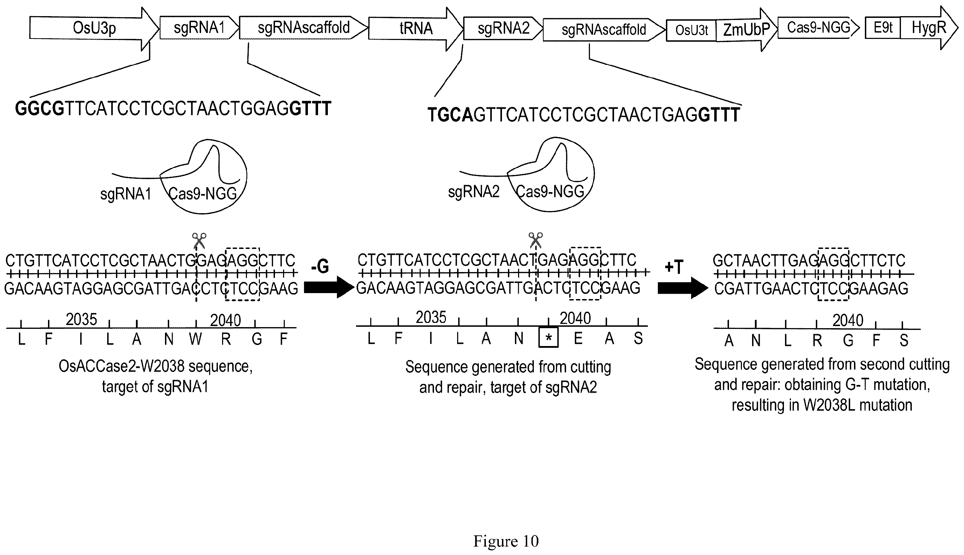

Figure 10 shows a design of the programmed sequential cutting/editing scheme at W2038 site of ACCase2 gene of *Oryza sativa.* This site corresponds to site W2027 of ACCase2 gene of *Alopecurus myosuroides.* The T-DNA sequence expressed four genes, sgRNA1, sgRNA2, Cas9 and HygR. Among them, sgRNA1 and Cas9 formed a complex, which cut the W2038 codon of ACCase in the genome. A -G genotype was formed after cellular spontaneous repair. This sequence could be recognized and cut by the complex formed by sgRNA2 and Cas9. After cellular spontaneous repair, a +T genotype was formed, resulting in W2038L.

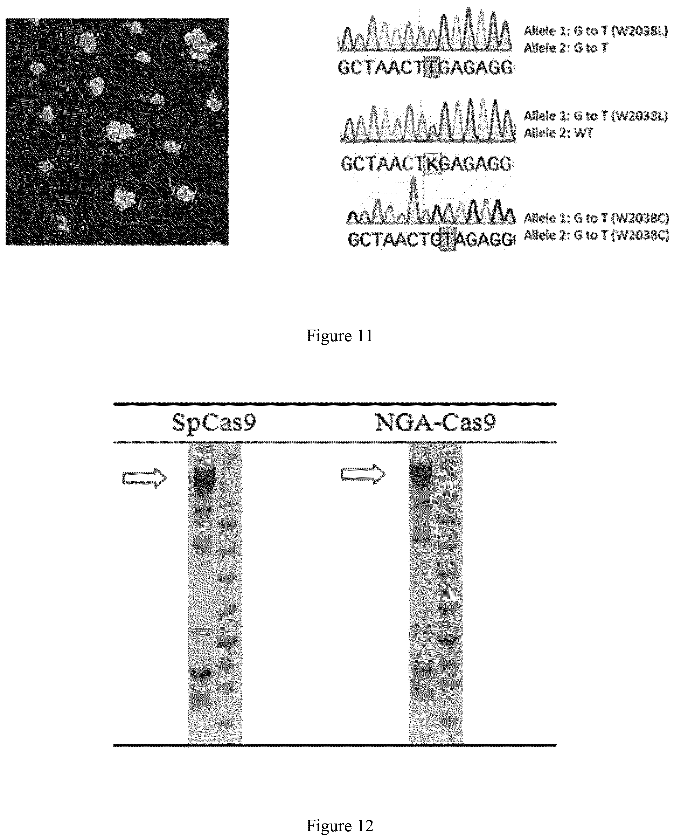

Figure 11 shows the resistant callus of *Oryza sativa* co-screened with hygromycin (50 ug/L) and quizalofop-p (50 ug/L) and the sequencing results of W2038 site.
Figure 12 shows a diagram of polyacrylamide gel electrophoresis of SpCas9 and NGA-Cas9 proteins which were produced by prokaryotic expression and purified. The band indicated by arrow is the Cas9 protein band.

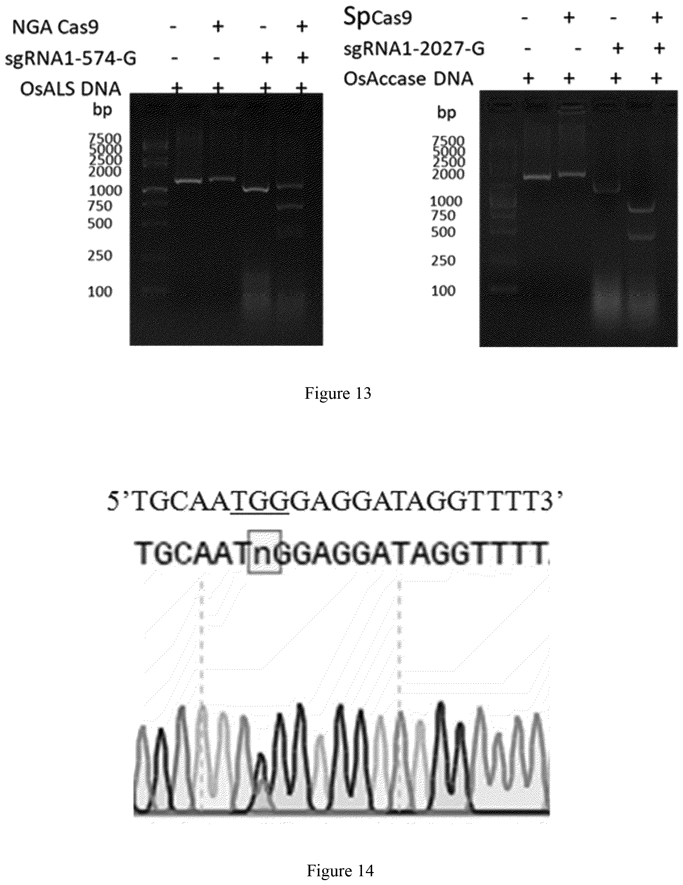

Figure 13 shows the in vitro cutting activity of purified Cas9 protein on DNA fragments containing OsALS W548 target site and OsACCase2 W2038 target site, which was detected by agarose gel electrophoresis, and it can be seen that only when Cas9 protein and sgRNA fragment were added at the same time, the DNA fragments could be cut to an expected size.
Figure 14 shows a diagram of the sequencing peaks of the OsALS W548 target site of RNP transformed *Oryza sativa* protoplasts, and the site corresponds to ALS W574 site of *Arabidopsis thaliana.* At the place indicated by the arrow, in addition to the original G base signal peak, there was a T base signal peak obtained by mutation, which led to a W548L mutation.

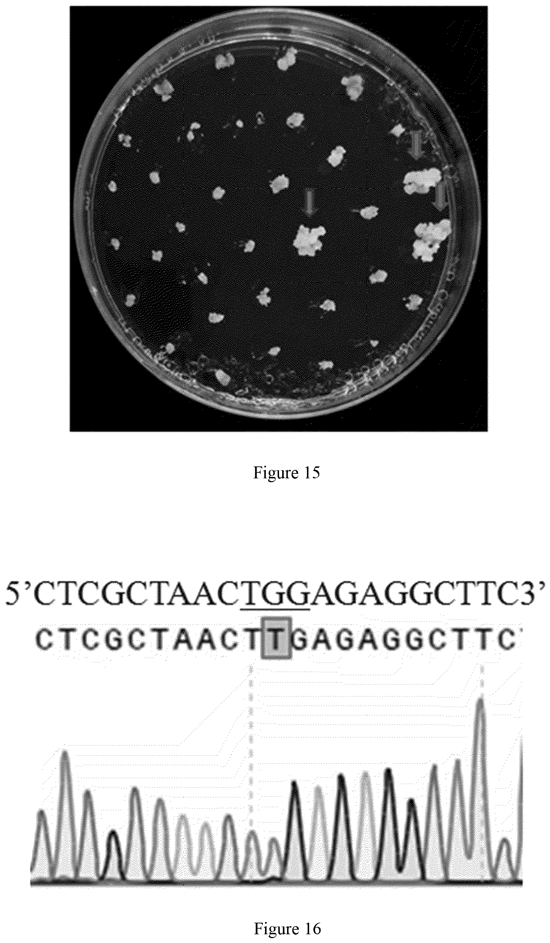

Figure 15 shows the resistant callus of *Oryza sativa* edited by RNP at OsACCase2 W2038 site and screened with 50ug/L quizalofop-p. The arrow indicates the resistant callus.
Figure 16 shows a diagram of sequencing peaks of the OsACCase2 W2038 target site of *Oryza sativa* seedlings differentiated from resistant callus. The T indicated by the arrow was mutated from G, resulting in a W2038L mutation.

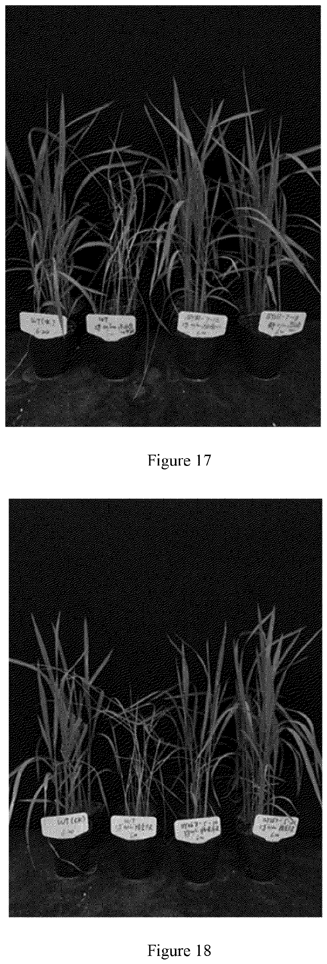

Figure 17 shows the resistance test of T1 generation OsACCase2 W2038L edited seedlings to Haloxyfop-p. From left to right, seedling No. 1 shows the results of the water treatment control of wild-type Huaidao No.5 (a rice variety), and seedlings Nos. 2 to 4 show the results of wild-type Huaidao No.5 and two RNP gene gun-transformed T1 generation W2038L edited strains QY367-7-12 and QY367-7-18, all of which were treated with 5 g/mu active ingredient of Haloxyfop-p (mu, a unit of area, 1 mu = 1/15 hectares). It is clearly that the edited strains developed resistance to Haloxyfop-p.
Figure 18 shows the resistance test of T1 generation OsACCase2 W2038L edited seedlings to quizalofop-p. From left to right, seedling No. 1 shows the results of the water treatment control of wild-type Huaidao No.5, and seedlings Nos. 2 to 4 show the results of wild-type Huaidao No.5 and two RNP gene gun-transformed T1 generation W2038L edited strains QY367-5-10 and QY367-5- 21, all of which were treated with 5 g/mu active ingredient of quizalofop-p. It is clearlythat the edited strains developed resistance to quizalofop-p.

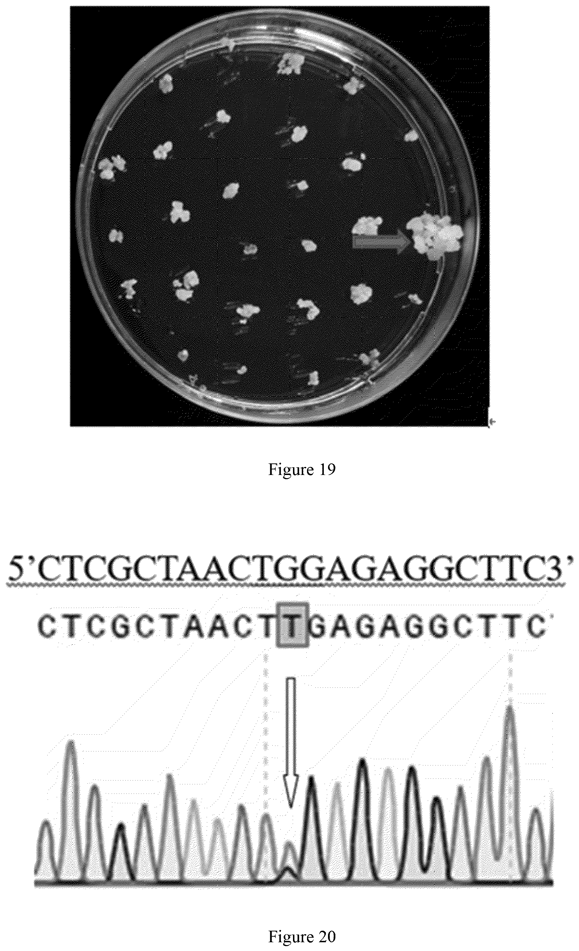

Figure 19 shows the resistant callus of *Oryza sativa* edited by RNP simultaneously at OsACCase2 W2038 site and OsBADH2 gene and screened with 50ug/L quizalofop-p. The arrow indicates the resistant callus.
Figure 20 shows a diagram of sequencing peaks of OsACCase2 W2038 target site of T0 generation double-site edited seedlings. The T indicated by the arrow was mutated from G, resulting in a W2038L mutation.

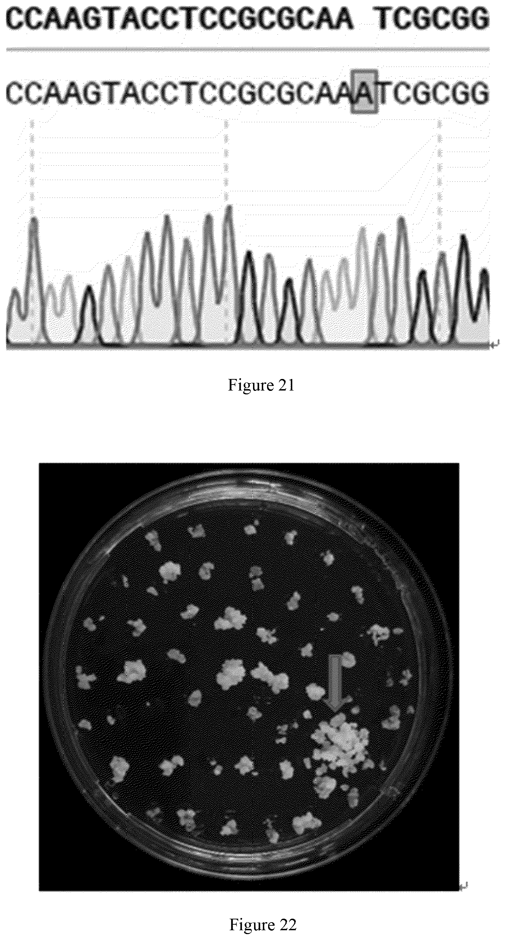

Figure 21 shows a diagram of sequencing peaks of OsBADH2 target site of T0 generation double-site edited seedling. +A homozygous mutation occurred at the point indicated by an arrow.
Figure 22 shows the resistant callus of *Oryza sativa* edited by RNP simultaneously at OsALS W548 site and OsSWEET14 gene and screened with 5mg/L pyroxsulam. The arrow indicates the resistant callus.

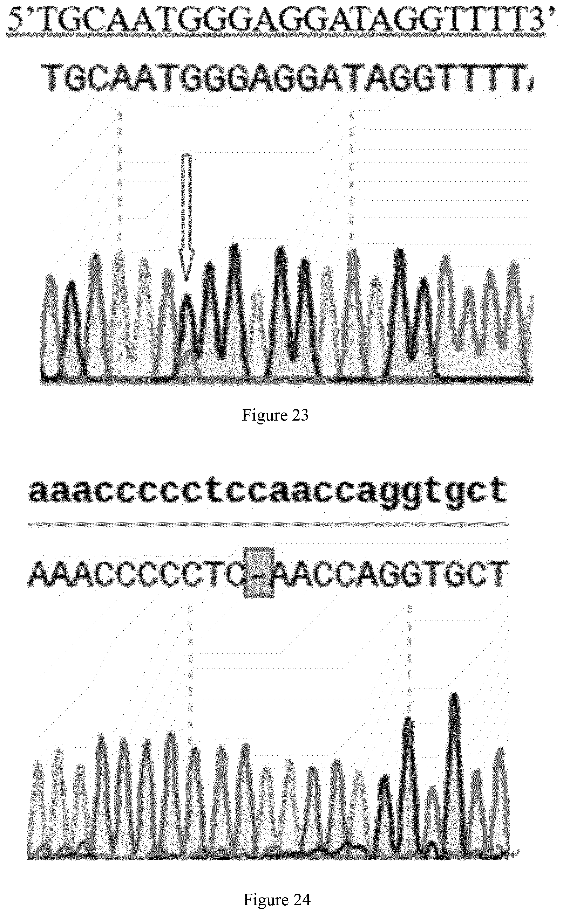

Figure 23 shows a diagram of sequencing peaks of OsALS W548 target site of T0 generation double-site edited seedlings. Both G base and T base signal peaks are present at the place indicated by an arrow, resulting in W548L mutation.
Figure 24 shows a diagram of sequencing peaks of OsSWEET14 target site of T0 generation double-site edited seedlings. -C homozygous mutation occurred at the site indicated by an arrow.

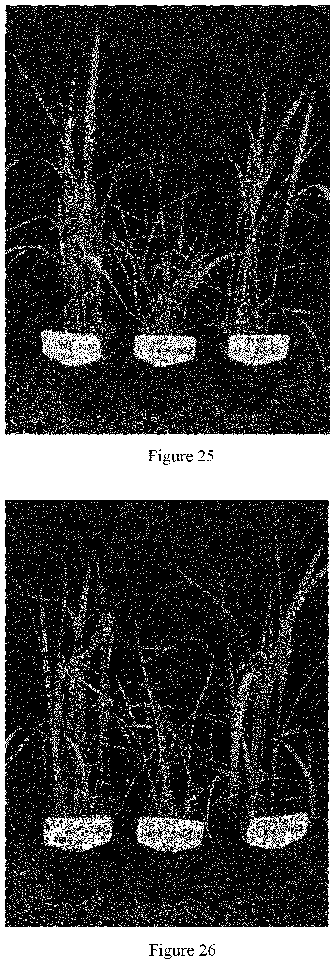

Figure 25 shows the resistance test of T1 generation OsALS W548 site and OsSWEET14 double-site edited seedlings to nicosulfuron. From left to right, seedling No. 1 shows the results of the water treatment control of wild-type Huaidao No.5, and seedlings Nos. 2 to 3 show the results of the wild-type Huaidao No.5 and the RNP gene gun-transformed T1 generation W548L edited strain QY360-7-11, both of which were treated with 4 g/mu active ingredient of nicosulfuron. It is clearly that the edited strain developed resistance to nicosulfuron.
Figure 26 shows the resistance test of T1 generation OsALS W548 site and OsSWEET14 double-site edited seedlings to flucarbazone-Na. From left to right, seedling No. 1 shows the results of the water treatment control of wild-type Huaidao No.5, and seedlings Nos. 2 to 3 show the results of the wild-type Huaidao No.5 and the RNP gene gun-transformed T1 generation W548L edited strain QY360-7-9, both of which were treated with 2 g/mu active ingredient of flucarbazone-Na. It is clearly that the edited strain developed resistance to flucarbazone-Na.

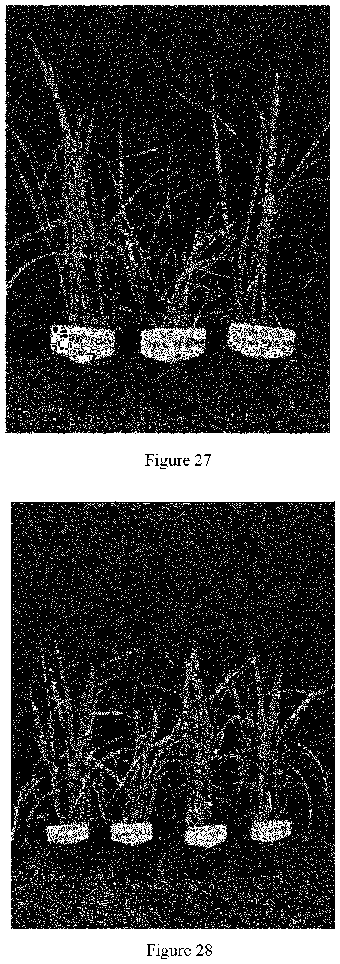

Figure 27 shows the resistance test of T1 generation OsALS W548 site and OsSWEET14 double-site edited seedlings to imazapic. From left to right, seedling No. 1 shows the results of the water treatment control of wild-type Huaidao No. 5, and seedlings Nos. 2 to 3 show the results of the wild-type Huaidao No.5 and the RNP gene gun-transformed T1 generation W548L edited strain QY360-7-11, both of which were treated with 7 g/mu active ingredient of imazapic. It is clearly that the edited strain developed resistance to imazapic.
Figure 28 shows the resistance test of T1 generation OsALS W548 site and OsSWEET14 double-site edited seedlings to pyroxsulam. From left to right, seedling No. 1 shows the results of the water treatment control of wild-type Huaidao No. 5, and seedlings Nos. 2 to 4 show the results of the wild-type Huaidao No.5 and the RNP RNP gene gun- transformed T1 generation W548L edited strains QY360-7-2 and QY360-7-11, all of which were treated with 2 g/mu active ingredient of pyroxsulam. It is clearly that the edited strains developed resistance to pyroxsulam.

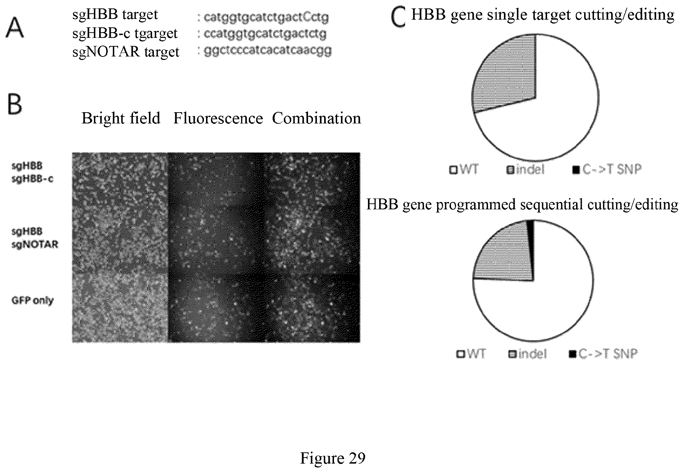

Figure 29 shows a scheme of programmed sequential cutting/editing of HBB gene in 293T cells. A. The design of HBB gene sites for programmed sequential cutting/editing in 293T cells is shown; B. The conversion efficiency of programmed sequential cutting/editing of HBB gene in 293T cells after 48 hours of transformation of each editing vector; C. The ratio of gene editing types generated from programmed sequential cutting/editing of HBB gene and single site cutting/editing of HBB gene in 293T cells; WT: wild type, indel: a genotype for deletion or insertion, C->T SNP: a genotype with C to T base substitution at cutting.

### Description of Sequence Listing

The main sequences involved in the present invention are summarized as follows, and related sequences are provided in the sequence listing.

| SEQ ID NO: | Sequence description |
|---|---|
| 1 | Amino acid sequence of ALS protein from *Arabidopsis thaliana* |
| 2 | DNA sequence of ALS gene from *Arabidopsis thaliana* |
| 3 | Amino acid sequence of AmACCase protein from *Alopecurus myosuroides* |
| 4 | DNA sequence of AmACCase gene from *Alopecurus myosuroides* |
| 5 | Amino acid sequence of OsHPPD protein from *Oryza sativa* |
| 6 | Genomic DNA sequence of OsHPPD protein from *Oryza sativa* |
| 7 | Amino acid sequence of OsPPO 1 protein from *Oryza sativa* |
| 8 | Genomic DNA sequence of OsPPO1 protein from *Oryza sativa* |
| 9 | Amino acid sequence of OsTIR1 protein from *Oryza sativa* |
| 10 | Genomic DNA sequence of OsTIR1 protein from *Oryza sativa* |
| 11 | Amino acid sequence of ALS protein from *Oryza sativa* |
| 12 | DNA sequence of ALS gene from *Oryza sativa* |
| 13 | Amino acid sequence of ACCase2 protein from *Oryza sativa* |
| 14 | Genomic DNA sequence of ACCase2 from *Oryza sativa* |
| 15 | DNA sequence of SpCas9 protein optimized by plant codon |
| 16 | DNA sequence of NGA-Cas9 protein optimized by plant codon |
| 17 | Genomic DNA sequence of OsBADH2 from *Oryza sativa* |
| 18 | Genomic DNA sequence of OsSWEET14 from *Oryza sativa* |
| 19 | Amino acid sequence of StALS2 protein from potato (Solanum tuberosum L.) |
| 20 | DNA sequence of StALS2 gene from potato (Solanum tuberosum L.) |
| 21 | DNA sequence of HBB (hemoglobin subunit beta) gene in human embryonic kidney cell 293 T |
| 22 | CDS sequence of HBB gene in human embryonic kidney cell 293T |
| 23 | Amino acid sequence of HBB gene in human embryonic kidney cell 293T |

### Embodiments for Carrying Out the Invention

The present invention will be further explained in conjunction with the following examples. The following examples are illustrated by way of examples, but the protection scope of the present invention should not be limited to these examples. The experimental methods in the following examples, unless otherwise specified, were the methods described in commonly used molecular biology, tissue culture technology and agronomy manuals. For example, specific steps could be found in: "Molecular Cloning: A Laboratory Manual (3rd edition)" (Sambrook, J., Russell, David W., 2001, Cold Spring Harbor), "Plant Propagation by Tissue Culture" (Edwin F. George , Michael A. Hall, Geert-Jan De Klerk, 2008, Springer). The materials, reagents, instruments, etc. used in the following examples could be obtained from commercial sources unless otherwise specified.

### Example 1: Designing predictable base substitutions introduced by programmed sequential cutting/editing for W574 and S653 sites of ALS gene of Arabidopsis thaliana

### A. Experimental materials

### 1. Arabidopsis thaliana material

The wild-type *Arabidopsis thaliana* Col-0 was a model variety of dicotyledonous plant, its original seeds were provided by the Department of Weeds, College of Plant Protection, China Agricultural University, and the propagation and preservation thereof were performed by our laboratory according to standard methods in this field.

### 2. Vectors

The vector plasmids pCBC-dT1T2 (Xing HL, Dong L, Wang ZP, Zhang HY, Han CY, Liu B, Wang XC, Chen QJ 2014. A CRISPR/Cas9 toolkit for multiplex genome editing in plants. BMC Plant Biol. Nov 29;14(1):327, see https://www.addgene.org/50590/for details), pHEE401E (see Wang ZP, Xing HL, Dong L, Zhang HY, Han CY, Wang XC, Chen QJ Genome Biol. 2015 Jul 21; 16:144. doi: 10.1186/s13059-015-0715-0. https://www.addgene.org/71287/for specific information), and pHEE401E-NG (the mutation as reported in Nishimasu et al. 2018 Engineered CRISPR-Cas9 nuclease with expanded Targeting space. Science 361(6408):1259-1262. doi: 10.1126/science.aas9129 was introduced into pHEE401E to construct vector pHEE401E-NG capable of recognizing NG PAM) were purchased from Addgene website or constructed by our laboratory in accordance with conventional molecular biology methods, and kept by our laboratory.

### 3. Main equipment

Pipette gun, water bath, PCR instrument (Bio-rad T100), electrophoresis instrument (WIX-EP600), gel imager, electric blast dryer, centrifuge (Eppendorf 5424R), high-throughput tissue lyser, shaker, electronic balance, pH meter, etc.

### 4. Main reagents

High-fidelity DNA polymerase (purchased from Tsingke Bio), agarose gel recovery kit and plasmid extraction kit (purchased from Sparkjade), BsaI and T4 DNA ligases (purchased from NEB), Trans5α competent cells and EHA105 competent cells (purchased from TransGen Biotech, Beijing, China), GV3101 *Agrobacterium* competent cells (purchased from Shanghai AngyuBio), Tris, EDTA, kanamycin, cephalosporin, hygromycin, agarose, yeast powder, tryptone, NaCl (purchased from Sangon Biotech), MS powder, sucrose, Silwet-77, hygromycin (purchased from Solarbio), nucleic acid dye (Dured), absolute ethanol (purchased from Sinopharm), etc.

### 5. Preparation of main solutions

1) Seed disinfectant: 4mL of 10% SDS, 20mL NaClO, added with water to make up 200mL.
2) SDS extraction buffer: 40mL of 1M Tris ▪HCl (pH 8.0), 50mL of 0.1M EDTA, 10mL of 5M NaCl, 10mL of 10% SDS, added with water to make up 200mL.
3) Infection solution: 1.5g of sucrose, 9µL of Silwet-77, added with 30mL of ultrapure water.
4) LB solid medium: 5g of yeast powder, 10g of tryptone, 10g of NaCl, 15g of agar, added with water to make up 1L, sterilize at 121°C for 15 minutes, poured on a plate for later use.
5) 50× TAE stock: 242g of Tris, 37.2g of Na₂EDTA·2H₂O, added with 800mL of ultrapure water, stirred well to dissolve, added with 57.1mL of acetic acid, stirred well, finally diluted to 1L with deionized water, and stored at room temperature.
6) MS solid medium: 4.42g of MS powder and 10g of sucrose were weighed, added with 800mL of ultrapure water, adjusted to pH 5.8, added with water to make up 1L, added 10g of phytagel, sterilized at 121°C for 15 minutes, and poured on a plate for later use.

### B. Experimental methods

### 1. Design and construction of CRISPR/Cas9 dual target vector

### 1.1 Target design

The *Arabidopsis thaliana* ALS gene sequence was shown in SEQ ID NO: 2. The target sequence gRNA1 (5'-GCATGGTTATGCAATGGGA-3') of 19 bases was designed using the AGA near *Arabidopsis thaliana* ALS574 site as PAM, it was predicted that one G base would be deleted between the first 3-4 sites of PAM after editing, then a second target sequence gRNA2 (5'-GGCATGGTTATGCAATGGA-3') was designed based on the sequence generated from the deletion, and it was predicted that one T base would be inserted via a second editing, thereby realizing conversion of TGG-TTG, as shown in Figure 2.

Similarly, the target sequence gRNA3 (5'- TGCCGATGATCCCGAGTGG-3') of 19 bases was designed using the TGG near *Arabidopsis thaliana* ALS653 site as PAM, it was predicted that one G base would be deleted between the first 3-4 positions of PAM after editing, then a second target sequence gRNA4 (5'-TTGCCGATGATCCCGATGG-3') was designed based on the sequence generated from the deletion, and it was predicted that one A base would be inserted after the second editing, thereby realizing conversion of AGT-AAT, as shown in Figure 2.

### 1.2 Vector construction

The method described by Xing HL, Dong L, Wang ZP, Zhang HY, Han CY, Liu B, Wang XC, Chen QJ 2014. A CRISPR/Cas9 toolkit for multiplex genome editing in plants. BMC Plant Biol. Nov 29;14(1): 327 was followed. Specifically, dT1T2 plasmid was used as a template to amplify the ALS574 and 653 sites dual-target fragment respectively to construct an sgRNA expression cassette. The vector backbones of pHEE401E and pHEE401E-NG were digested with BsaI, and the bands were cut from the gel and recovered, and the target fragment was directly used for the ligation reaction after digestion. T4 DNA ligase was used to ligate the vector backbones and the target fragment, the ligation products were transformed into Trans5α competent cells, different monoclones were picked out for sequencing. After confirmation via sequencing, the Sparkjade High Purity Plasmid Mini Extraction Kit was used to extract the plasmids to obtain the recombinant plasmids, which were respectively named as pQY743 and pQY745.

### 2. Design of primers for target detection

Primers for target detection took ALS574 and ALS653 target sites as centers, wherein the primer for upstream detection was about 100bp from the ALS574 target site, and the primer for downstream detection was about 280bp from the ALS653 target site. The primer sequences were as follows: 574/653checking-F: 5'ATTGACGGAGATGGAAGCTT3', and 574/653checking-R: 5'CCAAACTGAGCCAGTCACAA3'.

### 3. Establishment of Arabidopsis thaliana genetic transformation system

### 3.1 Agrobacterium transformation

The constructed recombinant plasmids were transformed into *Agrobacterium* GV3101 competent cells to obtain recombinant *Agrobacterium* cells.

### 3.2 Preparation of Agrobacterium infection solution

1) The activated *Agrobacterium* was picked and inoculated in 30ml of YEP liquid medium (containing 25mg/L Rif and 50mg/L Kan), and cultured under shaking at 200 rpm and 28°C overnight until the OD600 value was about 1.0-1.5.
2) After centrifugation at 6000 rpm for 10 minutes, the *Agrobacterium* was collected, and the supernatant was discarded.
3) The *Agrobacterium* was resuspended in the infection solution (no need to adjust pH) to OD600=0.8 for later use.

### 3.3 Transformation of Arabidopsis thaliana

1) Before plant transformation, the attention was paid to whether the plant grew well, the inflorescence was luxuriant, and there was no stress response. The first transformation was carried out when plant height was about 20 cm. Watering could be properly carried out when the soil was dry. The grown siliques were cut off with scissors on the day before transformation.
2) The inflorescence of the plant to be transformed was immersed in the above solution for 30 seconds to 1 minute under gently stirring, and there should be a layer of liquid membrane on the infiltrated plant.
3) After the transformation was completed, the plant was cultured in the dark for 24 hours, and then taken out and placed in a normal light environment for growth.
4) After one week, the second transformation could be carried out in the same way.

### 3.4 Seed harvest

After the seeds were mature, they were harvested. After harvesting, the seeds were dried in an oven at 37°C for about one week.

### 4. Selection of transgenic plant

The seeds were treated with disinfectant for 5 minutes, washed with deionized water for 5 times, and evenly spread on the MS selection medium (containing 30µg/mL hygromycin, 100µg/mL cephalosporin), the medium was placed in a light incubator (temperature 22°C, 16 hours light, 8 hours dark, light intensity 100-150µmol/m²/s, humidity 75%), and one week later, the positive seedlings were selected and transplanted to soil.

### 5. Detection of T1 mutant plants

### 5.1 Extraction of genomic DNA

1) The leaves of *Arabidopsis thaliana* were cut and placed in a 2mL centrifuge tube, added with steel balls, and the leaves were ground with the high-throughput tissue lyser.
2) After the grinding was complete, 400µL of SDS extraction buffer was added, mixed upside down, and incubated in a 65°C water bath for 15 minutes and mixed upside down every 5 minutes.
3) Centrifugation was carried out at 13,000 rpm for 5 minutes.
4) 300µL of the supernatant was pipetted and transferred to a new 1.5mL centrifuge tube, an equal volume of isopropanol pre-cooled at -20°C was added to the centrifuge tube, and the centrifuge tube was placed at -20°C for 1 hour or overnight.
5) Centrifugation was carried out at 13,000 rpm for 10 minutes and the supernatant was discarded.
6) 500µL of 70% ethanol was added to the centrifuge tube to wash the pellet, the washing solution was discarded after centrifugation (be careful not to discard the pellet), dried at room temperature, 30µL of ultrapure water was added to dissolve the DNA, and the DNA was stored at -20°C.

### 5.2 PCR amplification

The extracted T1 plant genome was used as a template, the detection primers were used to amplify target fragment, 5µL of amplified product was pipetted and detected by 1% agarose gel electrophoresis, and imaged with the gel imager. The remaining product was delivered to the sequencing company to directly perform sequencing.

### 6. Detection of T2 mutant plant

After the seeds of the T1 strain were harvested from single plant, the seeds of different strains of two vectors were selected and spread on the imazapic selection medium (MS medium + 0.24µg/mL imazapic) to perform selection, and the positive seedlings were transplanted to the soil one week later and subjected to molecular detection, in which the method was the same as step 5.

The used primers and sequences thereof:

| Primer name | Sequence (5'-3') |
|---|---|
| ALS574-F | ATATATGGTCTCGATTGGCATGGTTATGCAATGGGAGTT TTAGAGCTAGAAATA |
| ALS574-R | ATTATTGGTCTCGAAACTCCATTGCATAACCATGCCCAA TCTCTTAGTCGACTC |
| ALS653-F | ATATATGGTCTCGATTGTGCCGATGATCCCGAGTGGGTT TTAGAGCTAGAAATA |
| ALS653-R | ATTATTGGTCTCGAAACCCATCGGGATCATCGGCAACAA TCTCTTAGTCGACTC |
| ALS574/653checking-F | ATTGACGGAGATGGAAGCTT |
| ALS574/653checking-R | CCAAACTGAGCCAGTCACAA |

### C. Experimental results

### 1. Genotype detection of T1 plant

T1 seeds were selected by MS hygromycin resistance medium, a total of 32 positive seedlings were obtained for the pQY743 vector, and a total of 18 positive seedlings were obtained for the pQY745 vector. For each vector, 10 seedlings were selected and leaf genomic DNA thereof was extracted to detect the target site. It was found that there was no editing occurred in the T1 generation at ALS574 site, and there were editing events that met the design expectations at ALS653 site. The detection results are shown in Table 1:

**Table 1: Mutation types of T1 plants**

| ALS site | Vector number | T1 plant number | Edit type |
|---|---|---|---|
| 574 | pQY743 | 1-10 | WT |
| 653 | pQY745 | 3,4-8,10 | Chimera |
| | | 1,2,9 | Heterozygote |

### 2. Selection results of T2 generation seeds

After the T2 generation seeds of single plant were harvested, they are spread on the imazapic resistant medium to perform selection, it could be seen that the wild-type Col-0 could not grow on the resistant medium, while the positive plants of the mutant strains could grow normally on the resistant medium, as shown in Figure 3.

For each vector, 10 seedlings were selected and genomic DNA thereof was extracted from leaves for molecular detection. It was found that there were 6 strains for the pQY743 vector that had homozygous mutation in line with the expectations, i.e., mutation from TGG to TTG, as shown in Figure 4. In addition, there was 1 strain with heterozygous mutation, and there were 3 strains with chimeric mutation. The detection results are shown in Table 2.

**Table 2: Mutation types of T2 plants**

| ALS site | Vector No. | T2 plant No. | Edit type |
|---|---|---|---|
| 574 | pQY743 | 2,3,5,6,7,9 | Homozygote |
| | | 4 | Heterozygote |
| | | 1,8,10 | Chimera |

The above results showed that by using the technical solution of programmed sequential cutting/editing of the present invention, the design and realization of expected mutations at the target site could be achieved, and the base substitution mutations could be realized by designing sequential sgRNA combinations only with Cas9 protein.

### Example 2: Achieving multiple mutation types by programmed sequential cutting/editing at W574 and S653 sites of Arabidopsis thaliana ALS gene

The operation steps for vector design, construction, and *Arabidopsis thaliana* transformation and selection were performed by referring to Example 1. For AtALS W574 site, the vector design was the same as that of Example 1. The schematic diagram of the vector was shown in Figure 5, and it was expected that the W574L mutation was to be realized by first -G then +T at a specific site. No editing event was detected in the T1 generation of the vector transformed *Arabidopsis thaliana.* The T2 generation of the transgenic *Arabidopsis thaliana* was selected with 0.24 mg/L imazapic, and a large number of herbicide-resistant plants were obtained, as shown in the left panel of Figure 6, the molecular detection of these resistant plants was performed, the PCR product sequencing results showed that not only the expected W574L mutation appeared at the W574 site, but also another resistant mutation W574M appeared, as shown in the right panel Figure 6.

For the AtALS S653 site, the vector design was the same as that of Example 1. The vector diagram was shown in Figure 7, and it was expected that the S653N mutation was to be realized by first -G then +A at a specific site. An expected editing event of S653N was detected in the T1 generation of the vector transformed *Arabidopsis thaliana.* The T2 generation of the transgenic *Arabidopsis thaliana* was continuously selected with 0.24 mg/L imazapic, and a large number of herbicide-resistant plants were obtained, as shown in the left panel of Figure 8, molecular detection was performed to these resistant plants, the PCR product sequencing results showed that not only the expected S653N mutation occured at S653 site, but also two other resistant mutations S653R and S653R/G654D occurred, as shown in the right panel of Figure 8.

The above results showed that by using the technical solution of programmed sequential cutting/editing of the present invention, in addition to the expected mutations designed for the target site, multiple functional mutation types could also be generated by designing sequential sgRNA combinations with only corresponding Cas9 protein, so that it is a suitable tool for creating new functional mutations.

### Example 3: Generating W574M resistance mutation by using different PAMs to perform programmed sequential cutting/editing near the W574 site of Arabidopsis thaliana ALS gene

The operation steps for vector design, construction, and *Arabidopsis thaliana* transformation and selection were performed by referring to Example 1, except that, for the sequence 5'CTTGGCATGGTTATGCAATG*gg*3' near the AtALS W574 site, the GG closer to the W574 site was used first as PAM to design sgRNA1: 5'CTTGGCATGGTTATGCAATG3', in which the W574 site was underlined and the NG PAM was shown in italics. It was predicted that a new sequence 5'CTTGGCATGGTTATGCAAATGGGA*ag*3' was to be formed by +A after cutting and repair. The AG was used as a new PAM site to design sgRNA2: 5'CTTGGCATGGTTATGCAAATGGGA3', and -G genotype was formed after spontaneous repair of cells, resulting in W574M. That was, different PAM sites were used for the two cuttings in this scheme. The vector diagram was shown in Figure 9. The vector was used to transform *Arabidopsis thaliana,* the T1 generation transgenic strain was subjected to genotype detection from which the expected editing event of W574M was detected, and the plant showed resistance to imazapic treatment.

The results showed that by using the technical solution of the present invention and using different PAMs to perform programmed sequential cutting/editing, the editing could be carried out in a wider sequence range to obtain amino acid substitution.

### Example 4: Designing predictable base substitutions for D350 and W548 sites of Oryza sativa ALS gene

The mutation as reported in Nishimasu et al. 2018 Engineered CRISPR-Cas9 nuclease with expanded targeting space. Science 361(6408): 1259-1262. doi: 10.1126/science.aas9129 was introduced into pHUE411 (A CRISPR/Cas9 toolkit for multiplex genome editing in plants. Xing HL, Dong L, Wang ZP, Zhang HY, Han CY, Liu B, Wang XC, Chen QJ. BMC Plant Biol. 2014 Nov 29;14(1):327. 10.1186/s12870-014-0327-y, see details in https://www.addgene.org/71287/) to construct the vector pHUE411-NG capable of recognizing NG PAM

The *Oryza sativa* ALS gene sequence was shown in SEQ ID NO: 12. By using 5'GGCGTGCGGTTTGATGATCG3' (the underlined part was the OsALS-D350 site corresponding to *Arabidopsis thaliana* ALS-D376) and a new sequence 5'GGCGTGCGGTTTGATGACG3' that was predicted to be generated from editing as targets, a dual-target vector was constructed according to the method described in Xing HL, Dong L, Wang ZP, Zhang HY, Han CY, Liu B, Wang XC, Chen QJ. BMC Plant Biol. 2014, and it was expected to obtain the conversion of GAT-GAA, resulting in OsALS D350E mutation.

By using 5'GGTATGGTTGTGCAATGGGA3' (the underlined part was the OsALS-W548 site corresponding to *Arabidopsis thaliana* ALS-W574) and a new sequence 5'GGTATGGTTGTGCAATGGA 3' that was predicted to be generated from editing as targets, a dual-target vector was constructed, and it was expected to obtain the conversion of TGG-TTG, resulting in OsALS W548L mutation.

Then the two vectors were transferred into *Oryza sativa* to obtain transgenic plants, and the identification indicated that the plants with expected substitutions D350E and W548L were obtained. The results of herbicide resistance biotest in field showed that the D350E and W548L mutants acquired the resistance to ALS inhibitor herbicides.

### Example 5: Designing predictable base substitutions and selecting multiple mutation types for W2038 site of Oryza sativa ACCase2 gene

The *Oryza sativa* ACCase2 gene sequence was shown in SEQ ID NO: 14, in which OsACCase2 W2038 site corresponded to the ACCase W2027 site of *Alopecurus myosuroides.* The AGG close to this site was used as PAM to design sgRNA1: 5'TTCATCCTCGCTAAC-TGAG3', and it was predicted that a new sequence was formed by -G after cutting and repair. The AGG was continuously used as PAM to design sgRNA2: 5'CTTC-ATCCTCGCTAACTGAG3', and +T genotype was formed after cutting and repair again, resulting in the W2038L mutation. The sgRNA1 and sgRNA2 were constructed on the pHUE411 vector to form an editing vector, and the vector diagram was shown in Figure 10.

The editing vector was used to transform the callus of Huaidao No 5 (a rice variety), and after 3 weeks of co-selection with 50 µg/L hygromycin and 50 µg/L quizalop-p, a large number of resistant calli were obtained, as shown on the left panel of Figure 11. The resistant callus was taken for genotype identification, and it was found that not only the expected W2038L mutation but also the W2038C mutation occurred, as shown on the right panel of Figure 11.

The above results of programmed sequential cutting/editing of *Oryza sativa* gene showed that the technical solution of the present invention was applicable to both monocotyledonous and dicotyledonous plants.

### Example 6: Expression and purification of SpCas9 and NGA-Cas9 proteins

### 1. Experimental instrument and reagents

**Table 3: Experimental reagents**

| Reagent | Preparation and usage |
|---|---|
| Q5 DNA polymerase | Purchased from NEB |
| I5 DNA polymerase and I5 reaction solution | Purchased from Tsingke Bio |
| dNTPs | Shanghai Sangon |
| Ni-NTA resin | Shanghai Sangon |
| Tris/NaCl/imidazole | Shanghai Sangong |
| DTT | Shanghai Sangong |
| β-mercaptoethanol | Sigma |
| SDS-PAGE precast gel | Nanjing GenScript |
| Superdex200 column | GE Healthcare |

**Table 4: Experimental Instruments**

| Instrument | Model |
|---|---|
| PCR instrument | Bio-rad T100 |
| Constant temperature shaking bed | Ounuo HNY-200B |
| Cell disrupter | Ningbo Xinzhi JY92-IIN |
| Electrophoresis apparatus | WIX-EP600 |
| Protein purifier | GE AKTA pure |
| High speed large capacity refrigerated centrifuge | Xiangzhi ZX21K |
| High-speed centrifuge | Eppendorf 5424R |
| White light film viewer | Beijing Liuyi WD-9406 |
| Nucleic acid gel imager | Beijing Sage ChampGel™ 5000 |
| Micro spectrophotometer | DS-II |

### 2. Experimental method

### 2.1 Construction of pET15b-Cas9 expression vector

The DNA sequences of SpCas9 and NGA-Cas9 proteins obtained after plant codon optimization were shown in SEQ ID NO: 15 and SEQ ID NO: 16, respectively, and the sequences were synthesized by GenScript as template DNAs.

After the NG-Cas9 and NGA-Cas9 sequences were amplified separately, the two fragments were ligated to a pET15b expression vector by infusion method, transformed into DH5a, and sequenced after verification.

**Table 5: PCR verification amplification system and primers**

| | Reaction system, 50 ul |
|---|---|
| 2 × I5 reaction solution | 25 ul |
| Template DNA | 1 ul |
| Forward and reverse primers | 2 ul each |
| Ultrapure water | Added to 50 ul |

| Primer sequence: | 5'-3' |
|---|---|
| pET15b-NG Cas9 -F | gtgccgcgcggcagccatatggattacaaggaccacgacg |
| pET15b-NG Cas9-R | tttgttagcagccggatcctcacttcttcttcttcgcctgc |
| pET15b-NGA Cas9 -F | tgccgcgcggcagccatatgatggattacaaggaccacgacg |
| pET15b-NG Cas9 -R | tttgttagcagccggatcctcacttcttcttcttggcctgtccc |

### 2.2 Expression and purification of proteins

The constructed expression vectors were transformed into *Escherichia coli* Rosetta (DE3), the expression thereof was induced by IPTG, and the bacteria were harvested, lyzed and purified by Ni-NTA column. The specific method was as follows:
a) The recombinant expression vectors were transformed into Rosetta (DE3) strain, a single colony was picked into 10 ml of LB medium, CmR+Amp (pET15b) or CmR+Kana (pET28a) resistance, cultured at 200 rpm at 37°C overnight, transferred to a 2 L shake flask containing 1 L of LB medium, cultured at 37°C and 200 rpm until the OD600 reached 0.6-0.8, cooled to 18°C, the expression thereof was induced with 0.5 mM IPTG overnight, and the bacteria were harvested by centrifugation at 4000g.
b) The harvested bacteria were resuspended in Ni-buffer A: 50 mM HEPES, pH7.4, 500 mM NaCl, 20 mM imidazole, 5 mM β-mercaptoethanol, added with PMSF with a final concentration of 1 mM and 250 ul of Cocktail inhibitor, and then mixed well.
c) After the resuspended cells were disrupted by an ultrasonic disruptor, they are centrifuged at 40000g for 30 minutes at 4°C, and the supernatant was collected and passed through a Ni-NTA column.
d) Purification via Ni-NTA column: the lysate supernatant was combined with resin for 20 minutes, eluted with buffer A containing 50 mM imidazole to remove impurities, and finally eluted with an elution buffer containing 400 mM imidazole.
e) SDS-PAGE gel electrophoresis system was used to detect the purification effect of protein.
f) Dialysis was performed by changing the buffer to 50 mM HEPES pH 7.5, 150 mM KCl, 1 mM DTT, 3% glycerol.
h) The final sample was subjected to SDS-PAGE gel electrophoresis to detect the purification effect of the protein. After concentration by ultrafiltration, the protein was frozen at -80°C for later use.

### 2.3 Expression and purification results of Cas9 fusion proteins

The purification results of SpCas9 and NGA-Cas9 proteins were shown in Figure 12. The arrows indicated the Cas9 protein bands, indicating that a higher purity was achieved.

### Example 7: In vitro enzyme cleavage activity of SpCas9 and NGA Cas9 proteins separately detected to OsACCase2 W2038 and OsALS W548 target sites

1. The DNA sequences of OsALS and OsACCase2 genes were input into the CRISPOR online tool (http://crispor.tefor.net/), the following sgRNAs were designed for the W548 of OsALS (the 548^{th} amino acid of OsALS, the amino acid sequence of *Oryza sativa* ALS protein was shown in SEQ ID NO: 11) corresponding to the amino acid site 574 of *Arabidopsis thaliana* ALS protein and the W2038 of OsACCase2 (the 2038^{th} amino acid of OsACCase2, the amino acid sequence of *Oryza sativa* ACCase2 protein was shown in SEQ ID NO: 13) corresponding to the amino acid site 2027 of *Alopecurus myosuroides*, and these sgRNAs were synthesized by GenScript:
>sgRNA1-548-G: 
5’GGGUAUGGUUGUGCAAUGGAguuuuagagcuagaaauagcaaguuaaaauaaggcuaguccguua
ucaacuugaaaaaguggcaccgagucggugc3’
> sgRNA1-2038-G:

5’GUUCAUCCUCGCUAACUGGAGguuuuagagcuagaaauagcaaguuaaaauaaggcuaguccguua
ucaacuugaaaaaguggcaccgagucggugc3’

2. The specific detection primers, OsALS265AA-F: 5'ggtcttgcgtctggttggc3' and OsALS-end-R: 5'ccatgccaagcacatcaaacaag3', were used to amplify the fragment containing the OsALS W548 target site, and the PCR product was 1200 bp in length.
The specific detection primers, OsACC1750AA-F: 5'gcgaagaagactatgctcgtattgg3' and OsACC2196AA-R: 5'cttaatcacacctttcgcagcc3', were used to amplify the fragment containing the OsACCase W2038 target site, and the PCR product was 1500 bp in length.
The PCR system was shown in the following table:

| Component | Volume |
|---|---|
| 2× I5 reaction solution | 25 µL |
| Forward primer (10 µM) | 2 µL |
| Reverse primer (10 µM) | 2 µL |
| Genomic DNA template | 2 µL |
| Ultrapure water | Added to 50 µL |

3. The PCR reaction was established, and the reaction conditions were shown in the following table:

| Step | Temperature | Time |
|---|---|---|
| Pre-denaturation | 98 °C | 30 s |
| 30-35 amplification cycles | 98 °C | 15 s |
| | 58 °C | 15 s |
| | 72 °C | 30 s |
| Final extension | 72 °C | 3 min |

4. The PCR products were detected by agarose gel electrophoresis and subjected to sequencing for further verification. After being correctly verified, the gel was cut to recover DNA fragments. The recovered DNA fragments were dissolved by adding 30ul of RNase-free ultrapure water, and the concentration thereof was measured.
5. The following detection system was used to detect Cas9 protein activity, and after the system was prepared, it was incubated at 37°C for 1 hour.

| Components | Volume |
|---|---|
| Cas9 protein | 0.5µL (lug) |
| sgRNA | 0.5µL (lug) |
| 10× Cas9 reaction buffer | 2 µL |
| Recovered DNA fragment | XµL (100ng) |
| RNase-free ultrapure water | Added to 20 µL |

6. After the reaction was completed, the system was treated at 65°C for 10 minutes, added with 4 ul of 6× DNA loading buffer, and run on 2% agarose gel to detect band size.

The results were shown in Figure 13, and it can be seen that the purified Cas9 proteins could cut DNA double-strand at the designed target sites only in the presence of sgRNA.

### Example 8: Achieving W548L mutation at OsALS548 site by transforming Oryza sativa protoplast with two different targeting RNP complexes

1. The *Oryza sativa* protoplast preparation and PEG-mediated transformation were performed based on the partially modified method that had been published (Bart et al., 2006), and the specific preparation steps were as follows:
   (1) The *Oryza sativa* seedlings for protoplasts were firstly prepared. The rice variety was Nipponbare. The rice seeds were hulled first, and the hulled seeds were rinsed with 75% ethanol for 1 minute, treated with 5% (v/v) sodium hypochlorite for 20 minutes, then washed with sterile water for more than 5 times, placed on an ultra-clean table and blow-dried, and then placed in a tissue culture flask containing 1/2 MS medium, with 20 seeds in each flask. Protoplasts were prepared by incubating the seeds at 26°C and 12 hours of light for about 10 days.
   (2) The leaf sheath of the seedlings was selected, cut with a sharp Gillette razor blade into about 1 mm pieces, and placed in 0.6M mannitol and MES culture medium (formulation: 0.6M mannitol, 0.4M MES, pH 5.7) for later use. After all the materials were cut, they were transferred to 20 mL of enzymolysis solution (formulation: 1.5% cellulase R10/RS (YaKult Honsha), 0.5% Mecerozyme R10 (YaKult Honsha), 0.5M mannitol, 20mM KCl, 20mM MES, pH5.7, 10mM CaCl₂, 0.1% BSA, 5 mM β-mercaptoethanol), wrapped in aluminum foil and placed in a shaker at 28°C. The enzymolysis was carried out at 50 rpm in the dark for about 4 hours, and the speed was increased to 100 rpm for the last 2 minutes;
   (3) After enzymolysis, an equal volume of W5 solution (formulation: 154mM NaCl, 125mM CaCl₂, 5mM KCl, 15mM MES) was added, shaken horizontally for 10 seconds to release the protoplast. The cells resulted from enzymolysis were filtered through a 300-mesh sieve and centrifuged at 150 g for 5 minutes to collect protoplast;
   (4) The cells were rinsed twice with W5 solution, and the protoplast was collected by centrifugation at 150 g for 5 minutes;
   (5) The protoplast was resuspended with an appropriate amount of MMG solution (formulation: 3.05g/L MgCl₂, 1g/L MES, 91.2g/L mannitol), and the protoplast concentration was about 2×10⁶ cells/mL.

### 2. Preparation of RNP complex:

According to the OsALS548 target site sequence GGGTATGGTTGTGCAATGGGA*gga* (the OsALS W548 site was underlined, corresponding to *Arabidopsis thaliana* ALS W574, and the PAM site recognized by Cas9 protein was shown in italic lowercase), the purified NGA-Cas9 protein was selected to prepare the RNP complex. The GGA was used as PAM to design >CrRNA1-548-G: 5'-GGGUAUGGUUGUGCAAUGGGAguuuuagagcuaugcu-3', it was predicted that one G base would be deleted between the first 3-4 positions of PAM after editing, and then the sequence resulted from the above deletion was used to design a second >CrRNA2-548+T: 5'⁻UGGGUAUGGUUGUGCAAUGGAguuuuagagcuaugcu-3', it was predicted that one T base would be inserted after the second editing to obtain the conversion of TGG-TTG.

>CrRNA1-548-G and >CrRNA1-548+T were synthesized by GenScript Biotechnology Company, and sgRNA was also synthesized:
>sgRNA1-548-G: 
5’GGGUAUGGUUGUGCAAUGGAguuuuagagcuagaaauagcaaguuaaaauaaggcuaguccguua
ucaacuugaaaaaguggcaccgagucggugc3’
>sgRNA2-548+T:

5'UGGGUAUGGUUGUGCAAUGGAguuuuagagcuagaaauagcaaguuaaaauaaggcuaguccguua
ucaacuugaaaaaguggcaccgagucggugc3'.

The synthesized crRNA and GenCRISPR tracrRNA (GenScript SC1933) were mixed equimolarly, added with crRNA&tracrRNA annealing buffer (GenScript SC1957-B) and annealed to prepare gRNA according to the instructions. The tracrRNA sequence was 5'-agcauagcaaguuaaaauaaggcuaguccguuaucaacuugaaaaaguggcaccgagucggugcuuu-3'.

The RNP reaction system was prepared according to the following table, and after the reaction system was prepared, it was incubated at 25°C for 10 minutes.

| Components | Volume |
|---|---|
| NGA Cas9 protein | 10µL (20 µg) |
| gRNA or sgRNA | 10µL (20 µg) |
| 10x× Cas9 reaction buffer | 10 µL |
| RNase-free ultrapure water | 70 µL |

### 3. Protoplast transformation

(1) 200 µl of MMG resuspended protoplast as prepared above was taken and added with the RNP complex (20µg of Cas9 protein, 20µg of sgRNA) generated after the incubation, and gently flicked to mixed well.
(2) An equal volume of 40% (w/v) PEG solution (formulation: 40%(w/v) PEG, 0.5M mannitol, 100mM CaCl₂) was added, gently flicked to mix well, and stood at 28°C in dark for 15 minutes;
(3) After induction and transformation, 1.5 mL of W5 solution was added slowly, gently flicked to mix the cells well, and the cells were collected by centrifugation at 150 g for 3 minutes. This step was repeated once;
(4) the cells were resuspended by adding 1.5 mL of W5 solution, placed in a 28°C incubator and cultured in dark for 12-16 hours. The cells should be incubated for 48 to 60 hours if they were used to extract protoplast genomic DNA.

### 4. Detection of genome target editing event

(1) The protoplast DNA was extracted using the partially modified CTAB method, and the method was specifically described as follows: the protoplast was centrifuged and the supernatant was discarded, 500 µL of DNA extraction solution was added, shaken and mixed well, and incubated in a 65°C water bath for 1 hour; the sample was cooled after the incubation in water bath, added with an equal volume of chloroform, inverted for several times to mix well and centrifuged at 10,000 rpm for 10 minutes; 400 µl of supernatant was taken and transferred to a new 1.5 mL centrifuge tube, added with 1 mL of 70% (v/v) ethanol, and stood to precipitate at -20°C for 20 minutes; the DNA was precipitated by centrifugation at 12,000 rpm for 15 minutes, the precipitate was dried in the air and then dissolved by adding 50 µl of ultrapure water, and stored at -20°C for later use.
(2) The gene-specific primers were used to amplify the fragment containing the W548 target site, and the following detection primers were designed for the target site:
OsALS500AA-F: 5'GGCTAACCCAGGTGTCACAG3',
OsALS-3'UTR-R: 5'CCATGCCAAGCACATCAAACAAG3'

The PCR reaction system was shown in the following table:

| Components | Volume |
|---|---|
| 2× I5 reaction solution | 25 µL |
| Forward primer (10 µM) | 2 µL |
| Reverse primer (10 µM) | 2 µL |
| Genomic DNA template | 2 µL |
| Ultrapure water | Added to 50 µL |

(3) The PCR reaction was established, and the general reaction conditions were shown in the following table:

| Step | Temperature | Time |
|---|---|---|
| Pre-denaturation | 98 °C | 30 s |
| 30-35 amplification cycles | 98 °C | 15 s |
| | 58 °C | 15 s |
| | 72 °C | 30 s |
| Final extension | 72 °C | 3 min |

(4) The detection was carried out by agarose gel electrophoresis, and the PCR fragments were recovered and sequenced.

### 5. Experimental results:

By either using the gRNA prepared with the synthetic crRNA and tracrRNA by annealing or directly using the synthetic sgRNA, an active RNP complex could be formed with the purified NGA-Cas9 protein. By sequencing the OsALS548 targeted site, the mutation from TGG to TTG could be detected, which demonstrated that the site-specific mutation of the target site in the cell could be achieved by the programmed sequential cutting/editing generated from the RNP complex in combination with crRNA or sgRNA in sequential order. As shown in Figure 14, at the arrow point in the diagram of protoplast OsALS548 target sequencing peaks, in addition to the original G base signal peak, there was also the T base signal peak generated from the mutation, which resulted in the W548L mutation at OsALS548 site.

### Example 9: Achieving W2038L mutation at OsACCase2 W2038 site by bombarding Oryza sativa callus with two different targeted RNP complexes

### 1. Preparation of RNP complexes:

According to the OsACCase2 W2038 target site sequence GTTCATCCTCGCTAACTGGAG*agg*, in which the OsACCase2 W2038 site, corresponding to the *Alopecurus myosuroides* ACCase W2027, was underlined, and the PAM site recognized by the Cas9 protein was shown in italic lowercase, the purified SpCas9 protein was selected to prepare the RNP complexes. The GGA was used as PAM to design >CrRNA1-2038-G: 5'-GUUCAUCCUCGCUAACUGGAGguuuuagagcuaugcu-3', it was predicted that one G base would be deleted between the first 3-4 positions of PAM after editing, then the sequence generated from the deletion was used to design a second >CrRNA2-2038+T: 5'-UGUUCAUCCUCGCUAACUGAGguuuuagagcuaugcu-3', it was predicted that one T base would be inserted after the second editing, thus the conversion of TGG-TTG was obtained.

>CrRNA1-2038-G and >CrRNA2-2038+T were synthesized by GenScript Biotechnology Company, and sgRNA was also synthesized:
>sgRNA1-2038-G: 
5'GUUCAUCCUCGCUAACUGGAGguuuuagagcuagaaauagcaaguuaaaauaaggcuaguccguua
ucaacuugaaaaaguggcaccgagucggugc 3'
>sgRNA2-2038+T:

5’UGUUCAUCCUCGCUAACUGAGguuuuagagcuagaaauagcaaguuaaaauaaggcuaguccguua
ucaacuugaaaaaguggcaccgagucggugc 3’.

The synthesized crRNA and GenCRISPR tracrRNA (GenScript SC1933) were mixed equimolarly, added with crRNA&tracrRNA annealing buffer (GenScript SC1957-B) and annealed to prepare gRNA according to the instructions.

The RNP complexes were prepared by incubating in the same reaction system as in Example 8. Taking the amount of 10 gene gun bombardments for transformation as example: 20 µg of Cas9 protein, 20 µg of gRNA or sgRNA, 10 µl of 10× Cas9 reaction buffer, made up to 100 µl in total with RNase-free ultrapure water, incubated at 25°C for 10 minutes, and mixed gently.

### 2. Induction of Oryza sativa callus

The mature and plump *Oryza sativa* seeds were selected, hulled, and disinfected according to the following steps:
(1) The rice (*Oryza sativa*) seeds were hulled, wherein the rice variety was Huaidao No.5, purchased from the seed market.
(2) The seeds were washed with sterile water for unlimited times until the washed water became clear.
(3) After being sterilized with 70% alcohol for 1 minute, the seeds were then placed in 10% sodium hypochlorite on a horizontal shaker and incubated for 25 minutes under shaking.
(4) After sodium hypochlorite disinfection, the seeds were washed with sterile water for 5 times. The seeds were seeded on callus induction medium (formulation: MS powder (4.42g/L) + 2,4-D (2mg/L) + sucrose (30g/L) + phytagel (4g/L)), and cultivated in the dark at 28°C to induce callus.

### 3. Gene gun RNP bombardment:

(1) Hypertonic cultivation
   The callus with good embryogenicity was transferred to hypertonic medium (formulation: MS powder (4.42g/L) + 2,4-D (2mg/L) + sucrose (30g/L) + D-mannitol (0.4M) ) + phytagel (4g/L)), sterile operation was carried out on an ultra-clean bench, and cultivation was carried out in the dark at 25°C for 4-6 hours.
(2) Preparation of gold powder suspension: by using an imported 1.5 mL EP tube, 30 mg of gold powder (diameter 0.6 µm) was weighed; added with 1 mL of 70% ethanol, subjected to vortex well, and stood on ice for 10 minutes. After centrifugation for 1 minute, the supernatant was discarded; 1 mL of sterile water was added, subjected to vortex thoroughly, centrifuged for 1 minute, the supernatant was discarded, and the above operation was repeated for 3 times. 500 µL of sterilized glycerol (50%) was added, subjected to vortex thoroughly to prepare a gold powder suspension with a concentration of 60 µg/µl, which was stored at -20°C.
(3) 50µl of gold powder suspension (60µg/ml) was taken, added with 100µL of the prepared RNP complex, and mixed gently.
(4) 15 µl of RNP/gold powder mixture was taken and placed in the center of a cleavable membrane of PDS-1000 benchtop gene gun (Bio-Rad), blow-dried, and then bombarded according to the instructions of the instrument.
   Bombardment parameters: the vacuum degree was 26-28, the distance was 6 cm, and the air pressure was 1100 psi or 1350 psi.
(5) After the bombardment was completed, the callus was cultured on a hypertonic medium at 25°C overnight (for 16 hours) in the dark.

### 4. Selection, differentiation and rooting:

(1) After the bombarded callus was cultured overnight, it was transferred to the induction medium, and cultured to recover at 28°C for one week.
(2) After one week of recovery, the callus was transferred to a selection medium (formulation: 4.1g/L N6 powder + 0.3 g/L hydrolyzed casein + 2.8 g/L proline + 2 mg/L 2,4-D + 3% sucrose + 50 ug/L quizalofop-p + 500 mg/L Cef (cephalosporin) + 0.1 g/L inositol + 0.35% phytagel, pH5.8) to perform selection for 3-4 weeks; for the prospective editing of OsACCase2 W2038, 50 ug/L quizalofop-p was used for selection, as shown in Figure 15.
(3) The selected resistant callus with good growth status was transferred to a differentiation medium (formulation: MS powder (4.42g/L) + KT (1mg/L) + sucrose (30g/L) + phytagel (4.5g/L) + 50 ug/L quizalofop-p, pH 5.8), and cultured at 28°C under light to induce differentiation for 2-4 weeks.
(4) The differentiated seedlings were transferred to a rooting medium (formulation: 1/2 MS powder (2.3g/L) + sucrose (30g/L) + phytagel (4.5g/L)) for rooting, the seedlings were tempered after the rooting was completed, and then transferred to pots filled with soil and placed in a greenhouse for cultivation.

### 5. Detection of target editing events in resistant callus and T0 tissue culture seedlings:

A total of 11 resistant calli were obtained in the selection, the DNA thereof was extracted by the CTAB method. The detection primers for the target site were designed as follows: OsACC2038test-F: 5'CTGTAGGCATTTGAAACTGCAGTG3', OsACC2038test-R: 5'GCAATCCTGGAGTTCCT-CTGACC3', and the PCR fragment containing OsACCase2 W2038 site was amplified, recovered and sequenced. The sequencing detection indicated that 10 out of them had the mutation from TGG to TTG at the OsACCase2 W2038 site, in which 3 samples were homozygous mutants.

For the T0 generation tissue culture seedlings obtained by resistant callus differentiation, the DNA thereof was extracted for detecting the editing target site sequence, and there was also a homozygous mutation from TGG to TTG at the OsACCase2 W2038 site, as shown in Figure 16.

### 6. Resistance test of T1 generation seedlings to ACCase inhibitor herbicides

After the propagation of the T0 strain containing the W2038L mutation, the T1 generation mutant seedlings were tested for herbicide resistance with quizalofop-p and haloxyfop-p in field concentrations. It could be seen that the OsACCase2 W2038L mutant strain was significantly resistant to these two ACCase inhibitor herbicides, as shown in Figures 17-18.

In summary, by either using the gRNA prepared with the synthetic crRNA and tracrRNA by annealing, or directly using the synthetic sgRNA, an active RNP complex could be formed with the purified SpCas9 protein; the gene gun bombarded calli could be selected by using quizalofop-p in the tissue culture stage; the homozygous mutation from TGG to TTG could be detected by sequencing the OsACCase2 W2038 target site of the T0 generation tissue culture seedlings; the mutation could be inherited to the T1 generation and showed resistance to ACCase inhibitor herbicides, which further demonstrated that the programmed sequential cutting/editing generated from the RNP complex in combination with the sequential targeting crRNA or sgRNA could achieve site-specific mutation of a target site in the cell, and could guide the production of cell-endogenous selection markers for tissue culture selection, thereby creating herbicide-resistant crops.

### Example 10: Different targeting RNP complexes simultaneously edit OsACCase2 W2038 site and OsBADH2 gene of Oryza sativa callus

The RNP complex was prepared by the method according to Example 8, the gene gun bombardment and tissue culture procedures were the same as those of Example 9. Besides the crRNA or sgRNA targeting the OsACCase2 W2038 site, the crRNA or sgRNA targeting the OsBADH2 gene was added at the same time, and they were incubated with SpCas9 protein to form a targeting RNP complex for a second target gene OsBADH2. The gene gun bombardment was performed, the T0 generation tissue culture seedlings were obtained after recovery culture, screening, differentiation and rooting, and the T1 generation was obtained by propagation.

The *Oryza sativa* OsBADH2 genome sequence was shown in SEQ ID NO: 17. According to the CRISPOR online tool (http://crispor.tefor.net/), the target site sequence CCAAGTACCTCCGCGCAATCG*cgg* was selected, in which the PAM site recognized by the Cas9 protein was shown in italic lowercase, and the purified SpCas9 protein was selected to prepare an RNP complex. The CGG was used as PAM to design >CrRNA1-OsBADH2: 5'-CCAAGUACCUCCGCGCAAUCGguuuuagagcuaugcu-3', it was predicted that the resistant mutation of OsACCase2 W2038L and the knockout mutation event of OsBADH2 could be simultaneously detected in the resistant callus obtained by the selection of quizalofop-p.

>CrRNA1-OsBADH2 was synthesized by GenScript Biotechnology Company, and sgRNA was also synthesized:

>OsBADH2-sgRNA:5'CCAAGUACCUCCGCAAUCGguuuuagagcuagaaauagcaaguuaa
aauaaggcuaguccguuaucaacuugaaaaaguggcaccgagucggugc3'.

The resistant callus was selected according to the transformation steps in Example 9, as shown in Figure 19, and the resistant callus was selected and subjected to differentiation and seedling emergence, and the sequences at the OsACCase2 and OsBADH2 target sites of the callus and T0 generation tissue culture seedlings were sequenced. The OsBADH2 target detection primers were:
OsBADH2-check F: 5'CATCGGTACCCTCCTCTTC3'
OsBADH2-check R: 5'ATCGATCGATTTGGGGCTCA3'

As a result, a total of 13 resistant calli were obtained by selection, the OsACCase2 W2038L mutation event was detected in 11 out of them, and the detection of OsBADH2 target sequence for these 11 callus samples showed that 8 out of them simultaneously contained the editing event at OsBADH2 target site. In the T0 generation tissue culture seedlings obtained by differentiation, the existence of OsACCase2 W2038L mutation and OsBADH2 +A homozygous mutation was detected, as shown in Figures 20-21.

In sum, after the rice callus was subjected to gene gun bombardment with the sequential targeting crRNA- or sgRNA-targeting RNP complex to perform site-specific editing of OsACCase2 W2038 and simultaneously adding the targeting RNP complex targeting the second target gene OsBADH2, the selection of the callus could be performed in the tissue culture stage with quizalofop-p; the OsACCase2 W2038 mutation and the targeted knockout of OsBADH2 simultaneously occurred in 61% of the resistant callus, and the strains containing homozygous mutation of OsBADH2 were detected in the T0 generation tissue culture seedlings. These indicated that the sequential targeting in combination with the RNP transformation for site-specific editing of resistance genes could generate endogenous selection markers, and the addition of the corresponding selection pressure could simultaneously screen the editing events of the second target gene, thereby achieving the site-specific editing of genome by non-transgenic means.

### Example 11: Different targeted RNP complexes simultaneously edit OsALS548 site and OsSWEET14 gene of Oryza sativa callus

The targeting RNP complexes for the OsALS548 site were prepared by the method according to Example 8, in which the crRNA and sgRNA sequences were the same as in Example 8, respectively:
>CrRNA1-548-G: 5'-GGGUAUGGUUGUGCAAUGGGAguuuuagagcuaugcu-3',
>CrRNA2-548+T: 5'-UGGGUAUGGUUGUGCAAUGGAguuuuagagcuaugcu-3',
>sgRNA1-548-G: 
5’GGGUAUGGUUGUGCAAUGGAguuuuagagcuagaaauagcaaguuaaaauaaggcuaguccguua
ucaacuugaaaaaguggcaccgagucggugc3’,
>sgRNA2-548+T:

5'UGGGUAUGGUUGUGCAAUGGAguuuuagagcuagaaauagcaaguuaaaauaaggcuaguccguua
ucaacuugaaaaaguggcaccgagucggugc3'.

Referring to Example 8, the gRNA or sgRNA was incubated with NGA-Cas9 to prepare RNP complexes targeting OsALS548 site.

In addition to the targeted RNP complex for the OsALS548 site, the crRNA or sgRNA for the OsSWEET14 gene was added at the same time, and the targeting RNP complex for the second target gene OsSWEET14 was formed by incubation with the SpCas9 protein. The T0 generation tissue culture seedlings were obtained by performing gene gun bombardment, recovery culture, selection, differentiation, rooting, and the T1 generation was obtained by propagation. The selection pressure was 5 mg/L pyroxsulam.

The *Oryza sativa* OsSWEET14 genome sequence was shown in SEQ ID NO: 18. According to the CRISPOR online tool (http://crispor.tefor.net/), the target site sequence GAGCTTAGCACCTGGTTGGAGggg was selected, in which the PAM sites recognized by the SpCas9 protein was shown in italic lowercase, and the purified SpCas9 protein was selected to prepare the RNP complex. The GGG was used as PAM to design:
>CrRNA1-OsSWEET14:
5'-GAGCUUAGCACCUGGUUGGAGguuuuagagcuaugcu-3', and it was predicted that the resistant mutation of OsALS W548L and the knockout mutation of OsSWEET14 could be simultaneously detected in the resistant callus obtained by the selection of pyroxsulam.

>CrRNA1-Os SWEET14 was synthesized by GenScript Biotechnology Company, and the sgRNA was also synthesized:
>Os SWEET14-sgRNA:

5’GAGCUUAGCACCUGGUUGGAGguuuuagagcuagaaauagcaaguuaaaauaaggcuaguccguua
ucaacuugaaaaaguggcaccgagucggugc3’.

The resistant callus was selected according to the gene gun bombardment and tissue culture procedures as described in Example 9, as shown in Figure 22, and the resistant callus was selected to perform differentiation and seedling emergence, in which the selection medium formulation was: 4.1g/L N6 powder + 0.3 g/L hydrolyzed casein + 2.8 g/L proline + 2 mg/L 2,4-D + 3% sucrose + 5 mg/L pyroxsulam + 500 mg/L Cef (cephalosporin) + 0.1 g/L inositol + 0.35% phytagel, pH 5.8.

The sequences at the OsALS548 site and OsSWEET14 target site of the callus and T0 generation tissue culture seedlings were sequenced. The OsSWEET14 target site detection primers were:
OsSWEET14-check F: 5' ATGGGTGCTGATGATTATCTTGTAT3'
OsSWEET14-check R: 5' TGAAGAGACATGCCAGCCATTG3'

As a result, a total of 9 resistant calli were obtained by the selection, the OsALS W548L mutation event was detected in 8 out of them, and the detection of OsSWEET14 target sequence in these 8 callus samples showed that 5 out of them also contained the editing event at OsSWEET14 target site. In the T0 generation tissue culture seedlings obtained by differentiation, both the OsALS W548L mutation and the OsSWEET14-C homozygous mutation could be detected, as shown in Figures 23-24.

After the propagation of T0 strains in which the occurrence of OsALS W548L mutation was detected, the T1 generation mutant seedling strains were tested for herbicide resistance with pyroxsulam, imazapic, nicosulfuron and flucarbazone-Na at field concentrations. It could be seen that the OsALS W548L mutant strain showed significant resistance to all of these 4 ALS inhibitor herbicides, as shown in Figures 25-28.

In summary, by either using the gRNA prepared with the synthetic crRNA and tracrRNA by annealing or directly using the synthetic sgRNA, an active RNP complex could be formed with the purified NGA Cas9 protein, the site-specific mutation of a target site in the cells could be achieved by the programmed sequential cutting/editing generated from the RNP complex in combination with the sequential targeting crRNA or sgRNA, the selection of the callus bombarded by gene gun could be performed with pyroxsulam at the tissue culture stage, the mutation from TGG to TTG was detected by sequencing the OsALS548 target site of the T0 generation tissue culture seedlings, and this mutation could be inherited to the T1 generation and showed resistance to ALS inhibitor herbicides.

After the rice callus was subjected to gene gun bombardment by simultaneously adding the targeting RNP complex targeting the second target gene OsSWEET14 and using the SpCas9 protein that recognized NGG PAM, the selection of the callus was performed with pyroxsulam at the tissue culture stage. The OsALS W548L mutation and the targeted knockout of OsSWEET14 occurred simultaneously in 55% of the resistant callus, the occurrence of homozygous mutation of OsSWEET14 could be detected in the T0 generation tissue culture seedlings, which further indicated that the endogenous selection markers could be generated by the programmed sequential cutting/editing in combination with the site-specific editing of resistant genes generated from PNP transformation, and the editing events of the second target gene could be selected at the same time by simultaneously using the Cas9 proteins that recognized different PAM sites and adding a corresponding selection pressure, thereby achieving the site-specific editing of genome by non-transgenic means.

### Example 12: Generation of W561L mutation at StALS561 site by bombarding potato (Solanum tuberosum L.) explants with two different targeted RNP complexes

The amino acid sequence of *Solanum tuberosum L.* StALS2 protein was shown in SEQ ID NO: 19, and the sequence of *Solanum tuberosum L.* StALS2 gene was shown in SEQ ID NO: 20. The methods for the preparation of RNP complexes and the gene gun bombardment referred to Examples 8-9, and the sgRNAs for the original sequence and the edited sequence designed for StALS2W561 site of *Solanum tuberosum L.* StALS2 corresponding to the *Arabidopsis thaliana* ALS574 site were as follows:
>StALS561- G: 
5’GGGAAUGGUGGUUCAGUGGAguuuuagagcuagaaauagcaaguuaaaauaaggcuaguccguua
ucaacuugaaaaaguggcaccgagucggugc3’
>StALS561 +T:

5’UGGGAAUGGUGGUUCAGUGGAguuuuagagcuagaaauagcaaguuaaaauaaggcuaguccguua
ucaacuugaaaaaguggcaccgagucggugc3’,

And the RNP complexes were prepared according to the method described in Example 8.

The recipient potato variety was Atlantic or Favorita, and the leaves, stems and axillary buds thereof were used as explants, respectively. The methods for gene gun bombardment and selection and differentiation were as follows:
(1) Leaves: The whole leaf was cut and spread flat on the hypertonic M6 medium (formulation: 4.42g/L MS powder + 1 ml/L B5 vitamins (Phytotechlab, G219) + 30g/L sucrose + 8g/L agar + 2mg/L 2,4-D + 0.8mg/L zeatin riboside + 0.2M mannitol + 250mg/L Cef), about 5-6 leaves were used for each shot of bombardment. After pre-incubation for 24 hours, the bombardment with gold powder was performed. After bombardment, they were continuously cultured in hypertonic M6 medium in dark for 2 days, then transferred to M6 medium (4.42g/L MS powder+1 ml/L B5 vitamins (Phytotechlab, G219) + 30g/L sucrose + 8g/L agar + 2mg/L 2,4-D + 0.8mg/L zeatin riboside + 250mg/L Cef) and continuously cultured for 1 week, then transferred to M6 medium with selection pressure after 1 week, the selection of resistant callus was performed with the selection pressure of 20µg/L chlorsulfuron (formulation: 4.42g/L MS powder + 1 ml/L B5 vitamins (Phytotechlab, G219) + 30g/L sucrose + 8g/L agar + 2mg/L 2,4-D + 0.8mg/L zeatin riboside + 250mg/L Cef + 20µg/L chlorsulfuron), 4 weeks later, they were transferred to an bud induction medium R4 containing selection pressure (formulation: 4.42g/L MS powder + 1 ml/L B5 vitamins (Phytotechlab, G219) + 30g/L sucrose + 8g/L agar + 2mg/L GA3 + 0.8mg/L zeatin riboside + 250mg/L Cef + 20µg/L chlorsulfuron) until seedling emergence.
(2) Stems: The potato stems (without axillary buds) were taken and cut longitudinally with the incisions facing upwards, and placed on CIMI medium (4.42g/L MS powder + 20g/L sucrose + 8g/L agar + 0.5 mg/L zeatin nucleoside + 2mg/L 2,4-D + 250mg/L Cef + 20µg/L chlorsulfuron), followed by gene gun bombardment, cultured in the dark for 1 day after bombardment, the stems were transferred to fresh CIMI medium and continuously cultured for 1 week, then the stems were transferred to SIMI medium containing selection pressure (formulation: 4.42g/L MS powder + 20g/L sucrose + 8g/L agar + 1mg/L zeatin riboside + 0.1mg/L GA3 + 250mg/L Cef + 20µg/L chlorsulfuron) to perform the selection for resistant buds until seedling emergence.
(3) Axillary buds: The axillary buds were taken from potato stems, cut longitudinally, and transferred to CIMI medium, with the incisions facing upwards, placed on CIMI medium, followed by gene gun bombardment, cultured in the dark for 1 day after bombardment, they were transferred to fresh CIMI medium and continuously cultured for 1 week, and then they were transferred to SIMI medium containing selection pressure to perform the selection for resistant buds until seedling emergence.

The detection primers for StALS2 W561 site were:
StALS561-Check F: 5'GTGGATTAGGAGCAATGGGATTT3'
StALS561-Check R: 5' TTATTTTAGATAATACAATGCCTCG3'

After detection, the editing event of W561L occurred at the StALS2 W561 site of the resistance-screened T0 generation potato tissue culture seedlings, which demonstrated that the non-transgenic transient gene editing method provided by the present invention was suitable for crops such as potatoes from which an exogenous transgenic element can hardly be separated and removed by selfing or hybridization.

### Example 13: Successful use of programmed sequential cutting/editing scheme for base substitution in human 293T cells

The HBB (hemoglobin subunit beta) gene in human embryonic kidney cell 293T (the DNA sequence thereof was shown in SEQ ID NO: 21, the CDS sequence thereof was shown in SEQ ID NO: 22, and the amino acid sequence thereof was shown in SEQ ID NO: 23) was selected, the target site for sequential targeting of sgRNA was designed in the region of the first exon, in which the first target was catggtgcaCctgactcctgAGG. The sgRNA that recognized this target was named sgHBB, and it was predicted that the deletion of one C base could be generated at the sgRNA cut of this site. The second target was ccatggtgcatctgactctgAGG, which recognized the sequence with the deletion of one C base generated from the cutting/editing of the first target, and the sgRNA of this target was named sgHBB-c. The sgRNA with no target site in 293T cells was designed and named sgNOTAR, which was used as a complementing plasmid for transfection in the experiment.

According to the above design, complementary single-stranded DNA fragments were synthesized respectively. After annealing, they were ligated into px458 (addgene: 48138) plasmids digested with BbsI enzyme, and transformed to *E. coli* DH5a competent. After the resultant *E. coli* single colonies were verified by sequencing, the plasmids were extracted and purified with an endotoxin-free plasmid extraction kit (Tiangen Bio).

The vigorously growing 293T cells were digested and isolated with 0.05% trypsin (Gibico), diluted with DMEM medium (10% fetal bovine serum; penicillin + streptomycin double resistant) and inoculated into 24-well culture plates, and placed in a carbon dioxide incubator overnight. On the next day, they were mixed separately with, according to sequential cutting/editing: sgHBB and sgHBB-c plasmids each 0.5ug; single target cutting/editing: sgHBB and sgNOTAR plasmids each 0.5ug; no target control: pEGFP-cl plasmid 1µg. The transformation was performed with lipofectamine3000 (Invitrogen). There were 3 duplications for each group.

48 hours After transformation, pictures were taken with a fluorescence microscope to record the transformation efficiency, and the total DNA of each well was extracted with a nucleic acid extraction kit (Omega).

The designed Hi-tom sequencing primers were as follows:
Hi-HBB-F: gaggtgagtacggtgtgcGCTTACATTTGCTTCTGACACAACT;
Hi-HBB-R: gagttggatgctggatggTCTATTGGTCTCCTTAAACCTGTCTTG.

These primers were used to perform PCR for each DNA sample. The high-throughput sequencing of PCR products was performed by using Hi-tom method (Sci China Life Sci. 2019 Jan;62(1):1-7. doi: 10.1007/s11427-018-9402-9).

The statistical data of the sequencing results were shown in Table 6 and Table 7. These data indicated that the sequential cutting/editing method at HBB site produced an editing outcome from C to T (resulting in P6S mutation) at a ratio of about 1.67%, while the single target cutting/editing method could not produce such base substitution.

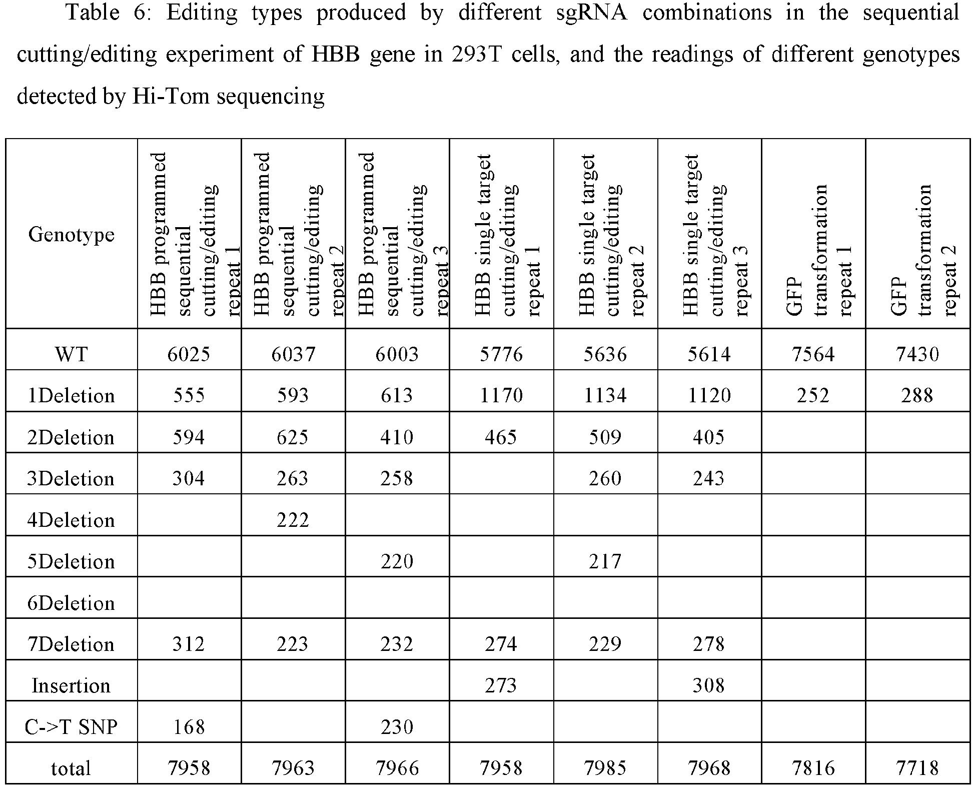

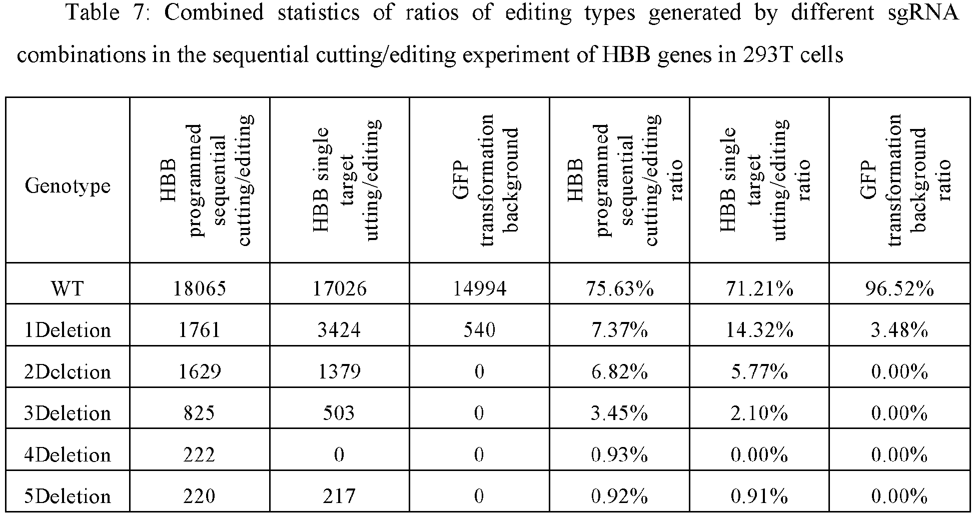

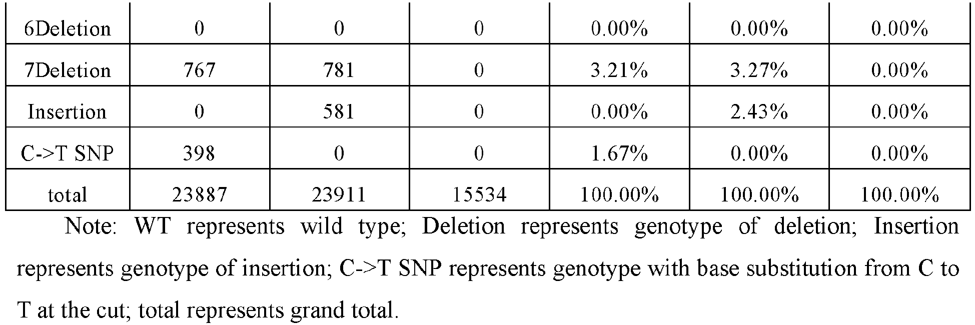

In addition, sickle cell anemia and β-thalassemia are both inherited anemias caused by mutations in the HBB gene encoding hemoglobin β subunit of adult. Patients with these diseases may need blood transfusion or other therapies throughout their lives. The results of the above experiment demonstrated that through the technical solution of programmed sequential cutting/editing as provided by the present invention, a combination of crRNA or sgRNA could be designed for the mutation site of the HBB gene to induce the expected repair at the mutation site, so that the cell produced active hemoglobin and restored function, thereby achieving therapeutic effect. That is, the composition provided by the present invention has a use in treatment of diseases.

After a variety of tests at the same time, because this novel method is completely based on the existing functions of Cas9, the method of the present invention will be fully applicable to achieve the new functions of base substitution, deletion and insertion of specific fragments in other organisms (plants, animals, fungi or bacteria, etc.) where Cas9 can work well.

All publications and patent applications mentioned in the description are incorporated herein by reference, as if each publication or patent application is individually and specifically incorporated herein by reference.

Although the aforementioned invention has been described in more details by way of examples and embodiments for clear understanding, it is obvious that certain changes and modifications can be implemented within the scope of the appended claims, and such changes and modifications are all within the scope of the present invention.

## Claims

1. A method for generating a new mutation in an organism, which comprises the following steps: sequentially generating two or more DNA breaks at a specific site in a genome of the organism and spontaneously repairing them respectively, wherein a later DNA break is generated based on a new sequence generated from a previous DNA break repair.

2. The method according to claim 1, wherein the "DNA break" is achieved by delivering a nuclease with targeting property into a cell of the organism to contact with the specific site of genomic DNA.

3. The method according to claim 2, wherein the "nuclease with targeting property" is a ZFN, TALEN or CRISPR/Cas system.

4. The method according to any one of claims 1 to 3, wherein the "sequentially generating two or more DNA breaks at a specific site" refers to that based on a new sequence formed by a previous DNA break repair event generated by ZFN or TALEN editing, a new ZFN or TALEN protein is designed to cut the site again.

5. The method according to any one of claims 1 to 3, wherein the "sequentially generating two or more DNA breaks at a specific site" refers to that based on a new sequence formed by a previous DNA break repair event generated by a CRISPR/Cas system, a new target RNA is designed to cut the site again.

6. The method according to any one of claims 1-4, wherein the "two or more DNA breaks" are generated by sequentially delivering different targeted nucleases into recipient cells of different generations, wherein a mutant cell that has completed the previous editing is used as a recipient to receive the delivery of the targeted nuclease for the later editing, thereby performing a second editing to generate a site-specific mutation.

7. The method according to any one of claims 1-3 and 5, wherein the "two or more DNA breaks" are generated by delivering different targeted nucleases for different targets into a same recipient cell.

8. The method according to any one of claims 1-3, 5 and 7, wherein the "two or more DNA breaks" are generated when RNP complexes formed by a same CRISPR/Cas nuclease respectively with different gRNAs or sgRNAs sequentially cut corresponding target sequences.

9. The method according to any one of claims 1-3, 5 and 7, wherein the "two or more DNA breaks" are generated when RNP complexes respectively formed by each of two or more CRISPR/Cas nucleases that recognize different PAM sequences with respective gRNA or sgRNA sequentially cut corresponding target sequences.

10. The method according to claim 7, wherein the targeted nuclease is any CRISPR/Cas nuclease capable of performing a genome editing.

11. The method according to claim 6 or 7, wherein the targeted nuclease is in a form of DNA.

12. The method according to any one of claims 7-9, wherein the targeted nuclease is in a form of mRNA or protein instead of DNA.

13. The method according to claim 6 or 7, wherein the method for delivering targeted nuclease into a cell is selected from: 1) a PEG-mediated cell transfection method; 2) a liposome-mediated cell transfection method; 3) an electroporation transformation method; 4) a microinjection; 5) a gene gun bombardment; or 6) an *Agrobacterium-*mediated transformation method.

14. A new mutation, which is obtained by the method according to any one of claims 1-13.

15. A protein or biologically active fragment thereof, which has the new mutation according to claim 14.

16. A nucleic acid, which comprises a nucleic acid sequence or complementary sequence thereof that encodes the protein or biologically active fragment thereof according to claim 15.

17. A nucleic acid, which comprises:
(a) a nucleotide sequence encoding a target RNA, wherein
the target RNA comprises at least two target RNAs, in which a first target RNA targets a DNA to cause a break in the DNA, and a latter target RNA targets a sequence generated from a previous break repair event and generates a break again.

18. The nucleic acid according to claim 17, further comprising (b) a nucleotide sequence encoding a Cas polypeptide.

19. The nucleic acid according to claim 17 or 18, wherein the target RNA is a sgRNA or gRNA.

20. The nucleic acid according to any one of claims 17-19, wherein the Cas polypeptide and the target RNA are present in an in vitro cell or an ex vivo cell.

21. A recombinant expression vector, comprising the nucleic acid according to any one of claims 16-20, and a promoter operably linked thereto.

22. An expression cassette, comprising the nucleic acid according to any one of claims 16-20.

23. A host cell, comprising the expression cassette according to claim 22.

24. An organism, which is regenerated from the host cell according to claim 23.

25. A method for lysing a target DNA, comprising contacting the target DNA with a complex, the complex comprising:
(a) a Cas polypeptide; and
(b) at least two target RNAs, in which the first target RNA targets the DNA to cause a break in the DNA, and the latter target RNA targets a sequence generated from a previous break repair event and generates a break again.

26. The method according to claim 25, wherein the target RNA is sgRNA or gRNA.

27. The method according to claim 25 or 26, wherein the target DNA is present in a bacterial cell, a eukaryotic cell, a plant cell or an animal cell.

28. The method according to any one of claims 25-27, wherein the target DNA is a chromosomal DNA.

29. The method according to any one of claims 25-28, wherein the Cas polypeptide and the target RNA are present in an in vitro cell or an ex vivo cell.

30. The method according to any one of claims 25-29, wherein the contacting comprises introducing the following into the cell: (a) the Cas polypeptide or a polynucleotide encoding the Cas polypeptide, and (b) the target RNA or a DNA polynucleotide encoding the target RNA.

31. A composition, comprising:
(a) a Cas polypeptide, or a polynucleotide encoding the Cas polypeptide; and
(b) at least two target RNAs, or DNA polynucleotides encoding the target RNAs, wherein a first RNA targets a DNA to cause a break in the DNA, and a latter target RNA targets a sequence generated from a previous break repair event and generates a break again.

32. The composition according to claim 31, wherein the target RNA is a sgRNA or gRNA.

33. The composition according to claim 31 or 32, wherein the Cas polypeptide and the target RNA are present in an in vitro cell or an ex vivo cell.

34. Use of the composition according to any one of claims 31-33 in manufacture of a medicament for treatment of a disease.

35. A kit, comprising:
(a) a Cas polypeptide, or a nucleic acid comprising a nucleotide sequence encoding the Cas polypeptide; and
(b) at least two target RNAs, or nucleic acids comprising nucleotide sequences encoding the target RNAs, wherein a first RNA targets a DNA to cause a break in the DNA, and the latter target RNA targets a sequence generated from a previous break repair event and generates a break again;
wherein (a) and (b) are in a same or separate containers.

36. The kit according to claim 35, wherein the target RNA is a sgRNA or gRNA.

37. The kit according to claim 35 or 36, wherein the target RNAs in (b) are in a same or separate containers.

38. A method for screening editing events independent of an exogenous transgenic marker, comprising the following steps:
1) two or more DNA breaks are sequentially generated in sequence at a specific site of a first target gene of a recipient cell and spontaneously repaired respectively, wherein a later DNA break is generated based on a new sequence generated from a repair of a previous DNA break;
2) certain editing events are generated after the specific site of the first target gene is sequentially cut and repaired, which can confer a mutant cell with a resistance to a certain selection pressure to produce a phenotypic selectable trait, a corresponding selection pressure is applied to make a selection for the trait, and a cell, tissue, organ or complete organism that contains such editing events is isolated;
3) optionally, in addition to the first target gene, a targeted nuclease for at least one second target gene is used to edit another target site at the same time, and an editing event of the second target gene is enriched and screened synchronously through the screening of the selectable trait generated by mutations of the first target gene, and a cell, tissue, organ or complete organism that simultaneously contains the editing events of the first target gene and of the at least one second target gene is isolated.

39. The method according to claim 38, wherein the "first target gene" is a gene locus encoding at least one phenotypic selectable trait, wherein the at least one phenotypic selectable trait is a resistance/tolerance trait or a growth advantage trait.

40. The method according to claim 38 or 39, wherein the "specific site of a first target gene" refers to a site at which a certain type of mutation is generated after sequential cuttings and repairs, which is capable of conferring the recipient cell with a resistance to a certain selection pressure to produce at least one phenotypic selectable resistance/tolerance trait or growth advantage trait.

41. The method according to claim 40, wherein the "certain type of mutation" comprises substitution of single base, substitution of a plurality of bases, or insertion or deletion of an unspecified number of bases.

42. The method according to any one of claims 38-41, wherein the "certain selection pressure" is an environmental pressure or a pressure resulted from an added compound, wherein the environmental pressure is preferably high temperature, low temperature or hypoxia; and the pressure resulted from an added compound is preferably a pressure resulted from a salt ion concentration, antibiotic, cytotoxin or herbicide.

43. The method according to any one of claims 38-42, wherein the "DNA break" is achieved by delivering a nuclease with a targeting property into a cell of the organism to contact with the specific site of genomic DNA.

44. The method according to claim 43, wherein the "nuclease with a targeting property" is any CRISPR/Cas nuclease capable of performing genome editing.

45. The method according to any one of claims 38-44, wherein feature, the "two or more DNA breaks are sequentially generated in sequence at a specific site", refers to that based on a new sequence formed by a previous DNA break repair event generated by a CRISPR/Cas system, a new target RNA is designed to cut the site again.

46. The method according to any one of claims 38-45, wherein the "two or more DNA breaks" are generated when RNP complexes formed by a same CRISPR/Cas nuclease respectively with different gRNAs or sgRNAs sequentially cut corresponding target sequences.

47. The method according to any one of claims 38-45, wherein the "two or more DNA breaks" are generated when RNP complexes respectively formed by two or more CRISPR/Cas nucleases that recognize different PAM sequences with respective gRNA or sgRNA, sequentially cut corresponding target sequences.

48. The method according to any one of claims 38-47, wherein the "second target gene" refers to another gene that is different in coding from the first target gene.

49. The method according to any one of claims 38-48, wherein the "targeted nuclease for at least one second target gene" and the CRISPR/Cas nuclease used for generating DNA break at a specific site of the first target gene are the same or different.

50. The method according to any one of claims 38-45 and 48-49, wherein the targeted nuclease is in a form of DNA.

51. The method according to any one of claims 38-49, wherein the targeted nuclease is in a form of mRNA or protein instead of DNA.

52. The method according to any one of claims 43-51, wherein the method for delivering targeted nuclease into a cell is selected from: 1) a PEG-mediated cell transfection method; 2) a liposome-mediated cell transfection method; 3) an electroporation transformation method; 4) microinjection; 5) a gene gun bombardment; or 6) an *Agrobacterium-*mediated transformation method.

53. A method for non-transgenic transient editing of an organism genome, comprising the following steps:
1) a combination of at least two crRNA fragments or a combination of at least two sgRNA fragments is designed and synthesized for a specific site of a first target gene of a recipient cell, wherein the crRNA combination combined with tracrRNA or the sgRNA combination alone is capable of guiding a corresponding Cas protein to sequentially generate two or more DNA breaks at the specific site in the first target gene of the recipient cell and to spontaneously repair them respectively, wherein a later DNA break is generated based on a new sequence generated from a previous DNA break repair;
2) an appropriate amount of CRISPR/Cas protein or corresponding mRNA thereof is mixed with the combination of crRNA fragments and tracrRNA fragment or with the combination of sgRNA fragments alone as above designed and synthesized in advance that is capable of guiding a site-specific editing of the first target gene to generate endogenous selection markers, optionally, at least one of artificially synthesized crRNA and tracrRNA fragments or artificially synthesized sgRNA fragments targeting a second, third or more target genes is further added, and incubation is carried out in vitro to form an RNP complex;
3) the above RNP complex is delivered into the recipient cell and contacts with the specific site of genomic DNA to achieve gene editing;
4) according to a phenotypic selectable trait generated by the site-specific editing of the first target gene by the RNP complex, a corresponding selection pressure is applied to make a selection for the trait, and a cell, tissue, organ or complete organism that contains the editing event is isolated, and optionally, a cell, tissue, organ or complete organism that simultaneously contains the editing events of the first target gene and of the at least one of a second, third or more target genes is isolated.

54. The method according to claim 53, wherein the "first target gene" is a gene locus encoding at least one phenotypic selectable trait, wherein the at least one phenotypic selectable trait is a resistance/tolerance trait or a growth advantage trait.

55. The method according to claim 53 or 54, wherein the "specific site of the first target gene" refers to a site at which a certain type of mutation is generated after sequential cuttings and repairs, which is capable of conferring the recipient cell with a resistance to a certain selection pressure to produce at least one phenotypic selectable resistance/tolerance trait or growth advantage trait.

56. The method of claim 55, wherein the "certain type of mutation" comprises substitution of single base, substitution of a plurality of bases, or insertion or deletion of an unspecified number of bases.

57. The method according to claim 55 or 56, wherein the "certain selection pressure" is an environmental pressure or a pressure e resulted from an added compound; wherein the environmental pressure is preferably high temperature, low temperature or hypoxia; the pressure e resulted from an added compound is preferably a pressure resulted from a salt ion concentration, antibiotic, cytotoxin or herbicide.

58. The method according to any one of claims 53-57, wherein the CRISPR/Cas protein is any CRISPR/Cas nuclease capable of performing genome editing.

59. The method according to any one of claims 53-58, wherein the feature "to sequentially generate two or more DNA breaks at a specific site" refers to that based on a new sequence formed by a previous DNA break repair event generated by a CRISPR/Cas system, a new target RNA is designed to cut the site again.

60. The method according to any one of claims 53-59, wherein the "two or more DNA breaks" are generated when RNP complexes formed by a same CRISPR/Cas nuclease respectively with different gRNAs or sgRNAs sequentially cut corresponding target sequences.

61. The method according to any one of claims 53-59, wherein the "two or more DNA breaks" are generated when RNP complexes respectively formed by each of two or more CRISPR/Cas nucleases that recognize different PAM sequences with respective gRNA or sgRNA, sequentially cut corresponding target sequences.

62. The method according to any one of claims 53-61, wherein the "second, third or more target genes" refer to other genes that are different in coding from the first target gene.

63. The method according to any one of claims 53-62, wherein the "at least one of artificially synthesized crRNA and tracrRNA fragments or artificially synthesized sgRNA fragments targeting a second, third or more target genes" shares the same Cas protein with the crRNA or sgRNA targeting the first target gene.

64. The method according to any one of claims 53-62, wherein the "at least one of artificially synthesized crRNA and tracrRNA fragments or artificially synthesized sgRNA fragments targeting a second, third or more target genes" and the crRNA or sgRNA targeting the first target gene use Cas proteins that recognize different PAM sequences.

65. The method according to any one of claims 53-64, wherein the method for delivering the RNP complex into a cell is selected from: 1) a PEG-mediated cell transfection method; 2) a liposome-mediated cell transfection method; 3) an electroporation transformation method; 4) a microinjection; or 5) a gene gun bombardment.

66. A method for non-transgenic transient editing of a plant genome, comprising the following steps:
1) a combination of at least two crRNA fragments or a combination of at least two sgRNA fragments is designed and synthesized for a specific site of a first target gene of a recipient plant cell or tissue, wherein the crRNA combination combined with tracrRNA or the sgRNA combination alone is capable of guiding a corresponding Cas protein to sequentially generate two or more DNA breaks at the specific site in the first target gene of the recipient cell and to spontaneously repair them respectively, wherein a later DNA break is generated based on a new sequence generated from a previous DNA break repair;
2) an appropriate amount of CRISPR/Cas protein or corresponding mRNA thereof is mixed with the combination of crRNA fragments and tracrRNA fragment or with the combination of sgRNA fragments alone as above designed and synthesized in advance that is capable of guiding a site-specific editing of the first target gene to generate endogenous selection markers, optionally, at least one of artificially synthesized crRNA and tracrRNA fragments or artificially synthesized sgRNA fragments targeting a second, third or more target genes is further added, and incubation is carried out in vitro to form an RNP complex;
3) the above RNP complex is delivered into the recipient plant cell or tissue and contacts with the specific site of genomic DNA to achieve gene editing;
4) according to a phenotypic selectable trait generated by a site-specific editing of the first target gene by the RNP complex, a corresponding selection pressure is applied to make a selection for the trait, and a cell, tissue, organ or complete plant that contains the editing event is isolated, and optionally, a cell, tissue, organ or complete plant that simultaneously contains the editing events of the first target gene and of the at least one of second, third or more target genes is isolated.

67. The method according to claim 66, wherein the "first target gene" is a gene locus encoding at least one phenotypic selectable trait, wherein the at least one phenotypic selectable trait is a resistance/tolerance trait or a growth advantage trait.

68. The method according to claim 66 or 67, wherein the "specific site of the first target gene" refers to a site at which a certain type of mutation is generated after sequential cuttings and repairs, which is capable of conferring the recipient cell with a resistance to a certain selection pressure to produce at least one phenotypic selectable resistance/tolerance trait or growth advantage trait.

69. The method according to claim 68, wherein the "certain type of mutation" comprises substitution of single base, substitution of a plurality of bases, or insertion or deletion of an unspecified number of bases.

70. The method according to claim 68 or 69, wherein the "certain selection pressure" is an environmental pressure or a pressure resulted from an added compound; wherein the environmental pressure is preferably high temperature, low temperature or hypoxia; and the pressure resulted from an added compound is preferably a pressure resulted from a salt ion concentration, antibiotic, cytotoxin or herbicide.

71. The method according to any one of claims 66-70, wherein the "recipient plant cell or tissue" is any cell or tissue that can serve as a recipient for transient expression and can be regenerated into a complete plant through tissue culture; wherein the cell is preferably a protoplast cell or a suspension cell; and the tissue is preferably a callus, immature embryo, mature embryo, leaf, shoot tip, young spike or hypocotyl.

72. The method according to any one of claims 66-71, wherein the CRISPR/Cas protein is any CRISPR/Cas nuclease capable of performing genome editing.

73. The method according to any one of claims 66-72, wherein the feature "to sequentially generate two or more DNA breaks at the specific site" refers to that based on a new sequence formed by a previous DNA break repair event generated by a CRISPR/Cas system, a new target RNA is designed to cut the site again.

74. The method according to any one of claims 66-73, wherein the "two or more DNA breaks" are generated when RNP complexes formed by a same CRISPR/Cas nuclease respectively with different gRNAs or sgRNAs sequentially cut corresponding target sequences.

75. The method according to any one of claims 66-73, wherein the "two or more DNA breaks" are generated when RNP complexes respectively formed by each of two or more CRISPR/Cas nucleases that recognize different PAM sequences with respective gRNA or sgRNA, sequentially cut corresponding target sequences.

76. The method according to any one of claims 66-75, wherein the "second, third or more target genes" refer to other genes that are different in coding from the first target gene.

77. The method according to any one of claims 66-76, wherein the "at least one of artificially synthesized crRNA and tracrRNA fragments or artificially synthesized sgRNA fragments targeting a second, third or more target genes" shares the same Cas protein with the crRNA or sgRNA targeting the first target gene.

78. The method according to any one of claims 66-76, wherein the "at least one of artificially synthesized crRNA and tracrRNA fragments or artificially synthesized sgRNA fragments targeting a second, third or more target genes" and the crRNA or sgRNA targeting the first target gene use Cas proteins that recognize different PAM sequences.

79. The method according to any one of claims 66-78, wherein the method for delivering the RNP complex into the plant cell is selected from: 1) a PEG-mediated protoplast transformation method; 2) a microinjection; 3) a gene gun bombardment; 4) a silicon carbide fiber-mediated method; or 5) a vacuum infiltration method, or any other transient introduction method.

80. The method according to any one of claims 66-79, wherein the "first target gene" is at least one endogenous gene that encodes at least one phenotypic selectable trait selected from herbicide resistance/tolerance, wherein the herbicide resistance/tolerance is selected from the group consisting of resistance/tolerance to EPSPS inhibitor, resistance/tolerance to glutamine synthesis inhibitor, resistance/tolerance to ALS or AHAS inhibitor, resistance/tolerance to ACCase inhibitor, resistance/tolerance to carotenoid biosynthesis inhibitor, resistance/tolerance to cellulose inhibitor, resistance/tolerance to lipid synthesis inhibitor, resistance/tolerance to long-chain fatty acid inhibitor, resistance/tolerance to microtubule assembly inhibitor, resistance/tolerance to photosystem I electron shunting agent, resistance/tolerance to photosystem II inhibitor or resistance/tolerance to PPO inhibitor, and resistance/tolerance to synthetic growth hormone; preferably, the "first target gene" is selected from PsbA, ALS, EPSPS, ACCase, PPO, HPPD, PDS, GS, DOXPS, TIR1 or AFB5.

81. The method according to claim 80, wherein the "first target gene" is ALS, and the "specific site of gene" refers to site A122, P197, R198, D204, A205, D376, R377, W574, S653 or G654 in an *Arabidopsis* AtALS protein amino acid sequence, and amino acid sites in an ALS protein of another plant which correspond to the above-mentioned amino acid sites by using the AtALS amino acid sequence as reference standard; or
the crRNA or sgRNA targets a target sequence comprising a sequence encoding an AtALS protein amino acid sequence site selected from the group consisting of A122, P197, R198, D204, A205, D376, R377, W574, S653, G654 or any combination thereof, and a target sequence comprising a sequence encoding an amino acid site in an ALS protein of another plant which corresponds to the above-mentioned amino acid site, and any combination thereof, by using the AtALS amino acid sequence as reference standard.

82. The method according to claim 80, wherein the "first target gene" is ACCase, and the "specific site of gene" refers to site 11781, E1874, N1878, W1999, W2027, 12041, D2078, C2088 or G2096 in an *Alopecurus myosuroides* AmACCase protein amino acid sequence, and amino acid sites in an ACCase protein of another monocotyledonous plant which correspond to the above-mentioned amino acid sites by using the AmACCase amino acid sequence as reference standard; or
the crRNA or sgRNA targets a target sequence comprising a sequence encoding an AmACCase amino acid sequence site selected from the group consisting of 11781, E1874, N1878, W1999, W2027, 12041, D2078, C2088, G2096 or any combination thereof, and a target sequence comprising a sequence encoding an amino acid site in an ACCase protein of another monocotyledonous plant which corresponds to the above-mentioned amino acid sites, and any combination thereof, by using the AmACCase amino acid sequence as reference standard.

83. The method according to claim 80, wherein the "first target gene" is HPPD, and the "specific site of gene" refers to site H141, L276, P277, N338, G342, R346, D370, P386, K418 or G419 in an *Oryza sativa* OsHPPD protein amino acid sequence, and amino acid sites in an HPPD protein of another plant which correspond to the above-mentioned amino acid sites by using the OsHPPD amino acid sequence as reference standard; or
the crRNA or sgRNA targets a target sequence comprising a sequence encoding an OsHPPD amino acid sequence site selected from the group consisting of H141, L276, P277, N338, G342, R346, D370, P386, K418, G419 or any combination thereof, and a target sequence comprising a sequence encoding an amino acid site in an HPPD protein of another plant which corresponds to the above-mentioned amino acid site, and any combination thereof, by using the OsHPPD amino acid sequence as reference standard.

84. The method according to claim 80, wherein the "first target gene" is PPO, and the "specific site of gene" refers to site S128, V217, S223, V364, K373, L423, Y425 or W470 in an *Oryza sativa* OsPPO1 protein amino acid sequence, and amino acid sites in a PPO protein of another plant which correspond to the above-mentioned amino acid sites by using the amino acid sequence of OsPPO1 as reference standard; or
the crRNA or sgRNA targets a target sequence comprising a sequence encoding an OsPPO1 amino acid sequence site selected from the group consisting of S128, V217, S223, V364, K373, L423, Y425, W470 or any combination thereof, and a target sequence comprising a sequence encoding an amino acid site in a PPO protein of another plant which corresponds to the above-mentioned amino acid site, and any combination thereof, by using the OsPPO1 amino acid sequence as reference standard.

85. The method according to claim 80, wherein the "first target gene" is TIR1, and the "specific site of gene" refers to site F93, F357, C413 or S448 in an *Oryza sativa* OsTIR1 protein amino acid sequence, and amino acid sites in a TIR1 protein of another plant which correspond to the above-mentioned amino acid sites by using the OsTIR1 amino acid sequence as reference standard; or
the crRNA or sgRNA targets a target sequence comprising a sequence encoding an OsTIR1 amino acid sequence site selected from the group consisting of F93, F357, C413, S448 or any combination thereof, and a target sequence comprising a sequence encoding an amino acid site in a TIR1 protein of another plant which corresponds to the above-mentioned amino acid site, and any combination thereof, by using the OsTIR1 amino acid sequence as reference standard.

86. A non-transgenic transient editing system, which uses the method according to any one of claims 53-85.

87. Use of the non-transgenic transient editing system according to claim 86 as a selection marker or in disease treatment or biological breeding.

88. A genetically modified plant obtained by the method according to any one of claims 66-85, wherein the genome thereof comprises:
1) an editing event of a first target gene;
2) an editing event of the first target gene and an editing event of at least one second target gene; or
3) at least one second target gene editing event, wherein the editing event of the first target gene has been removed by genetic separation;
wherein, the genetically modified plant is obtained in a non-transgenic manner.

89. A new plant gene mutation obtained by the method according to any one of claims 66-85.

90. A new mutation generated in a plant, which comprises one or a combination of two or more of the following types:
substitution of aspartic acid at a site corresponding to *Arabidopsis* ALS376 with any other amino acid, substitution of tryptophan at a site corresponding to *Arabidopsis* ALS574 with any other amino acid, substitution of serine at a site corresponding to *Arabidopsis* ALS653 with any other amino acid, or substitution of serine at a site corresponding to *Arabidopsis* ALS654 with any other amino acid; or substitution of tryptophan at a site corresponding to *Alopecurus myosuroides* ACCase2027 with any other amino acid.

91. The mutation according to claim 90, wherein the aspartic acid at a site corresponding to *Arabidopsis* ALS376 is substituted by glutamic acid, the tryptophan at a site corresponding to *Arabidopsis* ALS574 is substituted by leucine or methionine, the serine at a site corresponding to *Arabidopsis* ALS653 is substituted by asparagine or arginine, or the glycine at a site corresponding to *Arabidopsis* ALS654 is substituted by aspartic acid; or, the tryptophan at a site corresponding to *Alopecurus myosuroides* ACCase2027 is substituted by leucine or cysteine.

92. The mutation according to claim 90, wherein the aspartic acid at site 350 of *Oryza sativa* ALS is substituted by any other amino acid, the tryptophan at site 548 of *Oryza sativa* ALS is substituted by any other amino acid, or the tryptophan at site 561 of *Solanum tuberosum L.* ALS2 is substituted by any other amino acid; or, the tryptophan at site 2038 of *Oryza sativa* ACCase2 is substituted by any other amino acid.

93. The mutation according to any one of claims 90-92, wherein the aspartic acid at site 350 of *Oryza sativa* ALS is substituted by glutamic acid, the tryptophan at site 548 of *Oryza sativa* ALS is substituted by leucine or methionine, or the tryptophan at site 561 of *Solanum tuberosum L.* ALS2 is substituted by leucine or methionine; or, the tryptophan at site 2038 of *Oryza sativa* ACCase2 is substituted by leucine or cysteine.

94. A protein or biologically active fragment thereof, comprising the new mutation according to any one of claims 89-93.

95. A nucleic acid, comprising a nucleic acid sequence or complementary sequence thereof that encodes the protein or biologically active fragment thereof according to claim 94.

96. A recombinant expression vector, comprising the nucleic acid according to claim 95 and a promoter operably linked thereto.

97. An expression cassette, comprising the nucleic acid according to claim 95.

98. A plant cell, comprising the expression cassette according to claim 97.

99. A plant regenerated by using the plant cell according to claim 98.

100. A method for producing a plant with improved resistance or tolerance to herbicides, which comprises transforming or transfecting a plant cell with the recombinant expression vector according to claim 96 or the expression cassette according to claim 97, and regenerating the transformed or transfected plant cell into a plant.

101. A method for controlling weeds in a plant cultivation site, wherein the plant includes the plant according to claim 88 or 99 or the plant produced by the method according to claim 100, the method comprises applying to the cultivation site one or more herbicides in an effective amount to control the weeds.

102. Use of the new mutation according to any one of claims 89-93, the protein or biologically active fragment thereof according to claim 94, the nucleic acid according to claim 95, the recombinant expression vector according to claim 96, or the expression cassette according to claim 97 in improving resistance or tolerance of a plant cell, a plant tissue, a plant part or a plant to herbicides.
